(19) Europäisches Patentamt
European Patent Office
Office européen des brevets

(11) EP 0 413 762 B1

(12) **EUROPEAN PATENT SPECIFICATION**

(45) Date of publication and mention of the grant of the patent: **12.07.2000 Bulletin 2000/28**

(21) Application number: **89906570.0**

(22) Date of filing: **04.05.1989**

(51) Int. Cl.[7]: **C12N 9/14**, C12N 9/16, C12N 9/48, C12N 9/88, C07K 14/00, C07C 205/00

(86) International application number: **PCT/US89/01951**

(87) International publication number: **WO 89/10961 (16.11.1989 Gazette 1989/27)**

(54) **PEPTIDE ANALOGS AND THEIR USE AS HAPTENS TO ELICIT CATALYTIC ANTIBODIES**

PEPTIDANALOGE UND DEREN VERWENDUNG ALS HAPTENE ZUM AUSLÖSEN KATALYTISCHER ANTIKÖRPER

ANALOGUES DE PEPTIDES ET LEUR UTILISATION COMME HAPTENES POUR METTRE EN LUMIERE DES ANTICORPS CATALYTIQUES

(84) Designated Contracting States:
**AT BE CH DE FR GB IT LI LU NL SE**

(30) Priority: **04.05.1988 US 190271**

(43) Date of publication of application:
**27.02.1991 Bulletin 1991/09**

(73) Proprietor:
**IGEN International, Inc.**
**Gaithersburg, MD 20877 (US)**

(72) Inventors:
- **TITMAS, Richard, C.**
  **Rockville, MD 20851 (US)**
- **HANSEN, David, E.**
  **Amherst, MA 01002 (US)**
- **HONG, Wonpyo**
  **Potomac, MD 20854 (US)**
- **BOOTH, Paul, M.**
  **Germantown, MD 20874 (US)**
- **POWELL, Michael, J.**
  **Gaithersburg, MD 20852 (US)**
- **REES, Anthony, R.**
  **Rockville, MD 20852 (US)**
- **MASSEY, Richard, J.**
  **Rockville, MD 20852 (US)**

(74) Representative:
**Schlich, George William et al**
**Mathys & Squire**
**European Patent Attorneys,**
**100 Gray's Inn Road**
**London WC1X 8AL (GB)**

(56) References cited:

| | |
|---|---|
| **EP-A- 0 251 093** | **EP-A- 0 260 939** |
| **EP-A- 0 305 870** | **WO-A-88/09380** |
| **US-A- 4 659 567** | **US-A- 4 792 446** |

- **CHEMICAL ABSTRACTS, vol. 99, no. 9, 29th August 1983, page 600, abstract no. 70436q, Columbus, Ohio, US; C.C. WEI et al.: "Desulfurization of penicillins", & SYNTHESIS 1983,(4), 287-8**
- **SCIENCE, vol. 241, 2nd September 1988, pages 1188-1191; K.D. JANDA et al.: "Induction of an antibody that catalyzes hydrolysis of an amide bond**
- **SCIENTIFIC AMERICAN, vol. 258, no. 3, March 1988, pages 42-50, New York, US; R.A. LERNER et al.: "Catalytic antibodies"**
- **BIOCHEM. BIOPHYS. ACTA, Volume 629, issued 1980, F.Kohen, "Antibody-Enhanced Hydrolysis Of Steroid Esters",see pages 328-337**
- **SCIENCE, Volume 240, issued 22 April 1988, P.G.Schultz, "The Interplay Between Chemistry And Biology In The Design Of Enzymatic Catalysts", see pages 426-432.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 413 762 B1

- **SCIENCE, Volume 234, issued 19 December 1986, S.J. Pollack, "Selective Chemical Catalysis By An Antibody", see pages 1570-1573.**
- **SCIENCE, Volume 234, issued 19 December 1986, A. Tramontano, "Catalytic Antibodies", see pages 1566-1570.**

## Description

The invention pertains generally to antibodies, antigens, haptens and immunogens capable of eliciting antibodies which include a paratope that binds to and thereby stabilizes a transition state in the cleavage or formation of an amide bond so that the cleavage or formation is catalyzed by the antibodies.
Several publications are referenced in this application by Arabic numerals within parentheses. Full citation for these references are found at the end of the specification immediately preceding the claims. The references more fully describe the state-of-the-art to which this invention pertains as well as certain aspects of the invention itself.

## BACKGROUND OF THE INVENTION

There are numerous enzymes which have been identified as capable of catalyzing various chemical reactions. Similarly, it has been discovered that antibodies can be elicited to catalyze a variety of chemical reactions (U.S. Appln. Ser. No. 674,253). It is well known that antibodies and enzymes share a fundamental similarity in that both are specialized proteins that bind to other molecules. However, there are important physiological differences between antibodies and enzymes.
Antibodies typically bind to a molecule or antigen so that the antigen is marked as foreign to the organism that produced the antibody. The binding of the antibody to the antigen enables the antigen to be removed from the organism. Enzymes are biological catalysts which bind a molecule in such a way that the activation energy of a reaction involving a molecule or substrate is lowered, thereby increasing the rate of the reaction.
Linus Pauling hypothesized that there are two types of interactions between proteins and the molecules that bind them. Antibodies bind molecules in their ground states most strongly while enzymes bind molecules in higher energy states most strongly.
Pauling attempted to explain the mechanism of enzyme catalysis based upon such binding. During the course of the chemical reaction, the reactants undergo one or more transitions through intermediate structures or transition states which are energically less favorable than either the reactant or the product. The hydrolysis reaction of a peptide linkage or an ester bond in an aqueous medium passes through a tetrahedral carbon transition state, as depicted in Figs. 1 (peptide) and 2 (ester). In the transition state, a tetrahedral carbon atom is bonded to: a carbon atom of the acid portion of the peptide linkage or ester bond; two oxygen atoms, one corresponding to the carbonyl group and the other corresponding to a hydroxyl ion or water molecule of the medium; and either the oxygen atom of the alcohol portion of an ester or the nitrogen atom of the amine portion of the peptide linkage. The transition state can be neither isolated nor detected since it exists for only about $10^{-13}$ sec.
In molecular terms, these transition states reflect changes in bond lengths and bond angles as well as the formation and breakage of bonds. The energy required to achieve a transition state is denoted as the activation energy which may also be considered as the difference in energy between the energy of the transition state and the energy of the reactants. According to Pauling's hypothesis, an enzyme preferentially binds the transition state of a reaction, thereby stabilizing it relative to the substrate and products and reducing the activation energy of the reaction, thus increasing the reaction rate. For example, aspartic proteinases are enzymes which are known to catalyze the hyrolysis of peptide linkages within a protein molecule.
By extending this explanation, Pauling also predicted that stable analogs of a transition state would bind tightly to an enzyme. It has been suggested that the term "transition state analog" might be used to describe an inhibitor of this kind (1).
Pauling's prediction has become the basis for the now well established approach to enzyme inhibitor design. The strategy for designing enzyme inhibitors has suggested a strategy for preparing catalytic antibodies whereby antigens are designed based upon mechanistic principles so that antibodies raised in response to such antigens will catalyze a chemical reaction by carrying out the reaction mechanism implicit in the design of the antigen. This strategy has been attempted a number of times.
For example, a transition-state analog mimicking an intramolecular 6-member ring cyclization transition state was used to elicit a monoclonal antibody which acted as a stereospecific, enzyme-like catalyst (2). Specifically, the monoclonal antibody so elicited accelerated, by about a factor of 170, the formation of a single enantiomer of a δ-lactone from the corresponding racemic δ-hydroxyester.
Similarily, monoaryl phosphonate esters, designated as analogs of the transition state in the hydrolysis of carboxylic esters, were synthesized and used as haptens to elicit specific monoclonal antibodies capable of catalyzing the hydrolysis of carboxylic esters (3). Certain of the antibodies elicited were reportedly found to be catalytic and selective for the hydrolysis of particular aryl esters.
Phosphonamidates or phosphonate analog-ligands having conformations that substantially correspond to the conformation of a hydrolytic transition state of an amide or ester ligand and which have been used to produce antibodies are described in U.S. Patent 4,659,567 to Tramontano et al. (Tramontano). Antibodies so produced purportedly

include a paratope that binds to and stabilizes the tetrahedral carbon atom of the amide or ester hydrolysis transition state of the ligand to hydrolyze the ligand at a predetermined site.

Analog-ligands which can be used to produce antibody catalysts for the hydrolysis of esters and amides are also described in European Patent Application 0,251,093 of Kollmorgen Corp. (Kollmorgen).

In enzyme catalysis, groups on both the substrate and the enzyme which are not involved in the chemical mechanism of bond making and breaking make an important contribution to catalysis. This is illustrated by examining the action of the enzyme succinyl - CoA acetoacetate transferase, shown below, which involves nucleophilic attack of the enzyme's glutamate carboxyl on the thioester succinyl - CoA [1] to give an anhydride intermediate. The enzyme forms anhydride intermediates from "non-specific" substrates [2], as well.

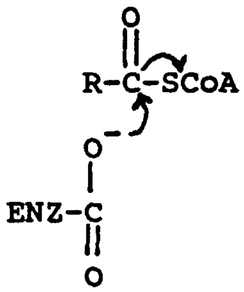

[1]

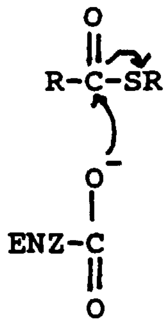

[2]

Even though the chemical reactivities of the two substrates, [1] and [2], are similar, e.g. towards alkaline hydrolysis, the enzyme reaction proceeds up to $3 \times 10^{12}$ fold faster with the so called specific substrate [1](4). The non-reacting part of the substrate, i.e., the CoA residue, lowers the activation energy by 72 $KJmol^{-1}$ compared with [2].

It has also been explicitly noted that to obtain catalysis, the Gibbs free energy of the enzyme-substrate and enzyme-product complex must be increased so that the transition state can be reached easily (5). This represents destabilization of the enzyme-substrate complex which can occur by physical strain, desolvation and other mechanisms.

Thus, the haptens disclosed in Tramontano do not provide the correct architecture to elicit antibodies that are capable of catalyzing the cleavage of a predetermined peptide sequence in a native protein. These haptens do not provide the correct side-chain groups for production of antibodies that can react with predetermined sites on a protein and cause selective proteolysis in a sequence specific manner. Furthermore, these haptens do not incorporate amino acid side-chain sub-sites on either side of the transition state analog. Without these sub-sites, the haptens cannot provide for the elicitation of catalytic antibodies capable of recognizing a specific amino acid sequence and selectively proteolyzing a peptide linkage within that sequence.

## OBJECTS, FEATURES AND ADVANTAGES OF THE INVENTION

It is an object of the invention to provide a rational design approach for designing haptens which are capable of molecular recognition and hydrolytic rate enhancement.

It is also an object of the invention to provide haptens which include an array of atoms which mimics the transition state in the cleavage or formation of an amide bond in a molecule and/or which mimics one or more high energy conformations of an amide, ester or glycosidic bond.

It is a further object of the invention to provide haptens which mimic the native conformation of biomolecules and which have complimentarity with biomolecules.

It is another object of the invention to provide catalytic antibodies which are capable of catalyzing the cleavage or formation of an amide bond in a molecule.

It is yet another object of the invention to provide catalytic antibodies capable of recognizing a specific amino acid sequence in a molecule containing numerous amino acids.

It is a further object of the invention to provide catalytic antibodies which are capable of catalyzing the cleavage or formation of a specific amide bond within a specific amino acid sequence of a molecule.

It is yet another object of the invention to provide a method for catalyzing the cleavage or formation of an amide bond in a molecule.

It is a further object of the invention to provide a method for catalyzing the cleavage or formation of a specific amide bond within a specific amino acid sequence of a molecule containing numerous amino acids joined by amide bonds.

These and other features and advantages of the invention will become readily apparent from the ensuing detailed description, and the novel features will be particularly pointed out in the appended claims.

## SUMMARY OF THE INVENTION

The invention is broadly directed to antigens capable of eliciting through immunogenic methods catalytic antibodies which can catalyze the cleavage or formation of an amide bond in a molecule. The invention is directed to antigens capable of eliciting through immunogenic methods catalytic antibodies which can catalyze the selective cleavage or formation of a predetermined amide bond in a native polypeptide sequence. In general, the antigens can be a hapten or an immunogen comprising a hapten coupled (linked, conjugated) to a carrier molecule via a suitable coupling moiety. The haptens include structural elements which are designed to (i) mimic one or more high energy intermediates or transition states in the cleavage or formation of the amide bond (ii) mimic one or more high energy conformations of the amide bond to be cleaved or (iii) mimic both one or more high energy intermediates or transition states and which mimic one or more high energy conformations in the cleavage or formation of the amide bond.

The haptens according to the invention provide the correct side chain groups for production of antibodies that can react with predetermined sites on a protein and can catalyze selective proteolysis in a sequence specific manner. The haptens further incorporate amino acid side-chain sub-sites surrounding the amide bond analog. These subsites provide for the elicitation of catalytic antibodies capable of recognizing a specific amino acid sequence and selectively proteolyzing a peptide linkage within that sequence.

Such catalytic antibodies are elicited with the haptens of the present invention. For example, a hapten according to the invention, shown below,

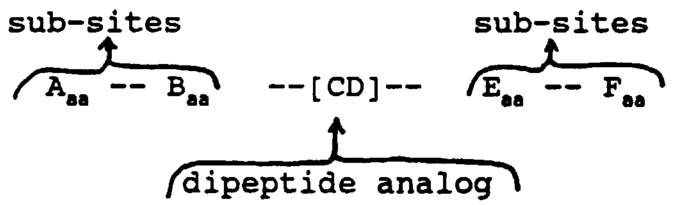


incorporates not only the dipeptide analog [CD] but also sub-site amino acid residues A, B, E, F. These subsite amino acid residues can be part of a cyclic structure as well as a linear structure. The optimum number of sub-site residues is determined by the size of the antibody combining site. It is likely that the only essential criterion for effective binding of antibody to a peptide is that complementarity between the antigen combining site of the antibody and the molecular surface of the binding peptide is maintained with regard to both shape and charge.

The haptens according to the invention are designed in such a way such that antibodies raised against these haptens can selectively stabilize one or any of the high energy intermediates or transition states in the cleavage or formation of an amide bond. These haptens fall into three general classes: one, those in which the hybridization of the atom corresponding to the carbonyl carbon of the scissile bond of the amide or ester bond is converted from $sp^2$ to $sp^3$ hybridization; two, those in which any of the atoms corresponding to the amide bond is replaced by a different atom; and three, those in which the atoms corresponding to the amide bond are part of a monocyclic or bicyclic system.

Peptide sequences containing dipeptide analogs, according to the invention, at the bond that is required to be hydrolyzed by the catalytic antibodies of the present invention define a sequence that the catalytic antibody will hydrolyze in a native protein. The binding energy of the antibody is distributed in such a way as to allow both sequence specific recognition and chemical reactivity with the native protein or peptide of interest.

It has been reported that it is not necessary to prepare peptides longer than eight amino-acid residues (octapeptides) to demonstrate all continuous epitopes (6). It has also been demonstrated that antibodies bind to peptides in a reproducible manner (7). It has also been established that optical isomerism of the amino acids used has a

powerful influence on the strength and specificity of antibody binding by dipeptides. Consequently, the importance of L and D amino acid residues in the immunizing antigen will have a profound effect on the chirality of the antibody combining site generated. In generating catalytic antibodies according to the invention with predetermined specificity for particular sequential (continuous) or assembled epitopes in a native protein, the relationship between measurable properties of a protein and its immunogenic sites are important (8). With the ready availability of protein sequences, the most widely used algorithm is based on the likelihood of finding a sequential epitope at the site of a local maximum in the hydrophilicity profile (9). Surface accessibility profiles (10) and protein flexibility (11) also provide information on the antigen sites in a native protein sequence. With knowledge of these sites and the importance of these epitopes in receptor mediated interactions or other disease associated mechanisms, peptide haptens having dipeptide analogs within these important "bioactive" epitopes can be designed in accordance with the invention. The catalytic antibodies elicited with these haptens can then be utilized, for example, to digest epitopes on viral proteins or tumor derived growth factors or other peptides involved in life-threatening situations (e.g., tumor necrosis factor in bacterial sepsis, etc.).

Thus, the haptens of the invention are distinguished from prior analog-ligands in that they have been rationally designed from knowledge of mechanistic features of enzyme catalysis and provide suitable templates for generating antibody combining sites endowed with catalytic properties. Consequently, they incorporate all the necessary features to provide for antibodies capable of molecular recognition and catalytic function.

Accordingly, the invention is a method for catalyzing the cleavage or formation of a specific amide bond within a molecule. The method comprises contacting the molecule with an amount of a monoclonal antibody effective to catalyze the cleavage or formation of the amide bond under conditions suitable for the cleavage or formation to take place; the monoclonal antibody having been prepared by a process comprising the steps of: selecting the specific amide bond to be cleaved or formed; selecting an antigen comprising an analog of the amide bond to be cleaved or formed, and also comprising moieties surrounding the analog of the amide bond, which moieties substantially correspond to the moieties surrounding the amide bond to be cleaved or formed; exposing cells capable of producing antibodies to the antigen and thereby generating antibody producing cells; hybridizing the antibody producing cells with myeloma cells and thereby generating a plurality of hybridoma cells each producing monoclonal antibodies; and screening the plurality of monoclonal antibodies to identify a monoclonal antibody which catalyzes the cleavage or formation of the amide bond.

In another aspect, the invention is a method for catalyzing the cleavage or formation of a specific amide bond in a molecule which comprises contacting the molecule with an amount of a monoclonal antibody effective to catalyze the cleavage or formation of the amide bond under conditions suitable for the cleavage or formation to take place; the monoclonal antibody having been prepared by a process comprising the steps of: selecting the specific amide bond to be cleaved or formed; selecting an antigen comprising an analog of the amide bond to be cleaved or formed, and also comprising moieties surrounding the analog of said amide bond, the moieties substantially corresponding to some or all of the moieties surrounding the amide bond to be cleaved or formed; exposing cells capable of producing antibodies to the antigen and thereby generating antibody producing cells; hybridizing the antibody producing cells with myeloma cells and thereby generating a plurality of hybridoma cells each producing monoclonal antibodies; and screening the plurality of monoclonal antibodies to identify a monoclonal antibody which catalyzes the cleavage or formation of the amide bond.

The haptens of the invention may be used as antigens for in vitro elicitation of catalytic antibodies. However, for purposes of in vivo elicitation, the haptens must be coupled to a suitable carrier molecule in order to obtain an immunogen suitable for immunization. Therefore, the invention is also directed to immunogens capable of eliciting catalytic antibodies. Such immunogens comprise a hapten as hereinbeforedescribed coupled to a carrier molecule by a suitable coupling moiety.

In another aspect the invention is directed to catalytic antibodies which are elicited by antigens comprising the haptens of the invention as described above. Similarily, the invention is also directed to catalytic antibodies which can catalyze a chemical reaction of interest and which are elicited through in vitro or in vivo techniques by antigens comprising haptens according to the invention as described above, wherein the antibodies have been prepared by exposing cells capable of producing antibodies to the antigens and thereby generating antibody producing cells; hybridizing the antibody producing cells with myeloma cells and thereby producing a plurality of hybridoma cells each producing monoclonal antibodies; and screening the plurality of monoclonal antibodies to identify a monoclonal antibody which catalyzes the chemical reaction of interest.

Alternatively, cells capable of producing catalytic antibodies can be stimulated to grow in culture and, therefore, can be immortalized using methodologies well known in the art. For example, lymphocytes can be so stimulated using a virus, a chemical agent or a nucleic acid (e.g., an oncogene).

In still another aspect, the invention is directed to a method for producing catalytic antibodies which can catalyze a chemical reaction of interest and which are elicited through in vitro or in vivo techniques by antigens comprising the haptens according to the invention as described above. The method comprises exposing cells capable of producing antibodies to the antigens and thereby generating antibody producing cells; hybridizing the antibody producing cells

with myeloma cells and thereby generating a plurality of hybridoma cells each producing monoclonal antibodies; and screening the plurality of monoclonal antibodies to identify a monoclonal antibody which catalyzes the chemical reaction of interest.

The invention is also directed to a method for catalyzing the cleavage or formation of an amide bond in a molecule. The method comprises contacting the molecule with an effective amount of a catalytic antibody which has been elicited by antigens comprising haptens according to the invention.

In another aspect, the invention is directed to a method for catalyzing the cleavage or formation of a specific amide bond within a specific amino acid sequence of a molecule containing numerous amino acids joined by amide bonds. The method comprises contacting the molecule with an effective amount of a catalytic antibody which has been elicited by antigens comprising haptens according to the invention. The haptens have complimentarity with the specific amino acid sequence.

As noted earlier, the catalytic antibodies elicited by antigens comprising haptens according to the invention can be used, for example, to digest epitopes on viral proteins or tumor-derived growth factors on other peptides involved in health- or life- threatening situations.

Thus, in another aspect, the invention is directed to a method for treating acquired immune deficiency syndrome (AIDS) by inhibiting human immunodeficiency virus (HIV). The method comprises treating a patient with an effective amount of a catalytic antibody elicited using a hapten of the invention.

The invention is also directed to a method for treating hypertension by inhibiting human renin activity. The method comprises treating a patient with an effective amount of a catalytic antibody elicited using a hapten of the invention.

## BRIEF DESCRIPTION OF THE DRAWINGS

The invention, as well as other objects, features and advantages thereof will be understood more clearly and fully from the following detailed description. Some of the following examples and drawings do not fall within the claims but are included so as to provide background for the various embodiments of the present invention, and the description is to be read with reference to the accompanying drawings, in which:

Fig. 1 shows the tetrahedral carbon transition state in the hydrolysis of a peptide bond;
Fig. 2 shows the tetrahedral carbon transition state in the hydrolysis of an ester bond;
Fig. 3 depicts pyradazinedione analogs;
Fig. 4 depicts bicyclic β-turn and rigid tricyclic analogs;
Fig. 5 depicts macrocyclic β-turn analogs;
Fig. 6 shows the reaction sequence for the synthesis of N-(N-phenylalanylsulfonylacetyl)glycine;
Fig. 7 shows the reaction sequence for the synthesis of 3-(N-Carbobenzyloxymethyl)sulfonyl-2-methylpropanoic acid;
Fig. 8 shows the reaction sequence for the synthesis of 3-Difluoro-5-(6-maleimidohexanoyl)amino-2-methyl-4-oxo-6-phenylhexanoic acid;
Fig. 9 shows the reaction sequence for the synthesis of 5-[N-Benzyloxycarbonylmethylamido]-3-3-difluoro-2-methyl-4-oxo-6-phenylhexanoic acid;
Fig. 10 depicts a type I β-turn;
Fig. 11 depicts a β-turn containing a N-aminomethylamidinium covalent link;
Fig. 12 depicts a phosphonamidate hapten according to the invention having the β-turn conformation;
Fig. 4 depicts bicyclic β-turn and rigid tricyclic analogs;
Fig. 5 depicts macrocyclic β-turn analogs;
Fig. 6 shows the reaction sequence for the synthesis of N-(N-phenylalanylsulfonylacetyl)glycine;
Fig. 7 shows the reaction sequence for the synthesis of 3-(N-Carbobenzyloxymethyl)sulfonyl-2-methylpropanoic acid;
Fig. 8 shows the reaction sequence for the synthesis of 3-Difluoro-5-(6-maleimidohexanoyl)amino-2-methyl-4-oxo-6-phenylhexanoic acid;
Fig. 9 shows the reaction sequence for the synthesis of 5-[N-Benzyloxycarbonylmethylamido]-3-3-difluoro-2-methyl-4-oxo-6-phenylhexanoic acid;
Fig. 10 depicts a type I β-turn;
Fig. 11 depicts a β-turn containing a N-aminomethylamidinium covalent link;
Fig. 12 depicts a phosphonamidate hapten according to the invention having the β-turn conformation;
Fig. 13 shows Scheme 1 in the synthesis of the conformationally constrained hapten shown in Fig. 12;
Fig. 14 shows Scheme 2 in the synthesis of the conformationally constrained hapten shown in Fig. 12;
Fig. 15 shows Scheme 3 in the synthesis of the conformationally constrained hapten shown in Fig. 12;

Fig. 16 shows the reaction sequence for the synthesis of [(6-maleimido)hexanyl]amino](2-phenylethyl)hydroxyphosphinyl D-alanine;

Fig. 17 shows the reaction sequence for the synthesis of [(6-maleimido)hexanoyl]amino](2-phenylethyl)hydroxyphosphinyl D-alanine;

Fig. 18 shows the reaction sequence for the synthesis of [(6-maleimide)hexanoyl]amino]-2-phenylethyl(2-carboxy-1-propy)hydroxyphosphonous acid; Hydroxycyclobutanecarboxylate and 2-(Benzyloxycarbonyl)amino-3-Oxocyclobutanecarboxylate;

Fig. 25 shows the reaction sequence for the synthesis of 3-*exo*-(Benzyloxycarbonyl)amino-2-*exo*-Hydroxynorbornyl-7-*anti*-carboxylate and 3-*exo*-(Benzyloxycarbonyl)amino-2-Oxonorbornyl-7-*anti*-carboxylate;

Fig. 26 shows the reaction sequence for the synthesis of 3-*endo*-(Benzyloxycarbonyl)amino-2-*endo*-Hydroxynorbornyl-7-*anti*-carboxylate and 3-*endo*-(Benzyloxycarbonyl)amino-2-Oxonorbornyl-7-*anti*-carboxylate;

Fig. 27 shows the dose dependent inhibition by clone AHIV 1.3 of HIV-I p 24 gag production in infected H9 cells; and

Fig. 28 shows the dose dependent inhibition by clones AHIV 1.3, AHIV 1.6 and AHIV 2.0 of HIV-1-induced cell-fusion.

## DETAILED DESCRIPTION OF THE INVENTION

Broadly, the invention relates to antigens which are capable of eliciting through immunogenic methods antibodies which can catalyze the cleavage or formation of a peptide linkage in a molecule. These antigens comprise a hapten or a hapten and a suitable carrier molecule. Because the antibodies so elicited can catalyze a chemical reaction, they are defined as "catalytic antibodies." Catalytic antibodies are identified and described in Schochetman and Massey, Application Serial No. 674,253 filed November 27, 1984, referred to above in the "Statement of the Invention."

During the course of a chemical reaction, the reactants undergo one or more transitions through

Fig. 28 shows the dose dependent inhibition by clones AHIV 1.3, AHIV 1.6 and AHIV 2.0 of HIV-1-induced cell-fusion.

## DETAILED DESCRIPTION OF THE INVENTION

Broadly, the invention relates to antigens which are capable of eliciting through immunogenic methods antibodies which can catalyze the cleavage or formation of a peptide linkage or the cleavage or formation of an ester in a molecule. These antigens comprise a hapten or a hapten and a suitable carrier molecule. Because the antibodies so elicited can catalyze a chemical reaction, they are defined as "catalytic antibodies." Catalytic antibodies are identified and described in Schochetman and Massey, Application Serial No. 674,253 filed November 27, 1984, referred to above in the "Statement of the Invention."

During the course of a chemical reaction, the reactants undergo one or more transitions through structures which are energetically less favorable than either the reactant or product. In molecular terms, these transition states (or intermediate structures) reflect changes in bond lengths and bond angles as well as formation and breakage of bonds. The energy required to achieve a transition state is denoted as the activation energy, which may also be considered as the difference in energy between the energy of the transition state and the energy of the reactants.

Catalysts increase chemical reaction rates by lowering the activation energy of a reaction. Antibodies elicited to a hapten or immunogen of the invention, which antigens are chosen because, inter alia, they resemble the presumed transition state structure (i.e., a transition state analog or a strained antibody can then catalyze the formation of peptide bonds wherein those cleaved strands having the right structural conformation are joined.

Thus, the haptens of the invention are designed to mimic the transition-states or strained ground states or both for a variety of chemical reactions. Preferably, though not exclusively, the reactions are the cleavage or formation of a peptide bond bond.

In an embodiment of the invention, a method is provided for catalyzing the cleavage or formation of a specific amide bond within a specific amino acid sequence contained in a molecule. The molecule is contacted with an amount of a monoclonal antibody effective to catalyze the cleavage or formation of the amide bond under conditions suitable for the cleavage or formation to take place. The monoclonal antibody is elicited using an antigen comprising a hapten of the invention.

The term "amide bond" refers to a simple amide bond (e.g., an amide bond in a side chain of a naturally occurring amino acid) or an amide bond which joins two adjacent amino acid residues, i.e., a peptide bond. The term "peptide" includes dipeptides and polypeptides.

The term "analog of an amide bond" as used herein is defined as a normal amide bond (- CO - NH -) in which one or more moieties in the normal amide bond are replaced by one or more different moieties similar in charge and/or size to the normal moieties replaced. "Moiety" is defined as a radical (e.g., an atom, $CH_3$, $C_6H_5$, OH, $NH_2$, etc.). For

example, in one embodiment of the invention, an analog of an amide bond is - CO - $CF_2$ wherein the normal NH moiety is replaced by the $CF_2$ moiety.

In its broadest sense, the term "antigen" is defined as a molecule which induces the formation of an antibody. As used herein, the term "antigen" means a molecule which is inherently immunogenic, a hapten according to the invention or an immunogen which comprises a hapten according to the invention coupled to a carrier molecule by a suitable coupling moiety. Carrier molecules include, for example, keyhole limpet hemocyanin (KLH), thyroglobulin, chicken immunoglobulin, ovalbumin, bovine serum albumin (BSA), T-helper peptides, etc. "Coupling moieties" as used herein refer to biotechnological cross-linking reagents well known in the art (e.g., commercially available from Pierce, Rockford, Illinois) and include, for example, Trout's reagent, dissuccinyl suberate, etc.

The term "transition state analog" as used herein refers to an array of atoms which is designed to approximate or "mimic" the configuration of an amide bond or an ester bond as such bonds exist in a hydrolytic transition state.

The term "strained ground state" as used herein refers to an array of atoms that is designed to approximate or "mimic" one or more high energy conformations of an amide or ester bond. As an example, formula V above represents a number of such arrays.

The term "dipeptide analog" as used herein refers to a structure which comprises a transition state analog or strained ground state analog or elements of both having side chains of two amino acids which are in positions analogous to those of the dipeptide being mimicked. In other words, in a dipeptide analog, the normal amide bond (i.e., -CO-NH-) between the two amino normal amide bond (i.e., -CO-NH-) between the two amino acids has been replaced by an array of atoms as defined above. Additional amino acid residues may be incorporated to surround the dipeptide analog to form a polypeptide. Thus, the dipeptide analog replaces the peptide bond "targeted" for cleavage in the substrate molecule. In one embodiment of the invention, the moieties surrounding the dipeptide analog contain peptide bond linkages which can be altered such that the naturally occurring C=O group is replaced by NH, O, S, $CH_2$, $CF_2$ or C=S and/or the naturally occurring NH group is replaced by O, S, $CH_2$, $CF_2$, C=O or C=S. For example, the moieties can be retropeptides in which the C=O and NH groups of the amide bonds are interchanged.

The terms "some or all" refer to a portion of the target molecule including at least the amide bond to be cleaved or all of the target molecule. For example, in an embodiment of the invention, haptens designed for the purpose of eliciting antibodies to catalyze the cleavage of a specific peptide bond in a protein molecule comprising a polypeptide of many amino acid residues, the dipeptide analog corresponding to the target peptide bond need only be surrounded by not more than about eight amino acid residues. However, if the target molecule is a relatively short peptide, it is advantageous to surround the peptide bond analog with all the amino acid residues of the target molecule. Of course, one of ordinary skill in the art will realize that the desired specificity, the nature of the target molecule and other factors will dictate the ideal number of amino acid residues needed to surround the dipeptide analog.

The term "substantially corresponds" refers to moieties which are similar in charge and/or size to glycosidic bond to be cleaved or all of the target molecule. For example, in an embodiment of the invention, haptens designed for the purpose of eliciting antibodies to catalyze the cleavage of a specific peptide bond in a protein molecule comprising a polypeptide of many amino acid residues, the dipeptide analog corresponding to the target peptide bond need only be surrounded by not more than about eight amino acid residues. However, if the target molecule is a relatively short peptide, it is advantageous to surround the peptide bond analog with all the amino acid residues of the target molecule. Of course, one of ordinary skill in the art will realize that the desired specificity, the nature of the target molecule and other factors will dictate the ideal number of amino acid residues needed to surround the dipeptide analog.

The term "substantially corresponds" refers to moieties which are similar in charge and/or size to moieties in the amide bond analog, dipeptide analog or naturally occurring amino acid side chain analog. Preferably, the moieties are identical in size and charge, although such identity is not necessary for the hapten of the invention.

The term "hapten" as used herein is defined as a molecule which can act as an epitope. Haptens according to the invention incorporate a dipeptide analog according to the invention.

Physiologically acceptable salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and as well as an "analog of said side chain." The term "analog of said side chain" as used herein is defined as a side chain of a naturally occurring amino acid in which one or more moieties of the naturally occurring side chain is replaced by one or more different moieties which substantially corresponds to the naturally occurring moiety. Those side chains containing a hydroxy group can be glycosylated, phosphorylated, sulphonylated or protected by a hydroxy protecting group. The hydroxy group of any of the side chains may be protected by any number of suitable hydroxy protecting groups well known in the art. These include, for example, a tertiary butyl group.

The term "terminal amino protecting group" means any group capable of protecting the terminal amino moiety of a peptide or amino acid. Therefore, terminal amino protecting groups include acetyl, succinyl, biphenylcarbonyl, benzoyl, t-butyloxycarbonyl, carbobenzyloxy, tosyl, dansyl, isovaleryl, phthalyl, 1-adamantanesulphonyl, acetimido, benzimido, amidino, carbamyl and the functional equivalents thereof.

The term "terminal carboxyl protecting group" means any group capable of protecting the terminal carboxyl moiety of a peptide or amino acid. Terminal carboxyl protecting groups include $(C_1\text{-}C_9)$alkyl, phenyl, substituted methyl esters such as methoxymethyl and phenacyl esters, 2- substituted ethyl esters such as cyclohexyl and allyl, substituted benzyl esters such as para-methoxybenzyl and para-bromobenzyl, amides such as piperidinyl and hydrazide and functional equivalents thereof.

Haptens according to the invention contain one or more asymmetric centers and therefore exist in enantiomeric and/or diastereomeric forms. In general, the corresponding haptens according to the invention are obtained in the form of racemates or mixtures of diastereomers. If desired, techniques well known in the art for the separation of the mixtures into sterically homogeneous constituents may be used. Preparation of the optical isomers in a pure state is also possible by using sterically homogeneous starting materials.

One of ordinary skill in the art will realize that arrays containing carbonyl adjacent to difluoromethylene or fluoromethine and boron will assume a tehrahedral like configuration upon reaction with water in an aqueous environment.

Haptens that contain a central phosphorus atom include the following cyclic structure

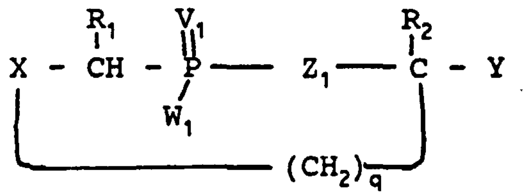

wherein $R_1$ and $R_2$ may be the same or different and each is a side chain of a naturally occurring amino acid, a hydroxy-containing side chain of a naturally occurring amino acid wherein said hydroxy group may be glycosylated, phosphorylated, sulphonylated or protected by a hydroxy protecting group, a primary amido containing side chain of a naturally occurring amino acid wherein said amido group may be glycosylated, or $C_{1\text{-}4}$ alkyl, $-CH_2\,CH(CO_2H)_2$, $-(CH_2)_2S(O)CH_3$, $-(CH_2)_2S(O)_2CH_3$, $-(CH_2)_3NH_2$ or $-(CH_2)_3ONHC(=NH)NH_2$;

X is oxygen, amino, amino protected by a protecting group selected from the group consisting of terminal amino protecting groups, amino bonded to the C-terminus of a naturally occurring amino acid to form a peptide bond, amino bonded to the C-terminus of a peptide to form a peptide bond, said amino acid and peptide being unprotected or protected by said protecting group, or X is alkene, $(C_1\text{-}C_9)$ alkyl, $(C_1\text{-}C_9)$ alkoxy phenyl, phenoxy, cyclohexyl, phenylthio, phenylsulfinyl or phenylsulfonyl wherein the aforementioned phenyl groups may be unsubstituted or mono-, di- or trisubstituted by halogen, $(C_1\text{-}C_4)$ alkyl, $(C_1\text{-}C_4)$ alkoxy or $(C_{1\text{-}}C_4)$ alkoxycarbonyl;

Y is hydrogen, carboxyl, carboxyl protected by a protecting group selected from the group consisting of terminal carboxyl protecting groups, a carbonyl bonded to the N-terminus of a naturally occurring amino acid to form a peptide bond, carbonyl bonded to the N-terminus of a peptide to form a peptide bond, said amino acid and peptide being protected or unprotected by said protecting group, or Y is $(C_1\text{-}C_9)$ alkyl, $(C_1\text{-}C_9)$ alkoxy, phenyl, phenoxy, cyclohexyl, phenylthio, phenylsulfinyl or phenylsulfonyl wherein the aforementioned phenyl groups may be unsubstituted or mono-, di- or trisubstituted by halogen, $(C_1\text{-}C_4)$ alkyl, $(C_1\text{-}C_4)$ alkoxy or $(C_1\text{-}C_4)$ alkoxycarbonyl; and

wherein each of $R_1$, $R_2$, X and Y may be unbound or bound to one or more of $R_1$, $R_2$, X and Y and if bound, then by a covalent bond or a linker moiety selected from the group consisting of $-(CH_2)_S\text{-}S\text{-}S\text{-}(CH_2)_t\text{-}$, $-(CH_2)_t\text{-}$, -S-(CH$_2$)t-S-, $-(CH_2)_S\text{-}S(CH_2)_t\text{-}$, $-(CH_2)_S\text{-}CH = CH\text{-}(CH_2)_t\text{-}$, $-(CH_2)_S\text{-}NH\text{-}CO\text{-}(CH_2)_t\text{-}$, $-(CH_2)_S\text{-}NH\text{-}(CH_2)_t\text{-}$ and $-(CH_2)_S\text{-}phenyl\text{-}(CH_2)_t\text{-}$;

$W_1$ is OH, $NH_2$, SH or H; $V_1$ is O or S;

$Z_1$ if unbound is O, NH, $CH_2$ or S and if bound is N or CH;

s and t may be the same or different and each is 0 or an integer from 1 to 10 unless the linker moiety is $-(CH_2)_t\text{-}$ in which case t is an interger from 1-10.

q is 0 or an integer from 1 to 10.

Especially preferred are those in which $V_1$ is 0, $W_1$ is OH, SH or H, $Z_1$ is O, NH or $CH_2$ and q is 0 or 1.

Haptens that contain a central sulphur atom include compounds in which $Z_2$ is NH. Such a hapten is aminomethanesulfonamidylalanyl acid. Other haptens containing a central sulfur atom include the following cyclic structure

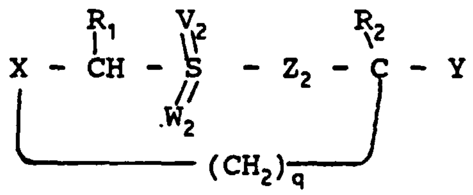

wherein $R_1$, $R_2$, X, Y, $V_2$, $W_2$ $Z_2$ and q are defined as above. Especially preferred are those in which $V_2$ and $W_2$ are O or a lone pair of electrons, $Z_2$ is O, $CH_2$ or NH if unbound and if bound is N or CH, and q is 0 or 1.

Haptens that contain a central carbon atom include compounds in which $V_3$ is OH and $Z_3$ is $CH_2$, $V_3$ is $NH_2$ and $Z_3$ is $CH_2$. Other haptens containing a central carbon atom include the following cyclic structure

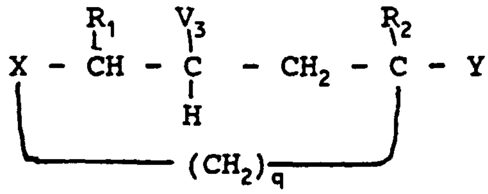

wherein $R_1$, $R_2$, X, Y, $V_3$ and q are defined as above. Especially preferred are those wherein $V_3$ is OH or $NH_2$ and q is 0 or 1.

Haptens that contain a central carbonyl include the compound in which $Z_4$ is $CF_2$. Such a preferred hapten is 5- (serinyl)amino 3,3-difluoro 4-oxo 6-hydroxy heptanoic acid. Other haptens contain a central carbonyl include the following cyclic structure

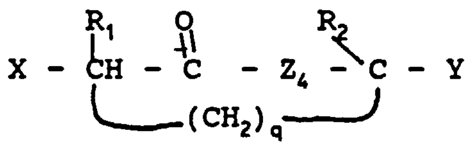

wherein $R_1$, $R_2$, X, Y, $Z_4$ and q are defined as above. Especially preferred are those in which $Z_4$ is $CF_2$ and q is 0 or 1.

Haptens that contain a central silicon atom include compounds in which $V_4$ is OH or $NH_2$ and $Z_5$ is O, and $V_4$ is OH or $NH_2$ and $Z_5$ is $CH_2$ or CH. A preferred hapten is 3-(aminomethyldihydroxysilyl) propionic acid. Other haptens containing a central silicon atom include the following cyclic structure

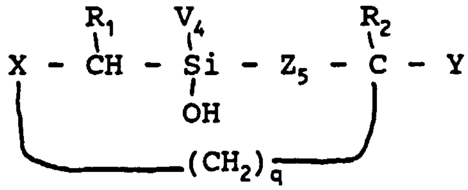

wherein $R_1$, $R_2$, X, Y, $Z_5$ and q are defined as above. Especially preferred are those in which $V_4$ is OH, $Z_5$ is $CH_2$ and q is 0 or 1.

Other preferred haptens include those in which $R_1$ is selected from the group consisting of $CH_2OH$ and $CH(OH)CH_3$; $R_2$ is selected from the group consisting of $CH(OH)CH_3$ and $CH_2CONH_2$; X is selected from the group consisting of amino bonded to the C terminus of alanine, amino bonded to the C terminus of serine in the dipeptide Ala-Ser, amino bonded to the C terminus of threonine in the tripeptide Ala-Ser-Thr and amino bonded to the C terminus of theronine in the polypeptide Ala-Ser-Thr-Thr; and Y is selected from the group consisting of carbonyl bonded to the N terminus of threonine in the polypeptide Thr-Thr-Asn-Tyr-Cys, carbonyl bonded to the N terminus of threonine in the polypeptide Thr-Asn-Tyr-Cys, carbonyl bonded to the N terminus of asparagine in the tripeptide Asn-Tyr-Cys and carbonyl bonded to the N terminus of tyrosine in the dipeptide Tyr-Cys. Still other preferred haptens include those in which $R_1$ is $CH(OH)CH_3$, $R_2$ is H, X is amino bonded to the C terminus of serine in the polypeptide Cys-Leu-Arg-Tyr-Ser and Y is carbonyl bonded to the N terminus of threonine in the tripeptide Thr-Val-Cys.

One application of catalytic antibodies having site-specific proteolysis capabilities is in the immunotherapy of viral infection. Viruses utilize their external coat proteins to attach to cellular receptors and invade the cell after attachment. For example, human immunodeficiency virus (HIV) uses a portion of the gp120 protein at its surface to attach to CD4 receptors on lymphocytes. The sequence for this cell attachment has been napped to a region on the viral protein. With this information, antibodies can be generated by the methodology described in this invention to bind to this peptide sequence and cleave it in a site-specific manner.

However, such antibodies preferably bind to the "native" sequence in the protein as opposed to a linear sequence (which would occur in a denatured protein). Thus, the antigenic determinants or epitopes in the "native" protein are often conformational (i.e., three-dimensional) rather than random linear arrangements. Here again, knowledge of epitopes on the protein is important in the design of antibodies having paratopes that can induce modifications of such epitopes.

Therefore, haptens according to the invention are designed to have the same structural features of the epitopes, rather than random conformations. These structural features can be adopted by simple linear peptides, the lowest energy conformer being the preferred structure in solution. Secondary structural features may be introduced by cross-linking of amino-acid side-chains or the use of β-turn mimetics. Conformationally constrained haptens incorporating structures which are compatible with the epitope in the native protein may be essential for inducing the correct motif within the tertiary structure of the catalytic antibody hyper-variable binding region. The advantages of conformationally constrained haptens are that they mimic the native structure in the protein and tend to mimic regions of the protein which are susceptible to cleavage. Accordingly, in the structural formulae shown above for the haptens of the invention, the substituents $R_1$, $R_2$, X and Y may be bound to one or more of the remaining substituents $R_1$, $R_2$, X and Y by a covalent bond or a linker moiety as defined above. Likewise, in formulae I, the substituents $Z_1$-$Z_6$ may be covalently bonded to the linker moiety by substitution at an appropriate atom in the linker moiety.

Preferred conformationally constrained haptens are represented by formula IA

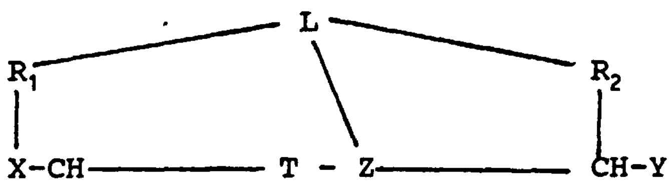

(IA)

wherein

$R_1$, $R_2$, X and Y and are as defined with reference to formula I above;
T is

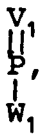 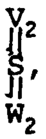 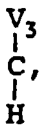 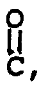 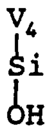 or 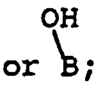

$V_1$, $V_2$, $V_3$, $V_4$, $W_1$ and $W_2$ are as defined with reference to formula I above;
Z is $Z_1$, $Z_2$, $Z_3$, $Z_4$ or $Z_5$ as defined with reference to formula I above; and
L is N or CH in the linker moiety as defined above. These include pyradazinedione analogs, shown in Fig. 3, bicyclic β-turn and rigid tricyclic analogs, shown in Fig. 4 and macrocyclic β-turn analogs in Fig. 5. On comparison of the haptens set forth in Figs. 3, 4 and 5 and the general formula IA, the skilled artisan will readily appreciate that the side chains $R_1$ and $R_2$ are $-CH_2-$ or $-CH_2CH_2-$ and these side chains $R_1$ and $R_2$ are joined by the linker moiety "L" which is $-CH_2-CO-N-CH_2-$,$-CH_2-N-CH_2-$, -CH2-CH-S- or -ortho-phenyl-CH- $CH_2-$. The nitrogen atom corresponding to the Z substituent is covalently bonded to the aforementioned linker moieties by substitution at an appropriate atom in the linker moiety, i.e., at the nitrogen atom in the first two linker moieties listed, at the carbon atom adjacent to the sulfur atom in the third linker moiety listed and at the carbon atom adjacent to the phenyl ring in the fourth linker moiety listed. While in Figs. 3, 4 and 5 phosphonamidate analogs are depicted, it is to be understood that any of the other tetrahedral carbon mimics according to the invention (e.g., $-SO_2-$, $-CHNH_2-$, CHOH-,$-Si(OH)_2-$, etc.) may also be used.

A further way in which conformational constraint can assist the cleavage reaction itself is where an amino acid side chain is constrained in a conformation which allows it to directly participate in peptide bond cleavage. Such a cyclization is formally equivalent to the process believed to occur during the hydrolysis of peptide bonds in which aspartic acid or asparagine is present on the N-terminal side of the bond to be cleaved. As a result of the assistance of the side chain during the cleavage, aspartic acid-X or asparagine-X bonds (where X is any amino acid but particularly glycine or proline) are known to undergo background hydrolysis at enhanced rates compared with other types of peptide bond. Indeed, such "metastable" bonds and their susceptibility to intramolecular assisted cleavage are well described in the literature (13,14). Thus, in the aforementioned haptens, the cyclic tautomer would represent an ideal mimic of the transition state for the intramolecular cleavage of aspartyl-X peptide bonds. Those skilled in the art will readily see that this concept can be extended to include the similar side Such a cyclization is formally equivalent to the process believed to occur during the hydrolysis of peptide bonds in which aspartic acid or asparagine is present on the N-terminal side of the bond to be cleaved. As a result of the assistance of the side chain during the cleavage, aspartic acid-X or asparagine-X bonds (where X is any amino acid but particularly glycine or proline) are known to undergo background hydrolysis at enhanced rates compared with other types of peptide bond. Indeed, such "metastable" bonds and their susceptibility to intramolecular assisted cleavage are well described in the literature (13,14). Thus, in the aforementioned formulation of hapten of formula I, the cyclic tautomer would represent an ideal mimic of the transition state for the intramolecular cleavage of aspartyl-X peptide bonds. Those skilled in the art will readily see that this concept can be extended to include the similar side chains of asparagine as well as the related side chains of glutamic acid and glutamine. It will also be apparent to one skilled in the art that similar cyclic forms to those described above may also be generated using hapten design in which the transition state is mimicked by phosphorus or sulfur analogs.

The haptens of the invention can also take on a configuration mimicking that of the native β-turn or "hairpin" configuration of proteins by the formation of disulphide bridges between sulfur containing amino acid side chains which are incorporated into the hapten. Formation of disulphide bridges also promotes hydrogen bonding interactions. Disulphide bridge formation can be achieved by chemical methodology well known in the art.

The synthesis of 3-(N-carbobenzyloxymethyl)sulfonyl-2-methyl-propanoic acid [**4**], a hapten containing a central sulfone group, is shown schematically in Fig. 7. The synthesis of both 3-mercapto-2-methylpropanoic acid [**1**] (15) and benzyl N-(bromomethyl)carbamate [**2**] (16) have been described in the literature. Reaction of the disodium salt of [**1**] with the bromoderivative [**2**] produces the sulfide compound [**3**]. Oxidation with periodate of [**3**] affords the desired sulfone product [**4**]. The synthesis is set forth in more detail in the examples below.

The synthesis of 3-difluoro-5-(6-maleimidohexanoyl)amino-2-methyl-4-oxo-6-phenylhexanoic acid [**12**], a

difluoroketone hapten, is shown schematically in Fig. 8. The difluoro acid [**1**] was prepared by reaction of crotyl alcohol with tetrafluoroethylene in the presence of catalytic amount of sodium hydride and the resultant intermediate was treated with n-butyllithium. The difluoro acid [**1**] was converted to its silver salt [**2**] with aqueous silver oxide. After drying the salt over phosphorus pentoxide, it was converted to the anhydride [**3**] by reacting with oxalyl chloride. The anhydride [**3**] was then treated with a p-phenylbenzoyl group to protect the N-terminus of amino acid.

The corresponding oxazolinone [**5**] was prepared by the reaction of D,L-phenylalanine with 4-biphenylcarbonylchloride and resulting protected phenylalanine [**4**] was reacted with 1-ethyl-3-(3-dimethylaminopropyl)carbodiimide hydrochloride. Treatment of oxazolinone [**5**] with the anhydride [**3**] at 60°C for 40 h followed by the addition of oxalic acid at 110°C for 15 min produced the difluoroketone [**6**]. The ketone [**6**] was reduced to the corresponding alcohol [**7**] with sodium borohydride and treatment of [**7**] with excess 3 % sodium-amalgam in methanolic phosphate buffer at pH 6-7 gave the amine compound [**8**]. The liberated amine compound [**8**] was converted to benzyloxycarbonylamino compound [**9**] with N-(benzyloxy-carbonyloxy)succinimide. Compound [**9**] is oxidized with Dess-Martin periodinane and treatment of resulting difluoroketone [**10**] with ozone at -78°C followed by the addition of dimethylsulfide and then Jones oxidation yields the acid [**11**]. The final product [**12**] is prepared by the removal of the benzyloxycarbonyl group by catalytic hydrogenation followed by the reaction of the liberated amine compound with 6-maleimidohexanoic acid anhydride to give the compound [**12**]. The synthesis is described in more detail in the examples below.

The synthesis of 5-[N-Benzyloxycarbonylmethyl)amido]-3,3-difluoro-2-methyl-4-oxo-6-phenylhexanoic acid [7], a retroamidodifluoroketone hapten is shown schematically in Fig. 9. The hydrocinnamic acid ester [**2**] and difluoroacid ester [**4**] were prepared by the reaction of diazomethane with hydrocinnamic acid [**1**] and difluoro acid [**3**] respectively. Treatment of ester [**2**] with LDA followed by difluoroacid ester [**4**] gives corresponding difluoroketone [**5**]. After hydrolysis of ester [**5**] with $K_2CO_3$, compound [**6**] is obtained by coupling with glycine benzylester. Ozonolysis of compound [**6**] followed by Jones oxidation should produce a final product [**7**]. The synthesis is set forth in more detail in the examples below.

As noted earlier, the invention is also directed to conformationally constrained haptens which mimic the native configurations of proteins. Fig 10 is a type I β-turn where the carbonyl group of the residue is hydrogen-bonded to the amino group of the residue C+3.

The synthesis of 5-[N-Benzyloxycarbonylmethyl)amido]-3,3-difluoro-2-methyl-4-oxo-6-phenylhexanoic acid [7], a retroamidodifluoroketone hapten according to formula IV, is shown schematically in Fig. 9. The hydrocinnamic acid ester [**2**] and difluoroacid ester [**4**] were prepared by the reaction of diazomethane with hydrocinnamic acid [**1**] and difluoro acid [**3**] respectively. Treatment of ester [**2**] with LDA followed by difluoroacid ester [**4**] gives corresponding difluoroketone [**5**]. After hydrolysis of ester [**5**] with $K_2CO_3$, compound [**6**] is obtained by coupling with glycine benzylester. Ozonolysis of compound [**6**] followed by Jones oxidation should produce a final product [**7**]. The synthesis is set forth in more detail in the examples below.

As noted earlier, the invention is also directed to conformationally constrained haptens which mimic the native configurations of proteins. Fig 10 is a type I β-turn where the carbonyl group of the residue is hydrogen-bonded to the amino group of the residue C+3. It is possible with a change of conformation with the same hydrogen bond intact to form other types of β-turns such as type II or type III ($3.0_{10}$ helix). In all of the different types of β-turns it is desirable to replace the hydrogen bond for a covalent link such as the N-amino- methylamidinium link as shown in Fig. 11. The molecule is now locked into a β-turn. Therefore, replacing the amide bond between residues $R_1$ and $R_2$ by a transition state analog gives a conformationally constrained hapten as shown in Fig. 12 useful for eliciting catalytic antibodies.

The synthesis of the above mimetic of a β-turn (Fig. 12) is in two parts: first, the preparation of the dithioester (7) as shown in Fig. 13 (Scheme 1); and second, the formation of the ten membered ring as shown in Fig. 14 (Scheme 2). The method for preparing the dithioester (7) follows essentially a literature procedure (17).

The BOC(t-butyldicarbonate)-protected glycine (1) is first converted to the triethylamine salt by treatment with triethylamine and is then transformed to the corresponding piperidide (2) by the reaction of the BOC-protected glycine triethylamine salt with Lawesson's reagent (LR) and piperidine. Removal of the BOC group by trifluoroacetic acid followed by the addition of FMOC-Cl gives the FMOC-protected glycylpiperidide (4). Lawesson's reagent is next used as a thionation reagent to form the corresponding thiopiperidide (5). The thiopiperidide is S-methylated by reaction with an excess of methyl iodide in THF for 12 hrs. to give (6). Thiolysis using hydrogen sulphide at 0°C yields the dithioester (7).

The formation of the ten membered ring compound (Scheme 2) begins with the reaction of bromomethylphthalimide with methylamine to give the secondary amine (8). Addition of 2-[(t-butoxycarbonyloxyimino)-2-phenylacetonitrile] followed by removal of the phthalimide group with hydrazine yields the primary amine (10). Thioacetylation of (10) with the dithioester (7) from Scheme 1 followed by activation with methyl iodide and condensation with (L)-2-aminophosphonamide acid (13) gives compound (14).

The polyamide gel resin (16) functionalized as a succinimide ester is prepared by reacting the polyamide gel resin functionalized as sarcosine methyl ester (commercially available as Pepsyn™ from CRB Ltd.) with ethylenediamine followed by Lomant's reagent as shown in Fig. 15 (Scheme 3). Compound (17) is prepared by first removing the

FMOC group from compound (14) with pyridine in DMF followed by reaction with the activated resin (16). Removal of the BOC group with trifluoroacetic acid followed by internal cyclization using IIDQ gives the final product (19). The presence of the disulphide linker in product (19) allows the release of the product into free solution by reductive cleavage using dithiothreitol under basic conditions. The product may then be purified and analyzed before attaching to a carrier protein using the bifunctional linker, sulfosuccinimidyl 4-(N-maleimidomethyl)cyclohexane-1-carboxylate.

The utility of the antigens of this invention coupled with appropriate screening procedures and reiterative thermodynamic perturbation studies of transition-state structures and free-energies of interaction with catalytic groups provide a methodology for production of catalytic antibodies by a rational design approach.

The invention also is directed to catalytic antibodies which are elicited by antigens comprising haptens according to the invention. These antibodies may be monoclonal or polyclonal but are preferably monoclonal and may be in the form of purified immunoglobulins (IgG, IgM, IgA, IgD or IgE) or antibody fragments, such as Fab, $F(ab')_2$, $F_v$, etc., of immunoglobulins.

A catalytic antibody in accordance with the invention is a substance which is capable of changing the rate of a chemical reaction, all other conditions (e.g., temperature, reactant/substrate concentration, etc.) being the same and which does not enter into the chemical reaction and therefore is not consumed in the reaction. It is also a substance which exhibits the capability of converting multiple moles of reactant/substrate per mole of catalytic antibody; which, from a mechanistic viewpoint, binds the reactant/substrate, effects the accelerated conversion of the reactant/substrate to the product and then releases the product; and which changes the rate of the chemical reaction without shifting the position of the equilibrium. The aforementioned definitions are characteristics of ideal catalysts. However, in practice, even the best of catalysts become poisoned or deactivated by contamination in the reaction system or as a result of chemical or physical destruction during the reaction process. For reasons well known in the art, the true operation of a catalyst may be obscured by components of the reaction system or by the condition of the reaction environment.

The art has adopted certain working definitions to express catalytic activity. These expressions are [1] $k_{cat}$, or "turnover" and [2] $k_{cat}/k_{uncat}$, the "rate enhancement factor". Turnover indicates the number of molecules of reactant/substrate which can be converted to product per mole of catalytic antibody per unit time. For example, if a molecule exhibits a turnover of $10^3$ molecules of substrate per minute and the molecule maintains its catalytic activity for 24 hours at room temperature and at its optimal pH, each molecule of catalyst would then make a total of $1.4 \times 10^6$ conversions, indicating its catalytic behavior. This total conversion is to be distinguished from the total conversion in a stoichiometric reaction, which will never exceed 1.0, no matter how long the reaction is carried out. The rate enhancement factor is a dimensionless number which expresses the rate of reaction in the presence of catalyst to the rate of reaction in the absence of catalyst, all other reaction conditions (e.g., reactant concentration, temperature, etc.) being equal.

Catalytic antibodies according to the invention may be elicited through both in vitro and in vivo techniques. The term "elicited" as used herein means elicitation of catalytic antibodies by antigens according to the invention through both in vitro and in vivo techniques. However, the skilled artisan will readily appreciate that when in vitro elicitation is involved, the haptens according to the invention, by themselves, may be used to elicit the catalytic antibodies. However, when elicitation is achieved through in vivo techniques, it is understood that immunogens comprising haptens complexed to a suitable carrier molecule are used to elicit the catalytic antibodies. Another aspect of the invention is directed to a method for producing antibodies which can catalyze a chemical reaction of interest and which are elicited through in vitro or in vivo techniques by an antigen. The antigen comprises a hapten according to the invention. The haptens are designed to elicit the appropriate hypervariable binding region in an antibody molecule to express intrinsic binding energy for the transition- state of a chemical reaction, particularly a hydrolytic reaction. Arrangement of amino-acid side chains generated in the combining-site will be appropriate for performing chemical modification of an epitope of interest. Additional improvements in catalytic efficiency can be achieved by site-directed mutagenesis.

Broadly, the method comprises exposing cells capable of producing antibodies to the antigen and thereby generating antibody producing cells; hybridizing the antibody producing cells with myeloma cells and thereby producing a plurality of hybridoma cells each producing monoclonal antibodies; and screening the plurality of monoclonal antibodies to identify a monoclonal antibody which catalyzes the chemical reaction of interest. The monoclonal antibody so identified may then be replicated, again by either in vivo or in vitro techniques, to obtain a quantity sufficient to catalyze the chemical reaction of interest.

The detection of antibodies with the desired catalytic activity and specificity is achieved by screening the hybridomas once they have been elicited. For example, screening may be achieved by high performance liquid chromatography (HPLC) or spectrophotometric methods (ELISA). Catalytic monoclonal antibodies are elicited "in vivo" by modification of the technique disclosed by Koprowski et al. in U.S. Patent No. 4,196,265, issued April 1, 1980, which is hereby incorporated by reference. The details of that process are known in the art. A series of monoclonal antibodies directed to a specific molecule are prepared under suitable conditions. This involves first immunizing BALB/C mice with an appropriate antigen. The antigen comprises a hapten according to the invention bound to a peptide or other carrier molecule.

Antibody-producing lymphocytes are then removed from the spleens of the immunized mice and hybridized

with myeloma cells such as SP2/0 cells to produce hybridoma cells. These hybridoma cells are then plated in the wells of microtiter plates. The series of monoclonal antibodies being produced by the hybridoma cells is screened under appropriate conditions to identify monoclonal antibodies which catalyze the desired reaction under appropriate conditions. Alternatively, the medium may be tested for antibodies that bind to the immunogen and the hybridomas producing these antibodies then expanded in tissue culture or grown in vivo. Screening may be conveniently accomplished by treating a standardized solution of the reactant with an aliquot of medium withdrawn from a microtiter well and measuring the presence of the desired product by conventional instrumental methods. This measurement may be readily conducted, for example by spectrophotometric methods or by gas-liquid or high pressure liquid chromatography. By comparison with standardized samples of the desired product or reactant, rates of reaction may be quantified. In this manner, wells containing hybridoma cells producing catalytic monoclonal antibodies are identified. The selected hybridoma cells are then cultured to yield colonies.

These colonies may be further propagated in in vitro or in vivo systems. In the latter case, mice such as syngeneic BALB/C mice are inoculated intraperitoneally with the selected hybridoma cells and produce tumors, generally within two or three weeks. These tumors are accompanied by the production of ascites fluid which contains the desired monoclonal antibodies. The monoclonal antibodies are then separately recovered from the ascites fluid by conventional methods such as ultrafiltration, ultracentrifugation, dialysis and immunoffinity chromatography.

The invention is also a method for catalyzing the cleavage or formation of an amide (peptide) or ester bond in a molecule. The molecule can be a natural or synthetic peptide or protein. Target molecules include bimolecules which are defined as any molecule which affects a biological system in vivo or in vitro. Biomolecules may be synthesized by cells or chemically synthesized. Examples of biomolecules include proteins, glycoproteins, peptides, steroids, and maleic acid. Also included are synthetic organic analogs of peptides, steroids, maleic acid, etc. Pharmaceutically active compounds such as theophylline, caproin, cyclosporin, etc., are also considered to be biomolecules. The method comprises contacting a molecule containing one or more amide (peptide) or ester bonds with an effective amount of a catalytic antibody which has been elicited by an antigen comprising a hapten of the invention.

In accordance with the invention, the separately recovered monoclonal antibodies are contacted with a molecule under suitable conditions permitting the formation of a complex between the monoclonal antibody and the molecule. In general, the concentration of the catalytic antibodies used is less than the equivalent concentration of the target molecule and may be in the picomolar range. The antibodies should function under normal physiologic conditions in vivo. The skilled artisan will appreciate that the conditions suitable for complex formation may vary depending on the particular molecule and monoclonal antibody under consideration.

Accordingly, the methods of this invention may be practiced under a variety of reaction conditions, in vivo and in vitro, as long as the monoclonal antibodies are not prevented from complexing with the molecules or otherwise rendered inactive. More specifically, suitable conditions for complex formation encompass solution phase and emulsion reaction systems including a protic solvent, preferably water, maintained at a pH value between about 6.0 and about 8.0, preferably between about 6.0 and about 7.5 and at a temperature from about 4°C to about 50°C, preferably from about 20°C to about 45°C. The ionic strength, $\nu = 1/2\ \Sigma\ c_i z_i^{\ 2}$, where c is the concentration and z is the electronic charge of an ionic solute, should be maintained at a value below about 2.0 moles/liter, preferably between 0.1 and 1.5 moles/liter. The method of this invention may be carried out at reduced or elevated pressure, but preferably is practiced at ambient pressure. In addition to solution phase and emulsion reaction systems, suitable conditions also include the use of support materials to which the monoclonal antibody is attached. Such support materials are well-known to those of ordinary skill in the art as are methods for attaching monoclonal antibodies to them.

Catalytic antibodies elicited with the antigens of the invention may be useful in the treatment of autoimmune disease, cancer and thrombolytic disease. The catalytic antibodies may also be useful for treatment of cardiovascular disease eliminating high density lipoproteins and for the detoxification of bacterial endotoxins. Vaccines comprising synthetic peptides optionally linked to carrier proteins, for example, against foot and mouth disease (FMD) and E. coli enterotoxin, have been proven efficacious in recent years.

Oligo peptides having variable lengths with sequences from the receptor-binding regions of viruses which employ a specific cellular receptor for penetration of the host cell and having a transition state analog dipeptide isostere in a critical region of the sequence induce on immunization, optionally after coupling to a suitable carrier protein, catalytic antibodies that cleave the viral coat protein and prevent virus penetrating the cell. The dimensional structure of Rhino 14 and Polio 1 virus particles has been charted by X-ray scattering. Regions have been identified which are binding sites to cellular receptors. The region of the human immunodeficiency virus type 1 (HIV I) critical for interaction with the CD4 receptor on T-lymphocytes has been located and mapped to sequences in the gp120 goat protein. Thus, in one embodiment of the invention, oligo peptides are used which contain partial sequences from the envelope proteins of viruses critical for host cell attachment and a transition-state dipeptide isostere selected from the haptens according to the invention. The resultant peptide analogs are used to induce catalytic antibodies that inactivate viruses by proteolysing segments (epitopes) of the viral coat protein critical for infectivity. Preferably, oligopeptides are used having sequences from the receptor binding region of retroviruses e.g., HIV I, HIVII and picorna viruses, e.g., Rhino 14, viral

polypeptides, inflammatory proteins, anaphylactic proteins, lymphokines, cytakines and other polypeptide mediators of host infection or toxic syndromes.

The invention will be more fully described and understood with reference to the following illustrative examples.

## Example 1

### Synthesis of N-[N-(1-carboxy-2-phenyl)ethylaminosulfonylacetyl]glycine

The reaction sequence is shown in Fig. 6.

### Sodium ethyl sulfoacetate [1]

A solution of sodium sulfite (126.0g, 1.00 mol) in water (400 mL) was cooled and stirred while a solution of ethyl bromoacetate (167.0g, 1.00 mol) in ethyl alcohol (200 mL) was added dropwise. After the addition was complete the mixture was heated briefly to 50°C, then concentrated to dryness (two portions of ethyl alcohol/benzene were added and stripped to aid in the removal of water). The solid residue was extracted with boiling 2:1 acetic acid/ethyl acetate (total 900 mL) and the hot solution was filtered through Celite and chilled overnight. The crystallized product was filtrated as a white solid and subsequent crops were obtained by diluting the mother liquors with additional ethyl acetate. The combined products gave 152g (80 %) of [**1**] as a white solid. (mp. ca. 158°C (dec)).

### Ethyl chlorosulfoacetate [2]

The sodium salt [**1**], (19.0g. 100 mmol) and phosphorus pentachloride (23.0g, 110 mmol) were separately pulverized then combined in a flask equipped with a condenser and drying tube. After swirling a few minutes, a reaction was occurred: after the exothermic reaction subsided the flask was warmed on a steam bath for 45 min, then the phosphoryl chloride was stripped in vacuo. A portion of benzene (50 mL) was added and the resulting solution was filtered through Celite and evaporated to give 15.9g (85 %) of [**2**] as a clear oil. This compound was used for next reaction without further purification.

### Ethyl N-(1-t-butoxycarbonyl-2-phenyl)ethylaminosulfonylacetate [3]

To a solution of [**2**], (15.9g, 85.0 mmol) in benzene (65 mL) is added a solution of L-phenylalanine t-butylester (20.1g, 91.0 mmol) and triethylamine (15 mL, 108 mmol) in benzene (50 mL) dropwise at 0°C. After addition is complete, the mixture is warmed gently for 5 min, then is cooled and filtered, and the filtrate is washed with water (50 mL), dilute hydrochloric acid (2x50 mL), aqueous sodium hydrogen carbonate (2x50 mL), and aqueous sodium chloride (50 mL). After drying ($MgSO_4$), the solvent is removed in vacuo to give crude [**3**] as an oil. Crystallization from benzene/n-hexane followed by recrystallization from the same solvent gives pure [**3**] as a solid.

### N-(1-t-butoxycarbonyl-2-phenyl)ethylaminosulfonylacetic acid [4]

The ester [**3**], (18.5g, 50.0 mmol) is refluxed in a solution of potassium hydroxide (7.0g, 125 mmol) in water (50 mL) and ethyl alcohol (20 mL) for 2.5 h. Charcoal is added, the solution is heated to boiling for 5 min, filtered through Celite, washed with ether (100 mL), acidified with hydrochloric acid, and extracted with ether (3x100 mL). The ether extract is washed with water (3x100 mL), dried ($MgSO_4$), and concentrated to give crude [**4**] which is crystallized from benzene to give pure [**4**] as a solid.

### N-[N-(1-t-butoxycarbonyl-2-phenyl)ethylaminosulfonylacetyl]glycine benzylester [5]

To a solution of [**4**], (5.00g 14.6mmol) in dichloromethane (20 mL) is added a solution of 1,3-dicyclohexylcarbodiimide (1.50g, 7.30mmol) in dichloromethane (20 mL) at room temperature under argon. It is stirred for 30 min and the resulting precipitate is filtered and the filtrate is concentrated in vacuo. The resulting solid is dissolved in DMF (20 mL) and a solution of glycine benzylester (1.19g, 7.30 mmol) in DMF (10 mL) followed by a solution of N-methylmorpholine (0.88 mL, 8.00 mmol) in DMF (5 mL) are added dropwise. The reaction mixture is stirred for an hour and diluted with ethyl acetate (100 mL). The organic layer is washed with 1 N aqueous hydrochloric acid (2x50 mL), saturated sodium bicarbonate (2x50 mL), and water (2x50 mL), dried (MgSO4), and concentrated in vacuo to give [**5**] as an impure solid. Pure product [**5**] is obtained by chromatography of the crude mixture with 100 g of silica gel and eluting with methyl alcohol/chloroform.

**N-[N-(1-carboxy-2-phenyl)ethylaminosulfonylacetyl]glycine [6]**

Into a Parr flask flushed with argon containing 0.30g of 10% palladium on charcoal catalyst is added a solution of [**5**], (3.00g. 6.12mmol) in ethyl acetate (30 mL). The mixture is shaken under 30 psi of hydrogen at room temperature over a period of 2 h. The mixture is filtered through Celite and trifluoroacetic acid (2 mL) is added into the filtrate. It is stirred at room temperature for 6 h. Excess solvents are evaporated in vacuo and the crude mixture is purified by chromatography using 50g of silica gel and eluting with ethyl alcohol/chloroform to give product [**6**] as a solid.

**Example 2**

**Synthesis of 3-(N-Carbobenzyloxyaminomethyl)sulfonyl-2-methyl-propanoic acid**

The reaction sequence is shown in Fig. 7.

**3-(N-Carbobenzyloxyaminomethyl)thio-2-methylpropanoic acid [3]** To a solution of 3-mercapto-2-methylpropanoic acid [**1**] (0.24 g , 2 mmol) in methanol (5 mL) is added sodium methoxide (0.216 g , 4 mmol) followed by benzyl N-bromomethylcarbamate [**2**] (0.488 g , 2 mmol). When the reaction is complete, $CH_2Cl_2$ (50 mL) is added and the solution is washed with aqueous 0.05 M HCl (50 mL). The organic layer is dried ($MgSO_4$), evaporated and purified by silica gel chromatography, using methyl alcohol/dichloro- methane to give the sulfide product [**3**].

**3-(N-Carbobenzyloxyaminomethyl)sulfonyl-2-methylpropanoic acid [4]**

To a stirred solution of [**3**] (0.151g , 0.535 mmol) in methanol (20 ml) is added a solution of $NaIO_4$ (1.145g, 5.35 mmol) in water (5 mL). The reaction mixture is stirred at room temperature for 60 h. Over the course of the reaction, additional $NaIO_4$ (3.45g , 6.1 mmol) is added periodically until no starting sulfide [**3**] is present. The reaction mixture is filtered and the filtrate is concentrated to give a solid. This impure product is purified using silica gel chromatography with methyl alcohol/dichloromethane to give the final product [**4**].

**Example 3**

**Synthesis of [(6-Maleimido)hexanoyl]amino](2-phenylethyl)hydroxyphosphinyl D-alanine**

The reaction sequence is shown in Fig. 16.

**Diphenyl (benzyloxycarbonylamino)-2-phenylethyl phosphonate. [1]**

To a solution of phenylacetaldehyde (35 ml, 300mmol) in glacial acetic acid (30ml), was added triphenylphosphite (52.4g, 200mmol) and benzyl carbamate (30.2g, 200mmol). The mixture was stirred at room temperature for 1h, and then heated at 85° for 2h. Volatiles were removed *in vacuo*, and the oily residue was dissolved in methanol (350ml) and allowed to crystallize at -20° overnight. The resulting crystals were collected by filtration and recrystallized by dissolving in hot chloroform (100ml) and adding methanol (300ml). After cooling to -20° for 3h, the resultant crystals were filtered from the mother liquors and dried. Yield: 34g (34.8%). N.M.R. and I.R. data were in accord with the assigned structure.

**Dimethyl (benzyloxycarbonylamino)-2-phenylethyl phosphonate. [2]**

To a solution of diphenyl-2(benzyloxycarbonylamino)-3-phenyl phosphonate (8.2g. 15 mmol) in dry methanol (450ml) was added potassium fluoride dihydrate (4.02g, 180mmol) and 18-crown-6 (0.1g). The solution was heated under reflux for 30min. After cooling volatiles were removed *in vacuo* and the residue dissolved in dichloromethane (300ml). The solution was washed with sodium hydroxide (50ml of a 3M solution) and the organic layer dried ($MgSO_4$) and evaporated to yield an oil which solidified upon standing. Yield: 6.1g (100%). N.M.R. and I.R. data were in accord with the assigned structure.

**Methyl hydrogen (benzyloxycarbonylamino)-2-phenylethyl phosphonate [3]**

To a solution of dimethyl-2(benzyloxycarbonylamino)-3-phenyl phosphonate (2.65 g, 7.3 mmol) in dioxan (10ml) was added sodium hydroxide solution (8 ml of a 1M solution, 8 mmol) and the solution stirred overnight. Solvent was removed *in vacuo* and the residue redissolved in water (5 ml) and acidified to pH 1 with 1M hydrochloric acid. The white precipitate was obtained by filtration and dried in vacuo. Yield: 1.86 g (73%). N.M.R. and I.R. data were in accord

with the assigned structure.

**[(benzyloxycarbonylamino)-2-phenylethyl methoxyphosphinyl] D-alanine methyl ester [4]**

To a solution of methyl hydrogen 2-(benzyloxycarbonylamino)-3-phenylpropyl phosphonate (4.009g, 11.48 mmol) in dry $CHCL_3$ (15 ml) cooled to 0°, was added $SOCl_2$ (0.92ml, 12.63 mmol) dropwise. After stirring for 3h, volatiles were removed *in vacuo*, and the resulting viscous oil was redissolved in dry $CHCL_3$ (10ml) and cooled to -30°. To a suspension of D-alanine methyl ester in dry chloroform (2 ml) was added $Et_3N$ (3.54 ml, 25.2mmol). The resultant suspension was added to the phosphochloridate solution with the aid of additional $CHCL_3$ (5 ml). The mixture was allowed to warm slowly to room temperature, stirred for 2h, diluted with EtOAc (200ml), washed with 1M HCl (2x 25 ml), water, and brine. The organic layer was dried and evaporated, and the resultant oil chromatographed on silica gel (EtOAc) to afford the phosphonamidate as a clear oil. Yield: 3.43g (69%). N.M.R. and I.R. data were in accord with the assigned structure.

**[(6-maleimido)hexanoyl] amino] (2-henylethyl)methoxyphosphinoyl D-alanine [5]**

To a solution of the phosphonamidate [4] (318 mg, 0.732 mmol) in EtOAc (5 ml) was added 5% Pd on charcoal (30 mg) and the mixture stirred under an atmosphere of hydrogen until T.L.C. (silica plates, 5% $MeOH/CH_2Cl_2$) indicated absence of starting material. The catalyst was removed by filtration and the solvent evaporated *in vacuo*. The residue was dissolved in $CH_2Cl_2$ (10 ml) and $Et_3N$ (0.103 ml, 0.732 mmol) was added, followed by 6-maleimido hexanoic acid anhydride (296 mg, 0.732 mmol). The solution was stirred for 3h. Solvent was removed *in vacuo* to yield a thick oil which was purified by reversed-phase HPLC to give the title compound. Yield: 90 mg (24%). N.M.R. and I.R. data were in accord with the assigned structure.

**[(6-maleimido)hexanoyl] amino] (2-phenylethyl)hydroxyphosphinyl D-alanine [6]**

To a cooled (0°) solution of [(6-maleimido)hexanoyl] amino] (2-phenylethyl)methoxyphosphinoyl D alanine (20 mg, 0.041 mmol) in $CH_2Cl_2$ (1 ml) was added 1,4-cyclohexadiene (780 ml), followed by trimethylsilyl iodide (12 ml, 0.08 mmol). After 2 min., solvent was removed *in vacu*o, and the residue dissolved in 5 ml of 0.5M $NaH_2PO_4/Na_2HPO_4$ buffer, pH 7.4 containing 2% $CH_3CN$. This solution was forced through 2 Waters "Sep-Paks". The Sep Paks were washed with 20 ml of 0.05M $NaH_2PO_4/Na_2HPO_4$ buffer, pH 7.4 containing 2% $CH_3CN$, and the product eluted with 0.005M $NaH_2PO_4/Na_2HPO_4$ buffer, pH 7.4 containing 50% $CH_3CN$ (10 ml). Lyophilization afforded a white powder containing the desired title compound (confirmed by N.M.R.) admixed with inorganic buffer. This powder was used directly for conjugation to carrier protein. (See Example 19).

**Example 4**

**Synthesis of O-[(6-maleimido)hexanyl]amimo](2-phenylethyl)phosphinyl lactic acid disodium salt**

The reaction sequence is shown in Fig. 17.

**Methyl O-[-(benzyloxycarbonyl)amino-2-phenylethyl methoxyphosphinyl] lactate. [7]**

To a solution of methyl hydrogen 2-(benzyloxycarbonylamino)-3-phenylpropyl phosphonate (777mg, 2.22 mmol) in dry $CHCL_3$ (10ml) cooled to 0°, was added $SOCl_2$ (0.15ml, 2.66 mmol) dropwise. After stirring for 3h, volatiles were removed *in vacuo*, and the resulting viscous oil was redissolved in dry $CHCL_3$ (3ml) and cooled to 0°. N-methylmorpholine (0.537 ml, 4.88 mmol) was added, followed by a solution of methyl (D) lactate (231 mg, 2.22 mmol) in dry $CHCl_3$. The mixture was stirred for 30 min, and then diluted with $CHCl_3$ (50ml) , washed with 1M HCl (15ml), dried ($MgSO_4$) and evaporated. The resultant oil was chromatographed ($SiO_2$) using 5% $CH_2Cl_2$/MeOH as eluant to give the title compound as a yellow oil. Yield: 0.54g (56%). n.m.r and i.r. data were in accord with the assigned structure.

**Methyl O-[-(benzyloxycarbonyl)amino-2-phenylethyl hydroxyphosphinyl] lactate, triethylammonium salt. [8]**

The starting phosphonate (253 mg, 0.58 mmol) was dissolved in a solution of dioxan/thiophenol/triethylamine (2:1:1, 5 ml), and stirred overnight. The solution was added dropwise to pentane (50 ml) affording an oily precipitate. The pentane layer was decanted and the oil redissolved in CH2Cl2 (2 ml). Pentane (50 ml) was added to again afford an oily precipitate. The pentane was decanted and the oil dried under vacuum to afford the title compound as an oil. Yield: 347 mg (100%). n.m.r and i.r. data were in accord with the assigned structure.

O-[-(benzyloxycarbonyl)amino-2-phenylethyl hydroxyphosphinyl] lactic acid [9]

Methyl O-[2-(benzyloxycarbonyl)amino-3-phenyl phosphinyl] lactate, triethylammonium salt (1.443 g, 2.41 mmol) was dissolved in aq. LiOH (7.2 ml of a 1.5M solution, 10.8 mmol) and the solution stirred at room temperature. Progress of the reaction was monitored by reversed-phase HPLC. When reaction was complete, the mixture was lyophilized. The resultant powder was dissolved in 200 ml of 0.05M $Na_2CO_3/NaHCO_3$ buffer, pH 8.65, and applied in two batches to a 28x2cm column of DEAE Trisacryl M. Each batch was eluted with a gradient of 0.05 to 0.25 M $Na_2CO_3/NaHCO_3$ $_b$uffer, pH 8.65 and fractions monitored by U.V. Fractions containing UV absorbing material were pooled and lyophilized to afford O-[-(benzyloxycarbonyl)amino-2-phenylethyl hydroxyphosphinyl] lactic acid as a white powder (555mg, 57%). 386mg (0.95 mmol) of the powder was dissolved in a minimum quantity of deionized water and applied to a column of 15g (dry weight) of SP Sephadex (Sigma SP-C25-120, previously swollen with deionized water for 3h and washed with 10% HCl w/v (250 ml), deionized water (800 ml),, 12% aq. NaCl w/v (250 ml), and deionized water (800 ml)). Product was eluted with 300 ml of deionized water, collecting all fractions.Yield: 427mg (100%). n.m.r and i.r. data were in accord with the assigned structure.

O-[(6-maleimido)hexanoyl] amino] (2-phenylethyl)phosphinyl lactic acid disodium salt. [10]

The phosphonate disodium salt (167 mg, 0.37 mmol) was dissolved in $H_2O$ (5 ml) and added to a suspension of previously hydrogenated 10% Pd/C (50 mg) in $H_2O$ (5 ml). The mixture was stirred under an atmosphere of hydrogen until HPLC indicated absence of starting material. The solution was filtered through Celite with the aid of extra MeOH (10 ml) and evaporated to yield an oil. This oil was dissolved in DMSO (1 ml) and $H_2O$ (1 ml). To this solution was added a solution of 6-maleimido hexanoic acid anhydride (168 mg, 0.416 mmol). The initially cloudy solution was stirred for 30 min. during which it became clear. The solution was applied directly to a reverse phase HPLC column for purification. Separation of the expected two diastereomers of the product was achieved. After lyophilization of appropriate fractions, the purified products were oils. Each oil was converted separately to the disodium salt via ion-exchange chromatography, as described above, to yield the pure diastereomers of the title compound as white powders. Combined yield: 43 mg (23% overall). n.r.r. and i.r. data were in accord with the assigned structure.

Example 5

Synthesis of [(6-Maleimido)hexanoyl]amino]-2-phenylethyl (2-carboxy-1-propyl)hydroxyphosphonous acid

The reaction sequence is shown in Fig. 18.

[1-(Benzyloxycarbonyl)amino]-2-phenylethyl phosphinic acid [11] was prepared by the method of Baylis *et al*. (18).

Methyl [1-(benzyloxycarbonyl)amino]-2-phenylethyl phosphinate. [12]

To a solution of [1-(benzyloxycarbonyl)amino]-2-phenylethyl phosphonous acid in EtOAc/ MeOH (10:1, 100 ml) was added ethereal diazomethane until the yellow color persisted. After standing for 10 min. excess diazomethane was destroyed by addition of glacial acetic acid. Solvent was removed *in vacuo* and the residue purified by chromatography on silica gel (5%MeOH/$CH_2Cl_2$ eluant) to afford the product as a clear oil which solidified on standing. Yield: 2.985 g (87%). N.M.R. and I.R. data were in accord with the assigned structure.

Methyl [(1-(benzyloxycarbonyl)amino]-2-phenylethyl)(2-carbomethoxy-1-propyl) phosphinate. [13]

To a solution of methyl [1-(benzyloxycarbonyl)amino]-2-phenylethyl phosphinate (936 mg, 2.81 mmol) in dry MeOH (5 ml) cooled to 0°, was added NaOMe (1.54 ml of a 2M solution) dropwise. After completion of addition, the solution was stirred for 5 min. and methyl methacrylate (300 ml, 2.82 mmol) was added in one portion. The solution was stirred at room temperature for 5h and poured into 1M HCl (10 ml). The solution was extracted with EtOAc (2x 100 ml) and the organic layers combined, washed with brine, dried ($MgSO_4$) and evaporated. Chromatography on $SiO_2$ afforded the title compound as a clear oil. Yield: 917 mg, (75%). N.M.R. and I.R. data were in accord with the assigned structure.

([1-(benzyloxycarbonyl)amino]-2-phenylethyl)(2-carboxy-1-propyl) hydroxyphosphonous acid [14]

To a solution of methyl [(1-(benzyloxycarbonyl)amino]-2-phenylethyl)(2-carbomethoxy-1-propyl) phosphinate (720 mg, 1.66 mmol) dioxan (3 ml) and water (2 ml) was added aq. LiOH (1.16 ml of a 3M solution, 3.48 mmol). The resultant solution was stirred at room temperature and progress of the reaction was monitored by HPLC. Upon

completion of reaction, the mixture was diluted with 200 ml of 0.05M $Na_2CO_3/NaHCO_3$ buffer, pH 8.65, and applied to a 28x2cm column of DEAE Trisacryl M. The column was eluted with a gradient of 0.05 to 0.25 M $Na_2CO_3/NaHCO_3$ buffer, pH 8.65 and fractions monitored by U.V. Fractions containing U.V. absorbing material were pooled and lyophilized to afford the title compound as a white powder. Yield: 496 mg, 74% . N.M.R. and I.R. data were in accord with the assigned structure.

**[(6-Maleimido)hexanoyl] amino]-2-phenylethyl(2-carboxy-1-propyl) hydroxyphosphonous acid [15]**

To a previously hydrogenated suspension of 10% Pd/C (20 mg) in MeOH (0.5 ml) was added a solution of methyl [(1-(benzyloxycarbonyl)amino]-2-phenylethyl)(2-carbomethoxy-1-propyl) phosphinate (75 mg, 0.185 mmol) in MeOH (1 ml). The mixture was stirred under an atmosphere of hydrogen for 2h. The catalyst was removed by centrifugation and the supernatant evaporated to give a white solid. This solid was dissolved in DMSO (250 ml) and $Et_3N$ (3 drops) were added. To this mixture was added a solution of 6-maleimido hexanoic acid anhydride (87 mg, 0.222 mmol) in DMSO (250 ml). After 2h, the product was isolated directly by reversed-phase HPLC. The product was repeatedly lyophilized to afford a white powder. Yield: 31 mg (36%). N.M.R. and I.R. data were in accord with the assigned structure.

**Example 6**

**Synthesis of (2-[(6-maleimido)butanoyl]amino]-3-phenylpropylimino-D-alanine**

The reaction sequence is shown in Fig. 19.

**2-amino-3-phenylpropanonitrile. [16]**

To a solution of freshly distilled phenylacetaldehyde (29.25 ml, 0.25 mol) in MeOH (100 ml) was added a solution of NaCN (12.25 g, 0.25 mol) and $NH_4Cl$ (26.75 g, 0.5 mol) in $H_2O$ (100 ml). The mixture was stirred for 18h, and MeOH was removed *in vacuo*. The resulting aqueous solution was extracted with $CH_2Cl_2$ and the organic layer dried and evaporated to yield the product as a yellow oil which was used without further purification. Yield: 34.46g (94%). N.M.R. and I.R. data were in accord with the assigned structure.

**2-(benzyloxycarbonyl)amino-3-phenylpropanonitrile. [17]**

To a solution of N-benzyloxycarbonyloxy succinimide (12.46g, 0.05 mol) in $CH_2Cl_2$ (25 ml) was added a solution of 2-amino-3-phenylpropanonitrile (7.31 g, 0.05 mol), followed by $Et_3N$ (7 ml, 0.05 ml). The exothermic reaction was cooled in an ice bath for 10 min, and then allowed to stir at room temperature for a further 10 min. The solution was poured into 0.05M NaOH solution (50 ml) and extracted with $CH_2Cl_2$. The organic layer was dried, evaporated and chromatographed ($SiO_2$, 5% $MeOH/CH_2Cl_2$) to afford the title compound as a white solid. Yield: 8.93g, (64%). N.M.R. and I.R. data were in accord with the assigned structure.

**2-(benzyloxycarbonyl)amino-3-phenylpropylimino-D-alanine. [18]**

a) 2-(benzyloxycarbonyl)amino-3-phenylpropanonitrile (112 mg, 0.40 mmol) was dissolved in dry $CH_2Cl_2$ (3 ml) and MeOH (12.82 mg, 0.4 mol). The mixture was cooled to 0° and saturated with dry HCl gas. The mixture was stirred at 0° for 48h affording a white precipitate of imino ester hydrochloride. The precipitate was filtered off and dried. Yield: 98 mg (70%).

b) The imino ester hydrochloride (446 mg, 1.28 mmol) was converted to the free base by extracting once with $CHCl_3$ (10 ml) and aq. NaOH (10 ml of a 1M solution). The organic layer was dried and evaporated to afford the free base as an oil (361 mg, 91 %). This was dissolved in dry MeOH (8 ml) and D-alanine (103 mg, 1.16 mmol, dried over $P_2O_5$) was added. The mixture was heated to reflux for 1.5 h. The solution was filtered and diluted with $Et_2O$ to afford the title compound as a white solid, obtained by filtration. Yield: 252 mg (59 %). N.M.R. and I.R. data were in accord with the assigned structure.

**(2-[(6-maleimido)butanoyl] amino])-3-phenylpropylimino-D-alanine [19]**

A solution of 2-(benzyloxycarbonyl)amino-3-phenylpropylimino-D-alanine (30.4 mg, 0.082 mmol) in MeOH

(5 ml) was hydrogenated over 10% Pd/C (6.5 mg) for 1.5h. The catalyst was removed by filtering through a pad of Celite and the filtrate evaporated *in vacuo*. The residue was dissolved in 0.5M $NaH_2PO_4/Na_2HPO_4$ buffer, pH 7.0 and added to a solution of N-(4-maleimido)butanoyl succinimide (27.6 mg, 0.098 mmol) in DMSO (0.1 ml). The mixture was stirred overnight, and purified directly by reversed-phase HPLC ($H_2O/CH_3CN$) to afford the title compound as a white solid. Yield: 3.9 mg (12%). N.M.R. and I.R. data were in accord with the assigned structure.

**Example 7**

**Synthesis of 3-Difluoro-5-(6-maleimidohexanoyl)amino-2-methyl-4-oxo-6-phenylhexanoic acid**

The reaction sequence is shown in Fig. 8.

**2,2-Difluoro-3-methyl-4-pentenoic acid [1]**

A 250 mL three neck flask equipped with a magnetic stir bar, a low temperature thermometer, a condenser filled with dry ice, and a balloon was charged with dry tetrahydrofuran (100 mL). The flask was cooled to approximately -50°C, and tetrafluoroethylene was passed through a tube of silica and then through a needle into tetrahydrofuran until saturation was reached (as judged by inflation of the balloon). The flask was allowed to warm to -5°C to -10°C which caused the balloon to inflate slightly under acetone-dry ice bath. To a separate 50 mL flask was added sodium hydride (100 mg, 4.20 mmol, 60 % suspension in oil), and the oil was removed by repeated washing with dry tetrahydrofuran under argon. A solution of crotyl alcohol (3.74 g, 52.0 mmol) in dry tetrahydrofuran (2.0 mL) was slowly added by syringe with stirring, and the mixture was allowed to stir for an additional 10 min. The solution was then transferred dropwise by syringe to the three-neck flask with stirring at -5°C to -10°C. After approximately 3 h, additional tetrafluoroethylene was bubbled into the solution for about 15 min. After 6 h, the reaction was placed under argon and cooled to -60°C and n-butyllithium (32.5 mL, 52.0 mmol, 1.6M in n-hexane) was added dropwise over an hour from an additional syringe with the temperature kept constant at -60°C. The mixture was cooled to -78°C and water (2.0 mL) was added. The cooling bath was removed, and the solution was stirred overnight. The solution was basified with aqueous sodium hydroxide to pH 10 and the solvent was removed in vacuo. The residue was acidified with 1N hydrochloric acid to pH 1 and extracted with diethyl ether (3x100 mL). The organic layers were combined and extracted with saturated aqueous sodium bicarbonate (3x200 mL). The aqueous extract was acidified to pH 1 with hydrochloric acid and extracted three times with diethyl ether, dried ($MgSO_4$), concentrated *in vacuo* to give 1.50g (20 %) of product [**1**] as a pale yellow oil. The N.M.R., I.R. and mass spectra were in accord with the assigned structure.

**Silver 2,2-difluoro-3-methyl-4-pentenoate [2]**

A suspension of silver oxide in water was prepared by adding a solution of NaOH (346 mg, 8.67 mmol) in water (15 mL) to an aqueous solution of silver nitrate (1.47g, 8.67 mmol) in water (15 mL), decanting the supernatant liquid, washing the residue with water (3x20 mL) and adding 20 mL of water. To this vigorously stirred suspension was added a solution of 2,2-difluoro-3-methyl-4-pentenoic acid [**1**], (1.30g, 8.67 mmol) in water (20 mL). After 30 min, the mixture was filtered and the filtrate concentrated to afford a solid residue which was dried over phosphorous pentoxide (0.05 Torr, 50°C, 24 hours) to give 1.38 g (62 %) of product [**2**] as a white amorphous powder. The N.M.R., I.R. and mass spectra were in accord with the assigned structure.

**2,2-Difluoro-3-methyl-4-pentenoic acid anhydride [3]**

A suspension of the silver salt [**2**], (5.09 g, 19.8 mmol) in dichloromethane (30 mL) was stirred under argon, cooled to 0°C and then oxalyl chloride (1.25 g, 9.86 mmol) was added slowly. The cooling bath was removed and the reaction mixture allowed to warm up to room temperature. Warming up to reflux for 30 minutes completed the reaction. Cooled again to room temperature, the supernatant liquid was decanted and the residue washed with dichloromethane (10 mL). The organic layers were combined and the solvents removed in vacuo to give a pale yellow oil. This extremely hygroscopic product [3] was used for a next reaction without further purification.

**N-p-Phenylbenzoyl-DL-phenylalanine [4]**

To a solution of D,L-phenylalanine (7.00g, 42.4 mmol) in 1N NaOH (700 mL) was added 4-biphenylcarbonylchloride (13.8g, 63.6 mmol) in a small portion over a period of 2 h. The mixture was stirred at room temperature for additional 3 h. It was slowly acidified with concentrated HCl to pH 3. The resulting solid was filtered and washed with cold water. The precipitate was dried in a desiccator under vacuum. The crude products was chromatographed with 150

g of silica gel (methyl alcohol/dichloromethane (1:20)) to give 10.5g (72 %) of product [**4**] as a pale yellow solid. The nmr and ir spectra were accord with the assigned structure.

**2-p-biphenyl-4-phenylmethyl-5(4H)-oxazolinone [5]**

To a solution of N-p-phenylbenzoyl-DL-phenylalanine [**4**], (2.00g, 5.80 mmol) in dichloromethane(100 mL) was added 1-ethyl-3-(3-dimethylaminopropyl)-carbodiimide hydrochloride (1.00g, 5.19 mmol) in a single portion. The resulting mixture was stirred 0°C for 30 minutes. It was diluted with dichloromethane (100 mL) and washed with cold water (2x200 mL), cold aqueous 1M $NaHCO_3$(2x200 mL), cold saturated aqueous NaCl solution (2x100 mL), and cold water (100 mL). It was dried ($MgSO_4$) and concentrated *in vacuo* to give 1.90g of product [**5**] as a white solid in quantitative yield. The N.M.R. and I.R. spectra were in accord with the assigned structure.

**2-p-phenylbenzoylamino-1-phenyl-4,4-difluoro-5-methyl-6-hepten-3-one [6]**

A mixture of 2,2-difluoro-3-methyl-4-pentenoic acid anhydride [**3**], (2.79g, 9.90 mmol) and 2-p-biphenyl-4-phenylmethyl-5(4H)-oxazolinone [**5**], (2.70g, 8.26 mmol) was stirred under argon for 40 h at 60°C (oil bath temperature) to give a lightly red viscous oil. Anhydrous oxalic acid (0.744 g, 8.28 mmol) was then added and the mixture was heated for 15 minutes (110-120°C, oil bath temperature). After the violent gas evolution had ceased, the oil was allowed to cool to room temperature and then diluted with ethyl acetate (100 mL). The organic layer was washed with saturated aqueous sodium bicarbonate (3x100 mL), brine (2x100 mL), water (100 mL), and dried ($MgSO_4$). The filtered mixture was concentrated and chromatographed with 100 g of silica gel (ethyl acetate/hexane (1:9)) to give 1.48g (42 %) of product (**6**) as a white solid. The N.M.R., I.R. and mass spectra were in accord with the assigned structure.

**2-p-phenylbenzoylamino-1-phenyl-4,4-difluoro-5-methyl-6-hepten-3-ol [7]**

To a solution of 2-p-phenylbenzoylamino-1-phenyl-4,4-difluoro-5-methyl-6-hepten-3-one [**6**], (0.84g, 1.94 mmol) in ethyl alcohol (50 mL) was added sodium borohydride (294 mg, 7.76 mmol) in a single portion and stirred at room temperature for 5 minutes. The resulting mixture was poured into water (50 mL) and excess ethyl alcohol was removed *in vacuo*. The resulting white precipitate was filtered and remaining filtrate was extracted with ethyl acetate (4x100 mL) and dried ($MgSO_4$). The resulting solid after concentration was combined with the precipitate to give 0.70 g (94 %) of product [**7**] as a white solid. It was pure enough for a next reaction without further purification. The N.M.R., I.R. and mass spectra were in accord with the assigned structure.

**2-Amino-1-phenyl-4,4-difluoro-5-methyl-6-hepten-3-ol hydrochloride [8]**

To a solution of 2-p-phenylbenzoylamino-1-phenyl-4,4-difluoro-5-methyl-6-hepten-3-ol [**7**], (786mg, 1.81 mmol) in methyl alcohol (50 mL) was added $NaH_2PO_4.H_2O$ (2.50g, 18.1 mmol) in a single portion. 3 % Na-Hg (16.7g, 21.7 mmol) was added at room temperature and stirred for 1 h. The mixture was filtered into a solution of 1N HCl (11.0 mL, 11.0 mmol) and was washed with additional methyl alcohol (20 mL). The excess methyl alcohol was removed in vacuo to give white precipitate. The resulting precipitate was filtered and washed with water (20 mL). The combined aqueous layers were lyophilized to give a white solid. It was dissolved in ethyl acetate (100 mL) and filtered. The filtrate was evaporated to give 420 mg (80 %) of product [**8**] as a white solid. The N.M.R., I.R. and mass spectra were in accord with the assigned structure.

**2-Benzyloxycarbonylamino-1-phenyl-4,4-difluoro-5-methyl-6-hepten-3-ol [9]**

To a solution of 2-amino-1-phenyl-4,4-difluoro-5-methyl-6-hepten-3-ol hydrochloride [**8**], (420 mg, 1.44 mmol) in 1:1 mixture of water and methyl alcohol (25 mL) was added triethylamine (0.2 mL, 1.44 mmol) in a single portion followed by N-(benzyloxycarbonyloxy)succinimide (476 mg, 1.87 mmol) at room temperature. The reaction was maintained at pH 8 by the addition of additional triethylamine (Ca 0.2 mL). It was stirred at room temperature for an hour, and acidified with aqueous 1N HCl to pH 5. The excess methyl alcohol was removed in vacuo and the aqueous layer was lyophilized to give a colorless oil. This was chromatographed with 50g of silica gel (ethyl acetate/hexane (1:9)) to give 550 mg (98 %) of product [**9**] as a colorless yellow oil. The N.M.R., I.R. and mass spectra were consistent with the assigned structure.

**2-Benzyloxycarbonylamino-4,4-difluoro-5-methyl-1-phenyl-6-hepten-3-one [10]**

To a solution of Dess-Martin periodinane (1.24g, 2.92 mmol) in dichloromethane (18 mL) is added the difluoro alcohol [**9**], (307mg, 0.790 mmol) in a single portion, and the mixture is stirred at room temperature for 3 h. The reaction mixture is then diluted with 20 mL of ether and poured into a 0.26 M solution of sodium thiosulfate in saturated aqueous sodium bicarbonate (60 mL). The layers are separated, and the organic phase is washed with saturated aqueous sodium bicarbonate (2x25 mL) and water (2x25 mL). The combined aqueous layers are then back extracted with ether (2x50 mL). The combined organic phases are dried ($MgSO_4$) and filtered. Excess solvents are then removed in vacuo and the crude mixture is purified by chromatography using 50 g of silica gel and eluting with ethyl acetate/n-hexane to give the product [**10**] as a solid.

**5-Benzyloxycarbonylamino-3-difluoro-2-methyl-4-oxo-6-phenylhexanoic acid [11]**

A solution of [**10**] (194mg, 0.50 mmol) in dichloromethane (20 mL) is treated with $O_3$ at -78°C for 5 min (about 2.5 mmol of $O_3$). Dimethylsulfide (0.2 mL, 2.7 mmol) is added and the solution allowed to warm to room temperature. After removal of solvents, the resulting mixture is dissolved with acetone (3.0 mL) and is treated with a Jones reagent (2 mL, 1 M $CrO_3$/$H_2SO_4$) overnight. The resulting solution is filtered through Celite and evaporated in vacuo. The crude product is diluted with water (50 mL), and the aqueous phase is extracted with ethyl acetate (4x20 mL). The combined organic layers are washed with brine (2x50 mL) and water (50 mL), dried ($MgSO_4$) and concentrated in vacuo and the resulting mixture is purified by chromatography using 50g of silica gel and eluting with methyl alcohol/dichloromethane to give the product [**11**] as a solid.

**3-Difluoro-5-(6-maleimidohexanoyl)amino-2-methyl-4-oxo-6-phenylhexanoic acid [12]**

To a solution of [**12**], (203mg, 0.50 mmol) in ethyl acetate (5 mL) is added 10% palladium on charcoal catalyst (20 mg) and the mixture is stirred under an atmosphere of hydrogen for 3 h. The mixture is filtered through Celite and the solvent evaporated in vacuo. The residue is dissolved in dichloromethane (10 mL) and triethylamine (0.070mL, 0.50 mmol) is added, followed by 6-maleimidohexanoic acid anhydride (202mg, 0.50 mmol). The reaction mixture is stirred for 3 h at room temperature. Solvent is removed in vacuo to give a thick oil which is purified by reverse-phase HPLC to give the product [**12**] as a solid.

**Example 8**

**Synthesis of 5-[N-Benzyloxycarbonylmethylamido]-3,3-difluoro-2-methyl-4-oxo-6-phenylhexanoic acid**

The reaction sequence is shown in Fig. 9.

**Hydrocinnamic acid methyl ester [2]**

A separatory funnel (with clear seal joint) was placed over the reaction vessel and charged with a solution of Diazald® (5.0g, 23 mmol) in ether (45 mL). The reaction vessel was warmed to 65°C with a water bath and the Diazald® solution was added over a period of 20 min. The rate of distillation approximated the rate of addition. When all the Diazald® had been used, additional ether (10 mL) was added slowly and the distillation was continued until the distillate was colorless. The ethereal distillate contained about 700mg (16.6 mmol) of diazomethane. The resulting diazomethane was poured into a solution of hydrocinnamic acid [**1**], (2.0g, 3.3 mmol) in ether (20 mL) in an ice bath. Acetic acid was then added dropwise into the reaction mixture until the yellow color and the evolution of gas had disappeared. The excess solvent was removed in vacuo to give 2.0g (90 %) of product [**2**] as a yellow oil.

**2,2-Difluoro-3-methyl-4-pentanoic acid methyl ester [4]**

Diazomethane was made in the same way described above for compound [**2**] on the same scale. The excess diazomethane was added to a cooled (0°C) solution of acid [**3**], (2.0g, 3.3 mmol) in ether (20 mL). Acetic acid was then added to the reaction mixture dropwise until the yellow color was discharged. Solvent was removed in vacuo and the residue distilled to give 1.75g (80 %) of ester [**4**] as a colorless oil (bp. 112°C).

**4,4-Difluoro-5-methyl-3-oxo-2-phenylmethyl-6-heptenoic acid methyl ester [5]**

A THF solution (10 mL) of methyl ester [**2**] , (1.64g, 10 mmol) is added dropwise to a solution of lithium diisopropylamide (15 mmol) in 30 mL of THF at -78°C . The mixture is stirred for 30 min at -78°C prior to the addition of a solution of difluoro acid methyl ester (3.28g, 20 mmol) in THF (10 mL). The reaction mixture is allowed to warm gradually to room temperature and stirred for a period of 26 h. The reaction is then quenched at room temperature by the

addition of 5 % aqueous hydrochloric acid (40 mL). The layers are then separated, and the organic phase is washed with water (2x20 mL) and saturated aqueous sodium chloride (2x20 mL). The combined aqueous layers are then back extracted with ether (25 mL). The combined organic phases are dried over anhydrous $MgSO_4$ and filtered, and the solvent is removed under reduced pressure to give product [**5**] as an oil. Pure product [**5**] is obtained by chromatography of the crude mixture using 100g of silica gel and eluting with ethyl acetate and n-hexane.

**2-[N-(Benzyloxycarbonylmethyl)amido]-4,4-Difluoro-5-methyl-1-phenylmethyl-6-hepten-3-one [6]**

To a solution of ester [**5**], (858mg, 2.0 mmol) in methanol (10 mL) is added a solution of potassium carbonate (2.76g, 20 mmol) in water (10 mL) at room temperature. The resulting solution is warmed under gentle reflux for 3 h. Excess methyl alcohol is removed in vacuo and the reaction mixture is acidified by the addition of concentrated hydrochloric acid dropwise to pH 6. The mixture is lyophilized and the resulting white solid is dissolved with ethyl acetate (50 mL) and filtered. The filtrate is concentrated in vacuo and is dissolved in dichloromethane (20 mL). A solution of 1,3-dicyclohexylcarbodiimide (205mg, 10 mmol) in dichloromethane (10 mL) is added at room temperature under argon and the mixture stirred for 30 min. The undissolved precipitate is filtered and the filtrate is concentrated in vacuo. The resulting solid is dissolved in DMF (10 mL) and a solution of glycine benzylester (165 mg, 1.0 mmol) in DMF (10 mL) followed by a solution of N-methylmorpholine (0.13 mL, 1.2 mmol) in DMF (5 mL) is added dropwise. The reaction mixture is stirred for an hour and then diluted with ethyl acetate (100 mL). The organic layer is washed with 1 N aqueous hydrochloric acid (2x50 mL), saturated sodium bicarbonate (2x50 mL), and water (2x50 mL), dried ($MgSO_4$), and concentrated in vacuo to give [**6**] as an impure solid. Pure product [**6**] is obtained by chromatography of the crude mixture using 100g of silica gel and eluting with ethyl acetate/n-hexane.

**5-[N-(Benzyloxycarbonylmethyl)amido]-3,3-difluoro-2-methyl-4-oxo-6-phenylhexanoic acid [7]**

A solution of [**6**] (429mg, 1.0 mmol) in dichloromethane (20 mL) is treated with $O_3$ at -78°C for 12 min (about 6 mmol of $O_3$). Dimethylsulfide (0.4mL, 5.4 mmol) is added and the solution allowed to warm to room temperature. After removal of solvents, the resulting mixture is dissolved in acetone (6.0 mL) and treated with Jones reagent (4.0mL, 1 M $CrO_3/H_2SO_4$) overnight. The resulting solution is filtered through Celite and evaporated in vacuo. The crude product is diluted with water (50 mL), and the aqueous phase is extracted with ethyl acetate (4x20 mL). The combined organic layers are washed with brine (2x50 mL) and water (50 mL), dried ($MgSO_4$) and concentrated in vacuo and the resulting mixture is purified by chromatography using 50g of silica gel and eluting with methyl alcohol/dichloromethane to give the product [**7**] as a solid.

**Example 9**

**Preparation of (S)-3-(Benzyloxycarbonyl)amino-2-Oxo-1-Azetidineacetic acid [5]**

The reaction sequence is shown in Fig. 20. The β-lactam derivative (S)-3-(*tert*-butoxycarbonyl)amino-2-azetidinone [1] was prepared by a published procedure (19).

**Preparation of (S)-Benzyl 3-tert-(Butoxycarbonyl)amino-2-Oxo-1-Azetidineacetic acid [2]**

1.5 g (8.2 mmol) of (S)-3-(*tert*-butoxycarbonyl)amino-2-azetidinone and 1.98 g (8.2 mmol) of benzyl 2-bromoacetate were dissolved in 40 ml of anhydrous DMF and stirred at 0°C under a nitrogen atmosphere. Sodium hydride (60% oily suspension), 0.394 g (16 mmol), was added quickly, and the reaction mixture was stirred for 2 hr at 0°C. At this time 175 ml of ethyl acetate was added to the mixture, and the solution was washed with water (150 ml), saturated aqueous $NaHCO_3$ (150 ml), and water (150 ml) respectively, and then dried by filtration over anhydrous magnesium sulfate. The solvent was removed by evaporation under reduced pressure, and the product was purified by chromatography on silica gel (eluted with 5:4 methylene chloride:diethyl ether) to yield 0.283 g (0.82 mmol) of (S)-benzyl 3-(*tert*-butoxycarbonyl)amino-2-oxo-1-azetidineacetic acid [2]. $^1H$ NMR ($CDCl_3$): δ 1.4 (s, 9H), 3.4 (m, 2H), 3.7 (t, 1H), 4.06 (s, 2H), 4.4 (bs, 1H), 5.2 (s, 2H), 7.3 (s, 5H).

**Preparation of (S)-Benzyl 3-Amino-2-Oxo-1-Azetidineacetic acid [3]**

(S)-benzyl 3-(*tert*-butoxycarbonyl)amino-2-oxo-1-azetidineacetic acid 0.025 g (0.075 mmol), and 0.014 g of *p*-toluenesulfonic acid monohydrate were dissolved in 12 ml of ethyl acetate, and the resulting solution was refluxed for 2-1/2 hr. Then 10 ml of 5% aqueous $NaHCO_3$ was added, and the aqueous layer was extracted with ethyl acetate (5 x 10 ml). The combined organic layers were dried by filtration over anhydrous magnesium sulfate and the solvent was

removed by evaporation under reduced pressure to yield 0.0081 g (0.035 mmol) of (S)-benzyl 3-amino-2-oxo-1-azetidineacetic acid [3]. $^1H$ NMR ($CD_3COCD_3$): δ 3.1 (bs, 1H), 3.5 (dd, 1H), 3.8 (t, 1H), 4.1 (s, 2H), 4.9 (m, 1H), 5.1 (s, 2H), 7.3 (s, 5H).

**Preparation of (S)-3-Amino-2-Oxo-1-Azetidineacetic acid [4]**

(S)-benzyl 3-amino-2-oxo-1-azetidineacetic acid, 0.0081g (0.035 mmol), was dissolved in 3 ml of methanol and 0.01 g acetic acid was added. Palladium, 0.10 g 10%, on activated carbon was added, and the mixture was stirred under one atmosphere of hydrogen gas for 1 hr. The catalyst was removed by filtration, and the solvent was removed by evaporation under reduced pressure to yield 0.004 g (0.028 mmol) of (S)-3-amino-2-oxo-1-azetidineacetic acid [4]. $^1H$ NMR ($D_2O$): δ 3.5 (m, 2H), 3.7 (s, 2H), 4.4 (dd, 1H); $^{13}C$ NMR ($D_2O$): δ 43.4, 47.2, 53.1, 167.0, 175.6.

**Preparation of (S)-3-(Benzyloxycarbonyl)amino-2-Oxo-1-Azetidineacetic acid [5]**

0.01 g (0.07 mmol) (S)-3-amino-2-oxo-1-azetidineacetic acid is dissolved in 1 ml $H_2O$. Carbobenzyloxy chloroformate 0.025 g (0.1 mmol) is in 0.5 ml of acetone is added slowly and the resulting solution is stirred for 2 hr. At this time 10 ml of water is added and the aqueous mixture is washed with methylene chloride (2 x 10 ml). The aqueous layer is then acidified to pH 3 with 1M HCl and extracted with methylene chloride (3 x 10 ml). The combined organic layers are dried by filtration over anhydrous magnesium sulfate and the solvent is removed by evaporation under reduced pressure to yield 3-(benzyloxycarbonyl)amino-2-oxo-1-azetidineacetic acid [5].

**Example 10**

**Preparation of (3'S,2R)-2-[3-Amino-2-Oxo-1-Azetidininyl]-3-Methylbutanoic Acid [14]**

This material was prepared exactly as described in a published procedure (20) by the reaction sequence shown in Fig. 21.

Treatment of 6-aminopenicillanic acid [11] with Raney nickel yielded a 2:1 mixture of the β-lactam derivatives (3'S,2R)-2-[3-(benzyloxycarbonyl)amino-2-oxo-1-azetidininyl]-3-methyl-3-butenoic acid [12] [$^1H$ NMR ($CD_3COCD_3$): δ 1.0 (d, 6H), 2.1 (m, 1H), 3.5 (dd, 1H), 3.8 (t, 1H), 4.1 (d, 1H), 4.8 (m, 1H), 5.1 (s, 2H), 7.3 (s, 5H)] and (3'S,2R)-2-[3-(benzyloxycarbonyl)amino-2-oxo-1-azetidininyl]-3-methylbutanoic acid [13] [$^1H$ NMR ($CD_3COCD_3$): δ 1.9 (s, 3H), 3.4 (dd, 1H), 3.9 (t, 1H), 4.8 (m, 1H), 4.9 (s, 1H), 5.0 (s, 1H), 5.05 (s, 1H), 5.1 (s, 2H), 7.3 (s, 5H)]. Hydrogenolysis of this mixture over palladium on activated carbon yielded (3'S,2R)-2-[3-amino-2-oxo-1-azetidininyl]-3-methylbutanoic acid [14] [$^1H$ NMR ($D_2O$): δ 0.9 (d, 6H), 2.1 (m, 1H), 3.3 (dd, 1H), 3.8 (d, 1H), 3.9 (t, 1H), 4.3 (m, 1H)].

**Example 11**

**Preparation of *cis*-3-(Benzyloxycarbonyl)amino4-Carboxy-2-Azetidinone [22]**

This material was prepared by a modification of a published procedure (21) as is shown in the reaction sequence in Fig. 22. (The (3)-lactam derivative *cis*-3-(benzyloxycarbonyl)amino-4-carbomethoxy-2-azetidinone [21] was prepared by a published procedure (22).

To a stirred solution 0.1 g (0.36 mmol) of *cis*-3-(benzyloxycarbonyl)amino-4-carbomethoxy-2-azetidinone in 5 ml of methanol at 0°C was slowly added 0.1 g $K_2CO_3$ in 3 ml of $H_2O$ at 0°C. The solution was stirred for 1 hr at room temperature and then neutralized to pH 7 with phosphoric acid at 0°C and filtered. The methanol was removed by evaporation under reduced pressure, and the remaining aqueous solution was extracted with ethyl acetate (2 ml). The aqueous layer was acidified to pH 2 with phosphoric acid and then saturated with NaCl. The resulting solution was extracted with ethyl acetate (5 x 5 ml), and the combined organic layers are washed with saturated NaCl (5 ml) and dried by filtration over anhydrous magnesium sulfate. The solvent was removed by evaporation under reduced pressure to yield 0.082 g (0.31 mmol) of *cis*-3-(benzyloxycarbonyl)amino-4-carboxy-2-azetidinone [22]. $^1H$ NMR ($CD_3SOCD_3$): δ 4.2 (d, 1H), 5.0 (s, 2H), 5.0 (dd, 1H), 7.2 (s, 5H), 8.1 (d, 1H), 8.6 (d, 1H).

**Example 12**

**Preparation of 3-(Benzyloxycarbonyl)amino-2-Oxo-1-Cyolobutaneacetic Acid [34] (as a mixture of diastereomers) and 3-(Benzyloxycarbonyl)amino-2-Hydroxy-1-Cyclobutaneacetic Acid [35] (as a mixture of diastereomers)**

The reaction sequence is shown in Figure 23. The derivative 2-(dibenzylamino)cyclobutanone [31] was prepared by a published procedure (23).

**Preparation of Benzyl 3-Dibenzylamino-2-Oxo-1-Cyclobutaneacetic Acid [32]**

To a stirred solution of 2.9 mmol lithium diisopropylamide in 5 ml THF is added dropwise 0.66 g (2.5 mmol) of 2-(dibenzylamino)cyclobutanone in 4 ml of THF at -78°C and the mixture is stirred for 15 min at - 78°C and then for 15 min at 0°C. The solution is then cooled to -78°C and 0.53 ml (2.9 mmol) of HMPA is added. After five min, 0.70 g (2.9 mmol) of benzyl 2-bromoacetate in 10 ml of diethyl ether is added and the mixture is stirred at -78°C for 1 hr. The solvents are removed by evaporation under reduced pressure, and the product benzyl 3-dibenzylamino-2-oxo-1-cyclobutaneacetic acid [32] (as a mixture of diastereomers) is purified by chromatography on silica gel.

**Preparation of 3-Amino-2-Oxo-1-Cyclobutaneacetic Acid [33]**

Benzyl 3-dibenzylamino-2-oxo-1-cyclobutaneacetic acid, 0.10 g (0.25 mmol) is dissolved in 3 ml of methanol and 0.01 g acetic acid is added. Palladium, 0.10 g 10% on activated carbon is added, and the mixture is stirred under one atmosphere of hydrogen gas for 24 hr. The catalyst is removed by filtration, and the solvent is removed by evaporation under reduced pressure to yield 3-amino-2-oxo-1-cyclobutaneacetic acid [33] (as a mixture of diastereomers).

**Preparation of 3-(Benzyloxycarbonyl)amino-2-Oxo-1-Cyclobutaneacetic Acid [34]**

0.01 g (0.07 mmol) of 3-amino-2-oxo-1-cyclobutaneacetic acid is dissolved in 1 ml $H_2O$. 0.025 g (0.1 mmol) of carbobenzyloxy chloroformate in 0.5 ml of acetone is then added slowly, and the mixture is stirred for 2 hr. At this time 10 ml of water is added, and the solution is washed with methylene chloride (2 x 10 ml). The aqueous layer is then acidified to pH 3 with 1M HCl and is extracted with methylene chloride (3 x 10 ml). The combined organic layers are dried by filtration over anhydrous magnesium sulfate, and the solvent removed by evaporation under reduced pressure to yield 3-(benzyloxycarbonyl)amino-2-oxo-1-cyclobutaneacetic acid [34] (as a mixture of diastereomers).

**Preparation of 3-(Benzyloxycarbonyl)amino-2-Hydroxy-1-Cyclobutaneacetic Acid [35]**

First, 0.01 g (0.036 mmol) of 3-(benzyloxycarbonyl)amino-2-oxo-1-cyclobutaneacetic acid is dissolved in 10 ml of methanol and 0.01 g (0.26 mmol) of $NaBH_4$ is added. The solution is stirred for 1 hr at room temperature and the solvent is removed by evaporation under reduced pressure. Additional methanol is added and removed by evaporation under reduced pressure (4 x 10 ml). At this time 10 ml of methanol and 10 ml of 1M HCl are added and the methanol is removed by evaporation under reduced pressure. The resulting solution is extracted with methylene chloride (5 x 10 ml), and the combined organic layers are washed with saturated NaCl (5 ml) and dried by filtration over anhydrous magnesium sulfate. The solvent is removed by evaporation under reduced pressure, and the product is purified by chromatography on silica gel to yield 3-(benzyloxycarbonyl)amino-2-hydroxy-1-cyclobutaneacetic acid [35] (as a mixture of diastereomers).

**Example 13**

**Preparation of 2-(Benzyloxycarbonyl)amino-3-Hydroxycyclobutanecarboxylate [44] (as a mixture of diastereomers) and 2-(Benzyloxycarbonyl)amino-3-Oxocyclobutanecarboxylate [45] (as a mixture of diastereomers)**

The reaction sequence is shown in Fig. 24. Methyl 3-hydroxycyclobutanecarboxylate (as a mixture of diastereomers) was prepared by a published procedure (24).

**Preparation of 3-Carboxymethylcyclobutanyl Azidoformate [42]**

To a stirred solution of 0.60 g (4.62 mmol) of methyl 3-hydroxycyclobutanecarboxylate and 0.89 ml (11 mmol) of pyridine in 74 ml benzene at room temperature was added 0.91 g (3.1 mmol) triphosgene, and the resulting

solution was stirred for 1 1/2 hr. The solution was then quickly washed with saturated NaCl (3 x 6 ml), and the organic layer was dried by filtration over anhydrous magnesium sulfate, and the solvent was removed by evaporation under reduced pressure. The resulting product was dissolved in 22 ml of dry DMF and 1.03 g (15.9 mmol) of sodium azide was added. The solution was stirred at room temperature for 2 hr, 72 ml of water was added, and the solution was extracted with diethyl ether (5 x 30 ml). The organic extracts were combined, washed with saturated NaCl (3 x 32 ml), and dried by filtration over anhydrous magnesium sulfate. The solvent was removed by evaporation under reduced pressure, and the product was purified by chromatography on silica gel (eluted with 1:1 ethyl acetate:hexanes) to yield 0.078 g (0.39 mmol) of 3-carboxymethylcyclobutanyl azidoformate [42] (as a mixture of diastereomers). $1^H$ NMR ($CDCl_3$): δ 2.6-3.2 (m, 5H), 3.8 (s, 3H), 4.9-5.3 (m, 1H); IR ($CDCl_3$): μ 2150 $cm^{-1}$ (azide).

**Preparation of 3-Carboxymethylcyclobutanyl-1,2-Cyclic Carbamate [43]**

First, 3-carboxymethylcyclobutanyl azidoformate, 0.078 g (0.39 mmol), was dissolved in 5 ml dry methylene chloride, and the solution was sealed in a glass reaction tube and heated to 135°C for 1 hr. The solvent was removed by evaporation under reduced pressure, and the product was purified by chromatography on silica gel (eluted with 1:1 ethyl acetate:hexanes) to yield 0.029 g (0.17 mmol) of 3-carboxymethylcyclobutanyl-1,2-cyclic carbamate [43] (as a mixture of diastereomers). $^1H$ NMR ($CDCl_3$): δ 2.8 (m, 2H), 3.2 (m, 1H), 3.8 (m, 3H), 4.4 (m, 1H), 5.1 (dd, 1H), 6.2 (bs, 1H).

**Preparation of 2-(Benzyloxycarbonyl)amino-3-Hydroxycyclobutanecarboxylate [44]**

First, 3-carboxymethylcyclobutanyl-1,2-cyclic carbamate, 0.118 g (0.68 mmol) of and 0.384 g (6.88 mmol) of potassium hydroxide are dissolved in 20 ml of 1:1 (v:v) dioxane:water. The solution is refluxed for 23 hours. The resulting solution is extracted with diethyl ether (2 x 20 ml) and 5 ml of water is added. The solution is cooled to 0°C and is vigorously stirred. 0.25 g (1.0 mmol) of carbobenzyloxy chloroformate is added, and the solution is stirred at room temperature for 1 hr. The mixture is acidified to pH 1 with 1M HCl and is extracted with methylene chloride (3 x 30 ml). The combined organic layers are washed with saturated NaCl (30 ml) and are dried by filtration over anhydrous magnesium sulfate. The solvent is removed by evaporation under reduced pressure, and the product 2-(benzyloxycarbonyl)amino-3-hydroxycyclobutanecarboxylate [44] (as a mixture of diastereomers) is purified by chromatography on silica gel.

**Preparation of 2-(Benzyloxycarbonyl)amino-3-Oxocyclobutanecarboxylate [45]**

To a stirred solution of 0.10 g (0.39 mmol) of 2-(benzyloxycarbonyl)amino-3-hydroxycyclobutanecarboxylate in 20 ml acetone is added 0.235 ml (0.591 mmol) of Jones' reagent (prepared by dissolving 2.5 g chromium(VI) oxide in 2.15 ml concentrated sulfuric acid and diluting to 10 ml with water) at room temperature over a period of 15 sec. After an additional 30 s, 2 ml of 2-propanol is added, the mixture is filtered, and the solvents are removed by evaporation under reduced pressure. The residue is taken up in 20 ml ethyl acetate, and the resulting solution is washed with saturated Nacl (2 x 10 ml) and is then dried by filtration over anhydrous magnesium sulfate. The solvent is removed by evaporation under reduced pressure and the product 2-(benzyloxycarbonyl)amino-3-oxocyclobutanecarboxylate [45] (as a mixture of diastereomers) is purified by chromatography on silica gel.

**Example 14**

**Preparation of 3-*exo*-(Benzyloxycarbonyl)amino-2-*exo*-Hydroxynorbornyl-7-*anti*-carboxylate [53] and 3-*exo*-(Benzyloxycarbonyl)amino-2-Oxonorbornyl-7-*anti*-carboxylate [54]**

The reaction sequence is shown in Fig. 25. Methyl 2-*exo*-hydroxynorbornyl-7-*anti*-carboxylate [51] is prepared by a published procedure (25).

**Preparation of *anti*-7-Carboxymethylnorbornyl2,3-*exo*-Cyclic Carbamate [52]**

To a stirred solution of 0.60 g (3.53 mmol) of methyl 2-*exo*-hydroxynorbornyl-7-*anti*-carboxylate and 0.67 ml (8.25 mmol) of pyridine in 60 ml benzene at room temperature is added in one portion 0.68 g (2.3 mmol) triphosgene, and the resulting solution stirred for 1 1/2 hr. The solution is then quickly washed with saturated NaCl (3 x 6 ml), the organic layer is dried by filtration over anhydrous magnesium sulfate, and the solvent is removed by evaporation under reduced pressure. The resulting product is dissolved in 16 ml of dry DMF, and 0.77 g (11.9 mmol) of sodium azide is added. The solution is stirred at room temperature for 2 hr, 55 ml of water is added, and the solution is extracted with diethyl ether (5 x 30 ml). The organic extracts are combined and washed with saturated NaCl (3 x 32 ml), are dried by

filtration over anhydrous magnesium sulfate, and the solvent is removed by evaporation under reduced pressure. The product is purified by chromatography on silica gel to yield *anti*-7-carboxymethylcyclobutanyl-2-*exo*- azidoformate, which is dissolved in 5 ml dry methylene chloride. This solution was sealed in a glass reaction tube and is heated to 135°C for 1 hr. The solvent is removed by evaporation under reduced pressure and the product is purified by chromatography on silica gel to yield *anti*-7-carboxymethylnorbornyl-2,3-*exo*-cyclic carbamate [52].

**Preparation of 3-*exo*-(Benzyloxycarbonyl)amino-2-*exo*-Hydroxynorbornyl-7-*anti*-Carboxylate [53]**

0.143 g (0.68 mmol) of *anti*-7-carboxymethylnorbornyl-2,3-*exo*-cyclic carbamate and 0.384 g (6.88 mmol) of potassium hydroxide are dissolved in 20 ml of 1:1 (v:v) dioxane:water. The solution is refluxed for 23 hours. The resulting solution is extracted with diethyl ether (2 x 20 ml) and 5 ml of water added. The solution was cooled to 0°C and is vigorously stirred. Then 0.25 g (1.0 mmol) of carbobenzyloxy chloroformate is added, and the solution is stirred at room temperature for 1 hr. The mixture is acidified to pH 1 with 1M HCl and is extracted with methylene chloride (3 x 30 ml). The combined organic layers are washed with saturated NaCl (30 ml) and are dried by filtration over anhydrous magnesium sulfate. The solvent is removed by evaporation under reduced pressure and the product is purified by chromatography on silica gel to yield 3-*exo*-(benzyloxycarbonyl)amino-2-*exo*-hydroxynorbornyl-7-*anti*-carboxylate [53].

**Preparation of 3-*exo*-(Benzyloxycarbonyl)amino-2-Oxonorbornyl-7-*anti*-Carboxylate [54]**

To a stirred solution of 0.123 g (0.39 mmol) of 3-*exo*-(benzyloxycarbonyl)amino-2-*exo*-hydroxynorbornyl-7-*anti*-carboxylate in 20 ml acetone is added 0.235 ml (0.591 mmol) of Jones' reagent (prepared by dissolving 2.5 g chromium(VI) oxide in 2.15 ml concentrated sulfuric acid and diluting to 10 ml with water) at room temperature over a period of 15 s. After an additional 30 s, 2 ml of 2-propanol is added, the mixture is filtered, and the solvents are removed by evaporation under reduced pressure. The residue is taken up in 20 ml ethyl acetate, is washed with saturated NaCl (2 x 10 ml), and is dried by filtration over anhydrous magnesium sulfate. The solvent is removed by evaporation under reduced pressure and the product is purified by chromatography on silica gel to yield 3-*exo*-(benzyloxycarbonyl)amino-2-oxonorbornyl-7-*anti*-carboxylate [54].

**Example 15**

**Preparation of 3-*endo*-(Benzyloxycarbonyl)amino-2-*endo*-Hydroxynorbornyl-7-*anti*-carboxylate [63] and 3-*endo*-(Benzyloxycarbonyl)amino-2-Oxonorbornyl-7-*anti*-carboxylate [64]**

The syntheses of these compounds are identical to those discussed above for the *exo*-isomers [53] and [54], except that methyl 2-*endo*-hydroxynorbornyl 7-*anti*-carboxylate [61] is utilized in place of methyl 2-*endo*-hydroxynorbornyl-7-*anti*-carboxylate [51], as is shown in the reaction sequence in Fig. 26. Methyl 2-*endo*-hydroxy-norbornyl-7-*anti*-carboxylate [61] is prepared by a published procedure (26).

**Example 16**

**Preparation of Amidine Hapten Immunogen Coupling of (2-[(6-maleimido)butanoyl]amino]-3-phenyl-propylimino-D-alanine to Bovine Serum Albumin And to Keyhole Limpet Hemocyanin**

Two protein conjugates were prepared for the immunization of mice and for coating the solid phase in an enzyme-linked immunosorbent assay (ELISA) screening procedure for detection of antigen-specific antibody. Thiolated bovine serum albumin (BSA) was prepared by mixing 30 mg of BSA with a 100-fold molar excess of iminothiolane (Traut's reagent) (27), 5.1 mg of Traut's reagent in 1 M triethanolamine, at pH 8.0, for 90 min. at 4°C. BSA-SH was purified by size-exclusion chromatography on a PD-10 desalting column (Sephadex G-25M) equilibrated and eluted with 50 mM phosphate, pH 7.1. A 100-fold molar excess of (2-[(6-maleimido)butanoyl]amino]-3-phenyl- propylimino-D-alanine (hapten, prepared as described in Example 6 above) (2.8 mg in 0.5 ml) was resuspended in 50 mM phosphate, pH 7.1, and mixed for 3 hours at room temperature with 5 mg of BSA-SH. The hapten-BSA conjugate was purified from uncoupled hapten by gel filtration using 10 mM phosphate, pH 7.4, 0.15 M NaCl (PBS) as the equilbration and elution buffer. A free-thiol analysis (28) of the conjugate showed that the hapten to protein ratio was 23:1 (using a M.W. of 64,000 for BSA). Protein concentrations were determined by the bicinchoninic acid method (29), using BSA as the standard.

The amidine hapten keyhole limpet hemocyanin (KLH) conjugate was prepared in a similar fashion with 3 mg of KLH-SH and 1.7 mg of the amidine hapten-GMBS reagent, except that dialysis of the hapten-KLH conjugate against 10 mM phosphate, pH 7.4, 0.15 M NaCl (PBS) was performed instead of gel filtration to remove unconjugated hapten. The thiol analysis indicated that three haptens were coupled per KLH (using a M.W. of 64,000, for easier comparison to BSA).

**Immunization of Mice, Preparation of Hybridomas, and Screening of Antibodies for Binding Activity**

Twelve-week-old BALB/c mice were injected intraperitoneally (IP) with 10 µg of the amidine hapten-KLH conjugate emulsified in complete Freund's adjuvant. The mice were boosted with the hapten-KLH conjugate emulsified in incomplete Freund's adjuvant after three weeks with 10 µg and after another three weeks with 30 ug. The inverse serum titer was 51,200 by ELISA. A final intrasplenic injection of 10 µg of the conjugate in PBS was administered three days before the mice were sacrificed and the splenocytes prepared for fusion. A fusion was performed by standard methods (30) using SP2/0 myelomas as the fusion partner. Of the 45 hybridomas which produced anitbodies against the hapten-BSA conjugate by ELISA, 24 produced antibodies which bound the cys-maleimide-linker hapten in a competitive inhibition ELISA. Of those 24, 17 also reacted with the acetylated-hapten.

**Chemiluminescent Assay for Catalytic Activity**

The production of D-alanine was indirectly measured by the D-amino acid oxidase catalyzed conversion of D-alanine to its alpha-keto acid, which is accompanied by a stoichiometric production of hydrogen peroxide. The $H_2O_2$ is in turn utilized in the chemiluminescent reaction with luminol and the resulting luminescence measured on a Berthold Bilumat LB9500C luminometer.

Eleven ml of the supernatant fluids from 24 hybridomas were concentrated, then washed and resuspended in 0.75 ml of 20 mM PBS, pH 8.0. One ml of the substrate (1 mM Ac-D-phe-D-ala in 20 mM PBS, pH 8.0) was added to 0.5 ml of the antibody solutions and incubated overnight at 37°C. All solutions were filter sterilized through a 0.2 micron filter unit. A reaction cocktail was prepared one hour before the chemiluminescent assay was performed and was composed of:

- 990 µl of 50 mM phosphate buffered saline at pH 12, containing 10 mM EDTA (PBD-EDTA)
- 15 µl of a 4 mg/ml microperoxidase stock solution
- 75 µl of a 400 mg/ml solution of diazabicyclooctane (DABCO)
- 375 µl of a 250 mM luminol solution in PBS-EDTA.

To eliminate the contribution of any endogenous $H_2O_2$ from the antibody sample, 100 µl of the antibody/substrate sample was mixed with 97 µl of the reaction cocktail and incubated at room temperature for 15 - 30 min. Then 3 µl of D-amino acid oxidase (a 4.2 mg/ml stock in 3.6 M ammonium sulfate, pH 6.5) was added to the reaction mixture, mixed and the luminescence monitored over 6 min. Eight hybridomas yielded signals greater than two times the signal of a negative control antibody. Four of these hybridomas were stable antibody producers after cloning. Ascites fluids are prepared for confirmation assays on the HPLC.

**Example 17**

**Preparation of Phosphonate Ester Hapten Immunogen**

BSA-SH and KLH-SH were prepared as described the previous example. Five mg of each of the proteins were mixed with 5 mg of O-[(6-maleimido)hexanyl]amino](2-phenyl-ethyl) phosphinyl lactic acid disodium salt (hapten, prepared as described in Example 4 above) for 3 hours at room temp. The conjugates were dialyzed against PBS to remove the uncoupled hapten.

**Immunization of Mice, Preparation of Hybridomas, and Screening of Antibodies for Binding Activity.**

Eight-week-old BALB/c mice were immunized intraperitoneally (IP) with 50 µg of the hapten-KLH conjugate emulsified in complete Freund's adjuvant. The mice were boosted IP with 10 µg of the antigen in incomplete Freund's adjuvant after three and eight weeks. The inverse serum titer was 25,600 by ELISA. Four weeks after the last injection, 10 µg of the antigen in PBS was injected intrasplenically. The mice were sacrificed three days later and a fusion with the splenocytes and SP2/0 myelomas was performed by standard methods. Forty-two hybridomas produced antibodies against the hapten-BSA conjugate by ELISA and could be inhibited by the free-hapten in a competitive inhibition assay. All but one bound to both the Ac-hapten and the cys-maleimide-linker-hapten.

**Catalytic Antibody Assay**

Antibodies from 41 supernatant fluids were assayed directly for activity against Ac-L-phe-D-ala and Ac-D-phe-D-ala by the chemiluminescent assay described earlier with the following modifications. Two aliquots of unconcentrated supernatant fluids (0.5 ml each) were mixed with 1 ml of 1 mM substrate solutions and incubated at 37°C for 2-8 days. The reaction cocktail used a 2 mg/ml stock of horseradish peroxidase instead of the microperoxidase. Eight hybridomas yielded a luminescent signal greater than 2 times the negative control supernatant against the Ac-D-phe-D-ala substrate. None of the 41 hybridomas yielded a positive luminescent signal for the Ac-L-phe-D-ala substrate. Four of the eight hybridomas remained stable antibody producers after cloning. One of the four also yielded a positive luminescent signal for the Ac-L-phe-D-ala substrate. Ten-fold concentrates of the antibody supernatant fluids from the four cloned hybridomas were rescreened for catalytic activity against the Ac-D-phe-D-ala and the cys-maleimide-linker-D-phe-D-ala substrates by the following modified chemiluminescent assay. The concentrated antibody, 0.23 ml, was incubated with 0.48 ml of the 1 mM substrate stock solutions (in 20 mM Tris-HCl, pH 8.0) overnight at 37°C. An aliquot (34 μl) of the antibody/substrate mixture was then mixed with 66 μl of 20 mM Tris-HCl and 3 μl of D-amino acid oxidase. After three min., 97 μl of the reaction cocktail (71 parts of 50 mM Tris-HCl, pH 9.0; 25 parts of 250 mM luminol in Tris-buffer, and 1 part of micro peroxidase at 4 mg/ml in 10 mM Tris-HCl, pH 8.0) was injected and the luminescence measured. Two of the hybridomas yielded positive luminescent signals for both substrates greater than one standard deviation of the signal obtained with the antibody alone. Ascites fluids are then prepared, the antibodies are purified by methods known in the art and are used to catalyze peptide bond hydrolysis.

**Example 18**

**Preparation of Dialkyl Phosphinate Hapten Immunogen**

BSA-SH and KLH-SH were prepared as described earlier. [6-Maleimido)hexanoyl]amino-2-phenylethyl(2-carboxy-1-propyl)hydroxyphosphonous acid (6mg), the hapten as prepared in Example 5 above, was dissolved in water and mixed with 5 mg of each of the two proteins for three hours at room temperature. The conjugates were dialyzed against PBS. The free thiol analysis showed that the ratio of hapten to BSA and KLH (using a M.W. of 64,000) was 8:1 and 3:1, respectively.

**Immunization of Mice, Preparation of Hybridomas, and Screening of Antibodies for Binding Activity**

Nine-week-old BALB/c mice were immunized intraperitoneally (I.P.) with 50 μg of the dialkyl phosphinate hapten-KLH conjugate emulsified in complete Freund's adjuvant. The mice were boosted IP after three, seven, ten, and 17 weeks with 10 μg of the conjugate emulsified in incomplete Freund's adjuvant. The inverse serum titer was 102,400 by ELISA. The final injection with 10 μg of the conjugate in PBS was given intrasplenically six weeks later. The mice were sacrificed three days after the last injection. A fusion was performed by standard methods using the BALB/c splenocytes and SP2/0 myelomas as the fusion partner. The hybridomas were screened for hapten-BSA specific antibody production by ELISA. Ten hybridomas produced antibodies which were competitively inhibited by Ac-hapten from binding to the hapten-protein conjugate. These hybridomas are then cloned to insure monoclonality.

**Example 19**

**Preparation of Phosphonamidate Immunogens**

BSA-SH and KLH-SH were prepared as described earlier except that the uncoupled Traut's reagent was removed by gel filtration with 50 mM phosphate at pH 8.4. The hapten, [(6-maleimido)hexanoyl]amino](2-phenylethyl)hydroxyphosphinyl D-alanine, prepared as described above in Example 3, (7-10 mg) was divided into two portions and mixed with 5 mg each of BSA-SH and KLH-SH at room temperature for three hours. The conjugates were dialyzed against PBS at pH 8.0 and then stored frozen at -20°C. The thiol determination results indicated that 26 hapten were conjugated to the BSA molecules and 12 haptens to KLH (assuming a M.W. of 64,000). These conjugates were injected into BALB/c mice.

**Example 20**

**Methodology For The Production, Screening And Isolation Of Monoclonal Catalytic Antibodies That Cleave The "Flap" Region Of Human Renin.**

The inhibition of renin, an aspartic proteinase whose action initiates the renin-angiotensin cascade has been the object of intense investigation in recent years. The potential for treatment of hypertension through the inhibition of renin has resulted in the synthesis of a variety of potent renin inhibitors based on the peptide sequence of the natural substrate angiotensinogen. An alternative approach is the use of a proteolytic antibody to renin.

It has been reported that the flap region of human renin is a hairpin with a loop region of four amino acid residues leading to the carbonyl group of Tyr 83 interacting with the amino groups of Thr 85 and Gly 86 (31). The cyclic decapeptide

CLRYSTGTVC,

[80-89] human renin, was found to adopt this same hairpin structure.

In this example, monoclonal antibodies are elicited with a difluroketone containing immunogen according to the invention. The monoclonal antibodies will catalyze the cleavage of the peptide sequence

```
81                 85  86               90
Leu-Arg-Tyr-Ser-Thr-Gly-Thr-Val-Ser-Gly
```

in the human renin molecule between residues 85 and 86. Cleavage causes disruption of the catalytic machinery of the enzyme since residues 85 and 86 constitute the "flap" region which holds the substrate in the catalytic site (32). It has previously been demonstrated that rabbit polyclonal antiserum elicited to a synthetic peptide fragment (sequence 81-90) could inhibit human renin activity by 40% as measured by its reaction with synthetic human tetradecapeptide substrate (33).

In order to raise a catalytic antibody to this flap region of human renin, the cyclic peptide hapten

CLRYST(TS)GTVC,

where (TS) represents an analog of the target amide (peptide) bond is synthesized. In this example, the synthesis of the cyclic peptide hapten

CLRYST(TS)GTVC

follows the solid-phase approach (34) wherein ST(TS)G, corresponds to a difluoroketone transition state analog tripeptide in accordance with the invention and is incorporated into the assembled peptide through its anhydride (34). The completed peptide is fully deprotected and cleaved from the solid support using trifluoroacetic acid. Cyclization of the peptide is achieved by allowing the peptide to stand at low concentration for one hour in an aqueous solution (pH 8) in order to generate a disulphide bridge between the two terminal cysteine residues. The N-terminal amino group of the peptide allows it to be attached to a carrier protein for immunization of mice.

**A. Preparation of the Immunogen**

**1. Peptide Synthesis**

The peptide hapten is synthesized by the solid phase technique using the polyamide-Kieselguhr composite resin (34). The side chain protecting groups are the following: O-tert-butyl (tyrosine, glutamic acid, serine, threonine); N-4-methoxy-2,3,6-trimethyl benzenesulphonyl (arginine); and S-trityl (cysteine). The temporary protection of the N

function is by fluorenylmethoxycarbonyl which is removed in 10 minutes with piperidine/DMF : 20/80. The coupling reactions are carried out using FMOC- amino acid anhydrides (34). The protected peptidyl-resin is fully deprotected by treatment with trifluoroacetic acid/thioanisole/m-cresol/thiophenol/ ethanedithiol solution: 90/2/2/2/4 for 3 hrs. After filtration the filtrate is concentrated under vacuum to a small volume. Ether is added to give a precipitate of the peptide. The ethereal supernatant is removed and the peptidic precipitate is washed twice with ether to yield the peptide hapten

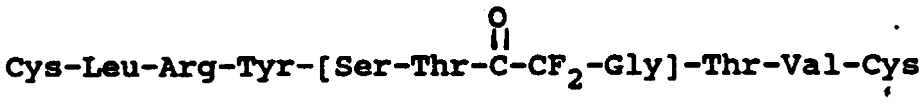

wherein the bracketed moiety is a difluoroketone transition state analog tripeptide isostere of the invention.

2. <u>Cyclization of Peptide Hapten</u>

The peptide hapten is cyclized to obtain the " β-hairpin" conformation by forming a disulphide bond between the two terminal cysteine residues. The disulfide bond is completed in one hour by air oxidation of an aqueous solution (pH 8) at a concentration of 0.3 mg of peptide per milliliter. The oxidized product is then removed by lyophilization and purified by HPLC.

3. <u>Conjugation of the Hapten to the Carrier Molecule</u>

The cyclic peptide hapten

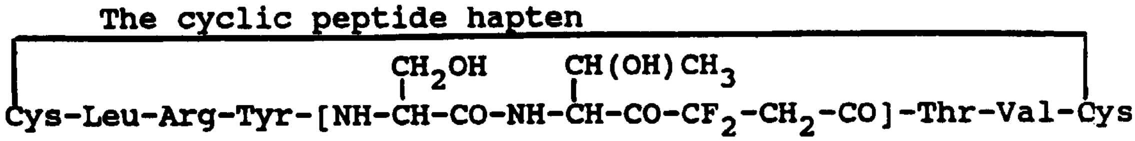

is conjugated to keyhole limpet hemocyanin (KLH) using glutaraldehyde (35). Coupling efficiency is 50-80% as estimated by binding of a trace amount of I125 peptide added to the reaction mixture.

B. <u>Preparation and Screening of Monoclonal Antibodies</u>

KLH conjugated peptide (50 μg) in complete Freund's adjuvant is injected into BALB/c mice. Hybridoma fusions are carried out by standard methods using SP2/0 myeloma as the fusion partner. Polyclonal antiserum response and hybridoma secreting cells resulting from the fusion are screened for binding activity by ELISA.

Wells of plastic microtiter plates (Falcon 3915 Probind, Becton Dickinson Labware CA, USA) are coated with 50 μL of peptide or renin (5 μg/mL) in Tris-HC1 buffer (0.1 M, pH 9.6).

Plates are first incubated for 30 minutes at 37°C and then overnight at room temperature. After washing three times with Tween- containing phosphate-buffered saline (PBS-Tween 0.1%, pH 7.4), 50 μL of serial dilutions of antisera in PBS-BSA 1% pH 7.4 are added in peptide- coated duplicate wells and incubated for 2 hours at 37°C. Plates are washed three times again with PBS-Tween 0.1% and wells are then treated with 50 μL of alkaline phosphatase-labelled goat anti-mouse IgG diluted 1:500 (Sigma, MO, USA). Incubation is carried out for 1 hour at 37°C.

Additional extensive washing with PBS-Tween 0.1% is followed by incubation with 150 μL of alkaline phosphatase substrate (2 tablets/10 ml of Sigma 104-105) dissolved in 0.1 M glycine-NaOH buffer (pH 10.4) containing MgCl2 and ZnCl2, 1/L. The enzymatic reaction is allowed to proceed for 2 hours at 37°C and stopped by addition of 50 uL of Na2CO3 (1.5 <u>M</u>).

Absorbance is read at 405 nm in a Titerteck multiskan ELISA reader (Flow laboratories). Titer expression is determined by multiplying the optical density by the maximal dilution giving an absorbance three times as high as the negative control (consisting of pooled normal mouse sera diluted 1:100).

Hybridomas giving a positive reaction in this screening assay against the peptide, renin or both are chosen for further study. IgG is purified from ascites fluid by HPLC with a Bakerbond ABx HPLC column.

C. Catalysis of Peptide Cleavage by Catalytic Antibody Specific for the "Flap" Region of Human Renin.

The decapeptide substrate
$H_2N$-Leu-Arg-Tyr-Ser-Thr-Gly-Thr-Val-Ser-Gly-$CO_2H$
(2.7 µM) is incubated with the catalytic antibodies produced by the procedure outlined above and the reaction monitored by reverse phase HPLC analysis of the mixture. The reaction is carried out at pH values optimal for high $K_{cat}$ by the catalytic antibody (optimum pH is determined employing the chromogenic p-nitroanilide substrate

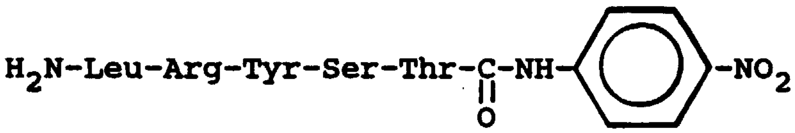

or the fluorescent coumarin substrate

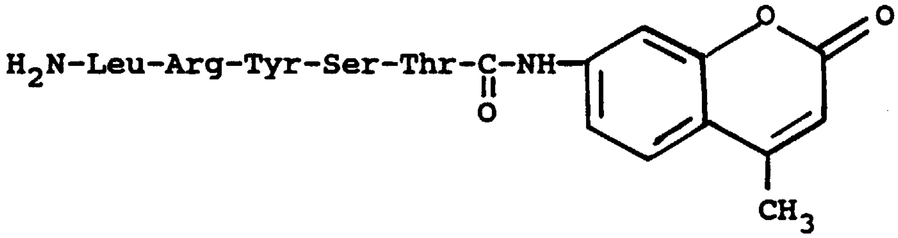

and taking into account the binding energy of the catalytic antibody for the residues on the C-terminal side of the cleavage site with change in pH).

Antibodies that show the best $K_{cat}$ values for cleavage of the decapeptide substrate are tested for their ability to inhibit human renin.

D. Binding of Anti-Transition-State Analog Antibodies to Human Renin and Inhibition of its Enzymatic Activity by Cleavage of Residues 85-86 in the "Flap" Region.

Inhibition of plasma renin activity - the ability of the catalytic antibodies to inhibit renin activity - is tested on a pool of human plasma having a high renin activity (40 ng of angiontensin I/h/mL). Plasma (25 µl) is pre-incubated with 100 µl of the catalytic antibody in PBS (pH 7.5) containing 1% EDTA (final volume 0.2 mL) for various periods of time. Next, an excess of plasma renin substrate (200 pmol) is added in order to ensure zero-order behavior, and the mixture is incubated for 30 min. at 37°C in PBS (pH 5.7). The final dilution of the catalytic antibody is 1:5 and 1:50. The angiotensin I generated is measured by radioimmunoassay (36). A blank is included using the same dilutions of the corresponding abzymes. The amount of angiotension I generated is less than that observed with intact renin, thus indicating cleavage of residues 85-86 in the "flap" region and inhibition.

Example 21

In vivo Preparation of Immunogenic Transition-State Analog Containing Peptide from HIV gp120 Coat Protein

A. Preparation of the Immunogen

The octapeptide sequence -Ala-Ser-Thr-Thr-Thr-Asn-Tyr-Thr- from HIV gp120 coat protein is important for virus interaction with the OKT4 antigen of T4 helper/inducer cells. Synthetic peptides identical or very similar in sequence to this octapeptide strongly inhibit attachment of HIV to the antigen receptor (37). Computer assisted searches have demonstrated homology to a peptide found in the envelope region of the Epstein-Barr virus. In addition, strong homologies exist between the HIV octapeptide and peptides which occur in human lymphoadenopathy virus (LAV) and in human T-cell leukemia virus (HTLV-IIIb) isolates. An additional homology exists between the HIV peptide and a sequence comprising residues 19-26 of bovine pancreatic ribonuclease A (RNase A). This sequence contains

the exposed subtilisin cleavage sites of RNase A between residues 20 and 21 and 21 and 22 (38).

Peptide haptens according to the invention are:

(1) -Ala-[Ser-U-Thr]-Thr-Thr-Asn-Tyr-Cys
(2) -Ala-Ser-[Thr-U-Thr]-Thr-Asn-Tyr-Cys
(3) -Ala-Ser-Thr-[Thr-U-Thr]-Asn-Tyr-Cys
(4) -Ala-Ser-Thr-Thr-[Thr-U-Asn]-Tyr-Cys

The dipeptide analogs isosteres (indicated in brackets) wherein U represents various amide bond analog arrays of atoms of the haptens of the invention (e.g., U is A in haptens of formula I and U is A-Z in haptens of formula IV) atoms as described earlier are incorporated into the peptides as described in Example 19. Each peptide hapten is coupled with keyhole limpet hemocyanin (KLH) carrier protein through the terminal cysteine residue utilizing the cross-linker m-maleimidobenzoyl-N-hydroxysuccinimide ester (39).

### B. Preparation of Monoclonal Antibodies

BALB/C mice are immunized with the KLH-peptide analog conjugates emulsified in complete Freund's adjuvant. A blood sample is obtained from each mouse and the serum separated by centrifugation and stored at 4°C. Sera obtained in this way are screened for binding activity to the original transition-state analog immunogen by standard ELISA procedures. Antibody producing mice immunized as described above and assayed for reactivity with the transition state analog peptide immunogens are sacrificed and their spleens removed and hybidoma cells are prepared using SP2/0 myeloma cells as described in Example 19B above.

### C. Screening Hybridoma Cells Producing Catalytic Monoclonal Antibodies

Screening for binding of antibodies to respective transition state analog-containing peptides is performed essentially as described in Example 6 above. Hybridomas secreting monoclonal antibodies and giving a positive binding reaction are chosen for further study. IgG is purified from ascites fluid by HPLC with Bakerbond $AB_X$HPLC.

## Example 22

### In vitro Elicitation of Catalytic Monoclonal Antibodies Against A Viral Epitope That Selectively Inhibits Human Immunodeficiency Virus (HIV)

### A. Preparation of the Immunogen

The dipeptide transition state isostere,

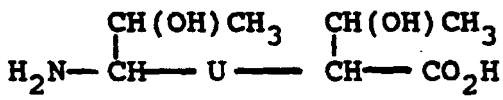

wherein U is as hereinbefore described, is incorporated into a peptide as described in Example 19 to give the hapten

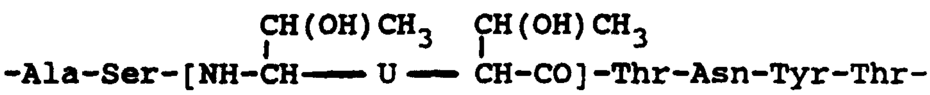

The resulting hapten is designed to mimic a portion of the HIV gp120 goat protein. The synthetic peptide is used in an in vitro immunization procedure using a modification of a literature procedure (40). Spleen cells are cultured at 106 cell/ml in a medium consisting of 50% fresh Eagle's MEM with 20% fetal bovine serum 5 x $10^{-5}$M β-mercaptoethanol, 2mM glutamine and 50% conditioned medium from BALB/c mouse thymocytes. To furnish conditioned medium, thymocytes are cultured at 3-5 x $10^6$ cells/mL in the same Eagles MEM medium as above. After 48-72 hours the medium is removed, sterilized by filtration and used immediately for in vitro activation. The antigen is added to the spleen cell culture medium at concentrations equivalent to approxamately 1 μg peptide/ml. Spleen cells are cultured in the presence

of antigen for four days without a change in the medium and are later hybridized.

Hybridizidations are carried out with the mouse plasmacytoma cell line 45 6TGL.7 (41) obtained from the Cell Distribution Center of the Salk Institute. Spleen cells (either freshly isolated or from in vitro activation cultures) and plasmacytoma cells are washed twice in serin-free Eagles MEM, then fused using PEG 1000 as previously described in the literature (42). Hybrids are selected by treatment of the cultures with HAT (43). Hybrid cells which produce antibody reactive with the peptide transition state analog (as judged by an ELISA assay) are cloned and re-cloned by limiting dilution in conditioned medium from parental plasmacytoma cells.

Screening for binding of antibodies to respective transition state analog-containing peptides or the virus itself is performed essentially as described in Example 19 above. Hybridomas secreting monoclonal antibodies giving a positive binding reaction are chosen for further study. IgG is purified from ascites fluid by HPLC with Bakerbond ABxHPLC. One of ordinary skill in the art will realize that these antibodies can be tested against the peptide not containing the transition state analog.

The virus replication assay is carried out essentially as described in the literature (44), except that cultures are propagated in microtube wells containing 200 μL. Graded concentrations of purified antibodies obtained from the in-vitro immunization procedure or buffer alone, each in 25 μL, are preincubated for I hr. at 37°C in 5% CO2 with 50 TCID50 HTLV-IIIB in 25 μL. Following preincubation, H9 cells (1 x $10^5$ cells in 150 μL RPM1-040 supplemented with 20% heat-inactivated FCS) are added to the wells, yielding final antibody concentrations ranging from 0.1 μgm1-1 to 10 μg $m1^{-1}$. Microtiter plates are incubated at 37°C in 5% $CO_2$ for 14 days. Cells are fed by exchanging 100 μL cell-free supernatant fluid on days 3, 7 and 10 with fresh medium, and no further antibody is added during this period. Cell-free supernatant fluid (100 μL) is analyzed for p24 antigen by RIA (Dupont, NEK-040). Since the amount of p24 correlates with the degree of infection and replication of the virus, those wells treated with catalytic antibodies having significant decreases in p24 when compared to virus treated with control antibodies demonstrate the inhibition resulting from cleavage of gp120 by the catalytic antibody.

The C8166 fusion assay is described in the literature (45). Three monoclonal antibodies (clone AHIV1.6; AHIV1.3 and AHIV2.0) are tested in 2 hr. assays. H9 cells (1 x $10^4$) infected chronically with HTLV-IIIB are preincubated with varying concentrations of antibody in 150 μL medium in 96-well plates. All assays are done in triplicate. After 1 hr. incubation at 37°C in 5% $CO_2$, 3 x $10^4$ C8166 cells (HTLV-I transformed umbilical cord lymphocytes) in 50 μL are added to the wells. Final well concentrations of antibodies are 41 $\mu gml^{-1}$ and 5 $\mu gm^{-1}$. Preincubation with OKT4A (Ortho Diognostics) at 25 $\mu gm1^{-1}$ served as a control. After the plates are incubated for 2 hrs. at 37°C in 5% $CO_2$, syncytia (ballooning cytoplasm greater than three lymphocyte cell diameters) are counted. To prevent bias during counting, samples are coded.

The antiviral activity of clones AHIV 1.3, AHIV 1.6 and AHIV 2.0 is examined in HIV-I replication and cell fusion assays. Fig. 27 shows the dose dependent inhibition by clone AHIV 1.3 of HIV-1 p. 24 gag production in infected H9 cells. Fig. 28 shows the dose dependent inhibition by clones AHIV 1.3, AHIV 1.6 and AHIV 2.0 of HIV-induced cell fusion.

The catalytic monoclonal antibodies elicited by in vitro immunization with the transition state dipeptide isostere analog incorporated into the exposed peptide sequence of HIV gp120 can prevent infection by HIV virus by causing rupture of an important region of the viral coprotein involved in binding to the CD4 receptor on lymphocytes. The catalytic monoclonal antibodies break the peptide bond in the chosen sequence (i.e., between the first two threonine residues from the left of the octapeptide shown in Example 20) in a manner analogous to the action of proteolytic enzymes. The mechanism of the antibody catalyzed hydrolysis of the octapeptide or gp120 does not involve a metal ion and consequently may involve either a nucleophile in the active site of the antibody or nucleophilic addition of water activated by amino acid residues in the antibody combining site.

## Example 23

### Site Specific Cleavage of the Threonine-Glyaine Bond, Residues 9 and 10, in a Tetradecapeptide Containing Two Other Threonine-Glycine Bonds, Residues 4, 5 and 12, 13

In this example, catalytic antibodies are elicited for the purpose of cleaving the threonine-glycine bond at residues 9 and 10 in a tetradecapeptide containing two other threonine-glycine bonds, one at residues 4 and 5 and the other at residues 12 and 13.

Catalytic antibodies are elicited by the methodology described in Example 19 to the tetradecapeptide immunogen:

Ala-Val-Leu-$Thr_4$-$Gly_5$-Ala-Val-[Ser-$Thr_9$-T.S.-$Gly_{10}$]-Tyr-$Thr_{12}Gly_{13}$-Val.

The tripeptide transition state analog isostere,

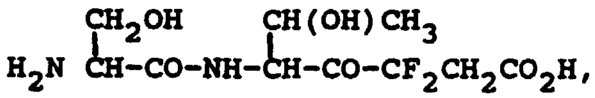

represented in the immunogen by [Ser-Thr-T.S.-Gly] is incorporated into the assembled peptide through its anhydride, as described in Example 19, to yield the tetradecapeptide immunogen shown above. Antibodies isolated from ascites fluid by $AB_x$HPLC chromatography and identified as being specific for the tripeptide transition state analog isostere in the tetradecapeptide immunogen are incubated for approximately one hour at 37°C with the tetradecapeptide substate: Ala-Val-Leu-Thr-Gly-Ala-Val-Ser-Thr-Gly-Tyr-Thr-Gly-Val.

The reaction mixture is then analyzed by HPLC. Acetic acid (3M) is added and the samples are centrifuged for 10 minutes. An aliquot of the resulting supenatant is analyzed by HPLC using a Vydac C- 18 analytical column. Absorbance at 214 nm is monitored and fractions are collected. Resultant peptide fractions are analyzed by standard amino acid analysis and sequencing. Analysis of the cleavage products from the action of the catalytic anti-transition-state analog antibodies shows that two peptide fragments, a nonapeptide, $H_2$N-Ala-Val-Leu-Thr-Gly-Ala-Val-Ser- Thr-$CO_2$H, and a pentapeptide, $H_2$N-Gly-Tyr-Thr-Gly-Val-$CO_2$H result. No cleavage is seen at the other Thr-Gly residues in the original tetradecapeptide substrate indicating the extreme specificity of the antibody to only the site of introduction of the tripeptide transition state analog isostere of the invention.

**REFERENCES**

1. See The Chemistry of Enzyme Action, Chapter 1, M.I. Page, editor (elsevier, Amsterdam 1984).
2. A.D. Napper et al., "A Stereospecific Cyclization Catalyzed by an Antibody", Science, 237, 1041-1043 (1987).
3. A. Tramontano et al., "Chemical Reactivity at an Antibody Binding Site Elicited By Mechanistic Design of a Synthetic Antigen", Proc. Natl. Acad, Sci, USA, 83, 6736-6740 (1986).
4. H. White and W.P. Jencks, J. Biol. Chem., 251, p. 1688 (1976); H. White et al., ibid, 1700.
5. W.P. Jencks, Syniposia on Quantitative Biology, 52, 65 (1987).
6. H.M. Geysen et al., J. Immonological Methods, 102, 259-274 (1987).
7. H.M. Geysen et al., Proc. Nat'l Acad. Sci. USA, 82, 178-182 (1985).
8. J.A. Berzofsky, Science, 229, 932-940 (1985).
9. T.P. Hopp and K.R. Woods, Proc. Nat'l. Acad. Sci, USA, 78, 3824-3828 (1981).
10. J. Novotny et al., Proc. Nat'l Acad. Sci., 226 1986).
11. H.M. Geysen et al., Science, 235, 1184 (1878).
12. A. Fersht, Enzyme Structure and Mechanism, 2d ed., 433-436 (W.H. Freeman and Co., New York, 1985).
13. D. Piszkiewiewicz et al., Biochem. Biophys. Res. Comm., 40(5), 1173-1178 (1970).
14. T. Geiger and S. Clark, J. Biol. Chem., 262(2), 785-793 (1987).
15. M. Shimazaki et al., Chem. Pharm. Bull., 30, 3139 (1982).
16. G. Osapay et al., J. Chem. Soc. Perkin Trans. I, 2485 (1984).
17. See D. Herschlag, "The Role of Induced Fit and Conformational Changes of Enzymes in Specificity and Catalysis", Bioorganic Chemistry, 16, 62-96 (1988).
18. E.K. Baylis et al., J. Chem. Soc. Perkin Trans. I, 2485 (1984).
19. D.M. Floyd, A.W. Fritz, J. Pluscec, E.R. Weaver, and C.M. Cimarusti, J. Org. Chem., 47, 5160 (1982).
20. E. Duranti and Bonifazi, P. Synthesis 1977, 494.
21. J. Finkelstein, K.G. Holden, R. Sneed, and C.D. Perchonock, Tet. Letters 22, 1855 (1977).
22. S. Kishimoto, M. Sendai, M. Tominoto, S. Hashiguchi, T. Matsuo, and M. Ochiai, Chem. Pharm. Bull. 32, 2646 (1984); W.F. Huffman, K.G. Holden, T.F. Buckley, III, J.G. Gleason, and L. Wu, J. Am. Chem. Soc. 99, 2352 (1977).
23. D.D. Miller, F.-L. Hsu, K.N. Salman, and P.N. Patil, J. Med. Chem. 19, 180 (1976).
24. K.B. Wiberg, G.M. Lampman, R.P. Cuila, D.S. Connor, P.Schertler, and J. Lavanish, Tetrahedron 21, 2749 (1965).
25. P. Flury and C.A. Grob, Helv. Chem. Acta 66, 1991 (1983).
26. P. Flury and C.A. Grob, Helv. Chem. Acta 66, 1991 (1983).
27. J.M. Lambert et al., Biochem., 22, 3913-3920 (1983).
28. G.L. Ellman, Arch. Biochem. Biophys., 82, 70-77 (1959).
29. P.K. Smith et al., Anal. Biochem., 150, 76-85 (1985).
30. S. Fazickas de St. Groth et al., J. Immunol. Met., 35, 1021 (1985).

31. C. Liu et al., Tetrahedron, 44(3), 675-683 (1988).
32. J. Blundell et al., Nature, 304, 273-275 (1983).
33. P. Corvol et al., J. Biol. Chem., 262(6), 2913-2918 (1987).
34. E. Atherton and R.J. Sheppard, J. Chem. Soc. Commun., 1151-1152 (1981).
35. M. Wilchek and S. Banniger, Methods Enzymol., 70, 151-159 (1980).
36. J. Menard and K.J. Catt, Endocrinology, 90, 422-430 (1972).
37. C.B. Pert at al., Proc. Nat'l. Acad. Sci, USA, 83, 9254-9258 (1986).
38. M.R. Pincus at al., Biochem. Biophys. Res. Commun., 143(a), 248-251 (1987).
39. D.M. Katz at al., Biochemistry, 18, 690-697 (1979).
40. Anderson at al., CRU, 10 27 (1977).
41. M.D. Schaff at al., Cell, 8, 405 (1976).
42. C. Milstein at al., Nature, 266, 550 (1977).
43. J.W. Littlefield, Expt'l. Cell Res., 41, 190 (1966).
44. D. Ho, J. Virol., 61, 2024 (1987).
45. B.D. Walker at al., Proc. Nat'l Acad. Sci. USA, 84, 8120 (1987).

## Claims

**1.** An immunogen comprising:

(a) a cyclic amide of formula:

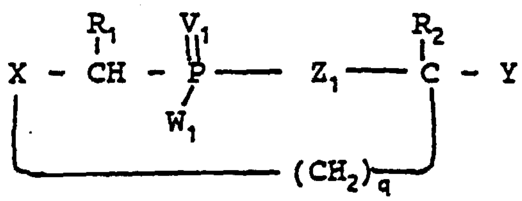

wherein $R_1$ and $R_2$ may be the same or different and each is a side chain of a naturally occurring amino acid, a hydroxy-containing side chain of a naturally occurring amino acid wherein said hydroxy group may be glycosylated, phosphorylated, sulphonylated or protected by a hydroxy protecting group, a primary amido containing side chain of a naturally occurring amino acid wherein said amido group may be glycosylated, or $C_{1-4}$ alkyl, $-CH_2\ CH(CO_2H)_2$, $-(CH_2)_2S(O)CH_3$, $-(CH_2)_2S(O)_2CH_3$, $-(CH_2)_3NH_2$ or $-(CH_2)_3ONHC(=NH)NH_2$;
X is oxygen, amino, amino protected by a protecting group selected from the group consisting of terminal amino protecting groups, amino bonded to the C-terminus of a naturally occurring amino acid to form a peptide bond, amino bonded to the C-terminus of a peptide to form a peptide bond, said amino acid and peptide being unprotected or protected by said protecting group, or X is alkene, ($C_1$-$C_9$) alkyl, ($C_1$-$C_9$) alkoxy phenyl, phenoxy, cyclohexyl, phenylthio, phenylsulfinyl or phenylsulfonyl wherein the aforementioned phenyl groups may be unsubstituted or mono-, di- or trisubstituted by halogen, ($C_1$-$C_4$) alkyl, ($C_1$-$C_4$) alkoxy or ($C_1$-$C_4$) alkcoxycarbonyl;
Y is hydrogen, carboxyl, carboxyl protected by a protecting group selected from the group consisting of terminal carboxyl protecting groups, a carbonyl bonded to the N-terminus of a naturally occurring amino acid to form a peptide bond, carbonyl bonded to the N-terminus of a peptide to form a peptide bond, said amino acid and peptide being protected or unprotected by said protecting group, or Y is ($C_1$-$C_9$) alkyl, ($C_1$-$C_9$) alkoxy, phenyl, phenoxy, cyclohexyl, phenylthio, phenylsulfinyl or phenylsulfonyl wherein the aforementioned phenyl groups may be unsubstituted or mono-, di- or trisubstituted by halogen, ($C_1$-$C_4$) alkyl, ($C_1$-$C_4$) alkoxy or ($C_1$-$C_4$) alkoxycarbonyl; and
wherein each of $R_1$, $R_2$, X and Y may be unbound or bound to one or more of $R_1$, $R_2$, X and Y and if bound, then by a covalent bond or a linker moiety selected from the group consisting of $-(CH_2)_s-S-S-(CH_2)_t-$, $-(CH_2)_t-$, $-S-(CH_2)t-S-$, $-(CH_2)_s-S(CH_2)_t-$, $-(CH_2)_s-CH = CH-(CH_2)_t-$, $-(CH_2)_s-NH-CO-(CH_2)_t-$, $-(CH_2)_s-NH-(CH_2)_t-$ and $-(CH_2)_s$-phenyl-$(CH_2)_t-$;
$W_1$ is OH, $NH_2$, SH or H;

$V_1$ is O or S;
$Z_1$ if unbound is O, NH, $CH_2$ or S and if bound is N or CH;
s and t may be the same or different and each is 0 or an integer from 1 to 10 unless the linker moiety is -$(CH_2)_t$- in which case t is an interger from 1-10.
q is 0 or an integer from 1 to 10; and

(b) a carrier molecule, said amide being coupled to said carrier molecule by a suitable coupling moiety.

2. An immunogen according to Claim 1 in which $V_1$ is O, $W_1$ is OH, SH or H, $Z_1$ is O, NH or $CH_2$ and q is 0 or 1.

3. An immunogen comprising:

(a) a cyclic amide of formula:

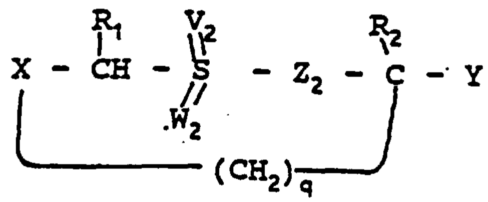

wherein $R_1$, $R_2$, X, Y, and q are as defined in Claim 1 and wherein
$V_2$ is O or a lone pair of elctrons;
$W_2$ is O or a lone pair of electrons;
$Z_2$ if unbound to said linker moiety is O, NH or $CH_2$ and if bound is N or CH; and

(b) a carrier molecule, said amide being coupled to said carrier molecule by a suitable coupling moiety.

4. An immunogen according to Claim 3 in which $V_2$ and $W_2$ are O or a lone pair of electrons, $Z_2$ is O, $CH_2$ or NH and q is 0 or 1.

5. An immunogen comprising:

(a) a cyclic amide of formula:

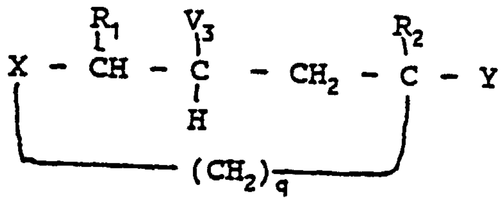

wherein $R_1$, $R_2$, X Y, and q are as defined in Claim 1 and wherein $V_3$ is OH or $NH_2$; and

(b) a carrier molecule, said amide being coupled to said carrier molecule by a suitable coupling moiety.

6. An immunogen according to Claim 5 in which q is 0 or 1.

7. An immunogen comprising:

(a) a cyclic amide of formula:

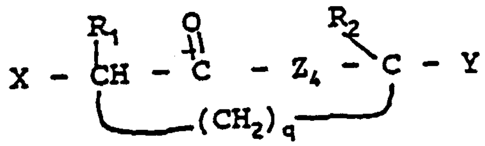

wherein $R_1$, $R_2$, X, Y and q are as defined in Claim 1 and wherein $Z_4$ if unbound is $CF_2$ or $CF_2CO$ and if bound is CF or CFCO; and

(b) a carrier molecule, said amide being coupled to said carrier molecule by a suitable coupling moiety.

**8.** An immunogen according to Claim 7 in which $Z_4$ is $CF_2$.

**9.** An immunogen according to Claim 8 in which q is 0 or 1.

**10.** An immunogen comprising:

(a) a cyclic amide of formula:

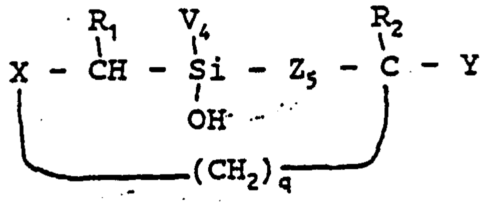

wherein $R_1$, $R_2$, X, Y and q are as defined in Claim 1 and wherein
$V_4$ is OH or $NH_2$;
$Z_5$ if unbound is O or $CH_2$ and if bound is CH; and

(b) a carrier molecule, said amide being coupled to said carrier molecule by a suitable coupling moeity.

**11.** An immunogen according to Claim 10 in which $V_4$ is OH.

**12.** An immunogen according to Claim 10 in which $V_4$ is OH, $Z_5$ is $CH_2$ and q is 0 or 1.

**13.** An immunogen according to any of Claims 1, 3, 5, 7, or 11 in which $R_1$ is selected from $CH_2OH$ and $CH(OH)CH_3$; $R_2$ is selected from $CH(OH)CH_3$ and $CH_2CONH_2$; X is selected from Ala-NH, Ala-Ser-NH, Ala-Ser-Thr-NH and Ala-Ser-Thr-Thr-NH; and Y is selected from CO-Thr-Thr-Asn-Tyr-Cys, CO-Thr-Asn-Tyr-Cys, CO-Asn-Tyr-Cys and CO-Tyr-Cys.

**14.** An immunogen according to any of Claims 1, 3, 5, 7, and 11 in which $R_1$ is $CH(OH)CH_3$; $R_2$ is H, X is Cys-Leu-Arg-Tyr-Ser-NH and Y is CO-Thr-Val-Cys.

**15.** An immunogen according to Claim 14 wherein said hapten has β-turn configuration mimicking the configuration of native protein wherein the sulfur atoms in the two terminal cysteine residues are joined to form a disulphide bridge.

**16.** A method for catalyzing the cleavage or formation of a specific amide bond within a molecule which comprises contacting said molecule with an amount of a monoclonal antibody effective to catalyze the cleavage or formation of said amide bond under conditions suitable for said cleavage or formation to take place, said monoclonal antibody having been prepared by a process comprising the steps of:

(a) selecting the specific amide bond to be cleaved or formed;
(b) selecting an immunogen according to any of Claims 1-15 and comprising an analog of said amide bond to be cleaved or formed, and also comprising moieties surrounding said analog of said amide bond, said moieties substantially corresponding to some or all of the moieties surrounding the amide bond to be cleaved or formed;
(c) exposing cells capable of producing antibodies to said immunogen and thereby generating antibody producing cells;
(d) hybridizing said antibody producing cells with myeloma cells and thereby generating a plurality of hybridoma cells each producing monoclonal antibodies; and
(e) screening said plurality of monoclonal antibodies to identify a monoclonal antibody which catalyzes the cleavage or formation of said amide bond.

**17.** A method according to claim 16 for catalyzing the cleavage or formation of a specific amide bond within a specific amino acid sequence contained in the molecule.

**18.** A method according to claim 17, said monoclonal antibody having been prepared by a process comprising the steps of:

(a) selecting the specific amide bond to be cleaved or formed;
(b) selecting an immunogen comprising a dipeptide analog which (i) mimics one or more high energy intermediates or transition states in the cleavage or formation of said amide bond or (ii) mimics one or more high energy conformations of said amide or ester bond or (iii) mimics both one or more high energy intermediates or transition states in the cleavage or formation of said amide bond and one or more high energy conformations of said amide bond, and said immunogen also comprising moieties surrounding the dipeptide analog which substantially correspond to some or all of the moieties surrounding the amide bond to be cleaved or formed;
(c) exposing cells capable of producing antibodies to said immunogen and thereby generating antibody producing cells;
(d) hybridizing said antibody producing cells with myeloma cells and thereby generating a plurality of hybridomna cells each producing monoclonal antibodies; and
(e) screening said plurality of monoclonal antibodies to identify a monoclonal antibody which catalyzes the cleavage or formation of said amide bond.

**19.** A method for producing catalytic antibodies capable of catalyzing the cleavage or formation of an amide bond comprising:

(a) exposing cells capable of producing antibodies to an immunogen according to any of Claims 1-15 and thereby generating antibody producing cells,
(b) hybridizing said antibody producing cells with myeloma cells and thereby generating a plurality of hybridoma cells each producing monoclonal antibodies, and
(c) screening said plurality of monoclonal antibodies to identify a monoclonal antibody which catalyses said cleavage or said formation.

**20.** The use of a catalytic antibody produced by the method of claim 19 in the manufacture of a pharmaceutical for treating acquired immune deficiency syndrome or inhibiting human immunodeficiency virus.

**21.** The use of a catalytic antibody produced by the method of claim 19 in the manufacture of a pharmaceutical for treating hypertension by inhibiting human renin activity.

**Patentansprüche**

**1.** Immunogen, umfassend

(a) ein cyclisches Amid der Formel

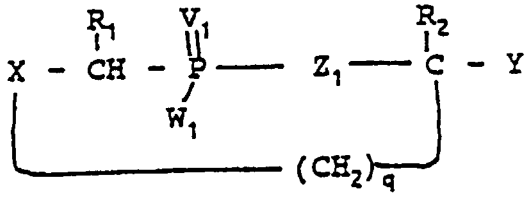

in der $R_1$ und $R_2$ gleich oder voneinander verschieden sein können und jeweils eine Seitenkette einer in der Natur vorkommenden Aminosäure, eine Hydroxy enthaltende Seitenkette einer in der Natur vorkommenden Aminosäure, in der die Hydroxygruppe glycosyliert, phosphoryliert, sulfonyliert oder durch eine Hydroxyschutzgruppe geschützt sein kann, eine primäres Amido enthaltende Seitenkette einer in der Natur vorkommenden Aminosäure, in der die Amidogruppe glycosyliert sein kann, oder $C_{1-4}$-Alkyl, -$CH_2CH(CO_2H)_2$, - $(CH_2)_2S(O)CH_3$, -$(CH_2)_2S(O)_2CH_3$, -$(CH_2)_3NH_2$ oder -$(CH_2)_3ONHC$-(=NH)$NH_2$ ist;
X Sauerstoff, Amino, durch eine Schutzgruppe geschütztes Amino, ausgewählt aus der Gruppe endständige Aminoschutzgruppen, zur Bildung einer Peptidbindung an den C-Terminus einer in der Natur vorkommenden Aminosäure gebundenes Amino, zur Bildung einer Peptidbindung an den C-Terminus eines Peptids gebundenes Amino, wobei die Aminosäure und das Peptid ungeschützt oder durch die Schutzgruppe geschützt sind, ist oder X Alken, ($C_1$-$C_9$)-Alkyl, ($C_1$-$C_9$)-Alkoxy, Phenyl, Phenoxy, Cyclohexyl, Phenylthio, Phenylsulfinyl oder Phenylsulfonyl ist, wobei die vorstehend erwähnten Phenylgruppen unsubstituiert oder durch Halogen, ($C_1$-$C_4$)-Alkyl, ($C_1$-$C_4$)-Alkoxy oder ($C_1$-$C_4$)-Alkoxycarbonyl mono-, di- oder trisubstituiert sein können;
Y Wasserstoff, Carboxyl, durch eine aus der Gruppe endständige Carboxylschutzgruppen ausgewählte Schutzgruppe geschütztes Carboxyl, einer zur Bildung einer Peptidbindung an den N-Terminus einer in der Natur vorkommenden Aminosäure, zur Bildung einer Peptidbindung an den N-Terminus eines Peptids gebundenem Carbonyl, wobei die Aminosäure und das Peptid durch die Schutzgruppe geschützt oder ungeschützt sind, oder Y ($C_1$-$C_9$)-Alkyl, ($C_1$-$C_9$)-Alkoxy, Phenyl, Phenoxy, Cyclohexyl, Phenylthio, Phenylsulfinyl oder Phenylsulfonyl ist, wobei die vorstehend erwähnten Phenylgruppen unsubstituiert oder durch Halogen, ($C_1$-$C_4$)-Alkyl, ($C_1$-$C_4$-Alkoxy oder ($C_1$-$C_4$)-Alkoxycarbonyl mono-, di- oder trisubstituiert sein können; und
wobei jedes $R_1$, $R_2$, X und Y ungebunden oder an eine oder mehrere von $R_1$, $R_2$, X und Y gebunden sein kann und wenn, dann durch eine kovalente Bindung oder eine Verknüpfungskomponente gebunden ist, ausgewählt aus der Gruppe -$(CH_2)_s$-S-S-$(CH_2)_t$-, -$(CH_2)_t$-, -S-$(CH_2)_t$-S-, -$(CH_2)_s$-S$(CH_2)_t$-, -$(CH_2)_s$-CH=CH-$(CH_2)_t$-, -$(CH_2)_s$-NH-CO-$(CH_2)_t$-, -$(CH_2)_s$-NH-$(CH_2)_t$- und -$(CH_2)_s$-Phenyl-$(CH_2)_t$-;
$W_1$ OH, $NH_2$, SH oder H ist;
$V_1$ O oder S ist;
$Z_1$ ungebunden O, NH, $CH_2$ oder S ist und gebunden N oder CH ist;
s und t gleich oder verschieden sein können und jeweils 0 oder eine ganze Zahl von 1 bis 10 sind, es sei denn, die Verknüpfungskomponente ist -$(CH_2)_t$-, in welchem Fall t eine ganze Zahl von 1 bis 10 ist;
q 0 oder eine ganze Zahl von 1 bis 10 ist und

(b) ein Trägermolekül, wobei das Amid durch eine geeignete Kupplungskomponente an das Trägermolekül gekuppelt ist.

**2.** Immunogen nach Anspruch 1, in dem $V_1$ O, $W_1$ OH, SH oder H, $Z_1$ O, NH oder $CH_2$ und q 0 oder 1 ist.

**3.** Immunogen, umfassend

(a) ein cyclisches Amid der Formel

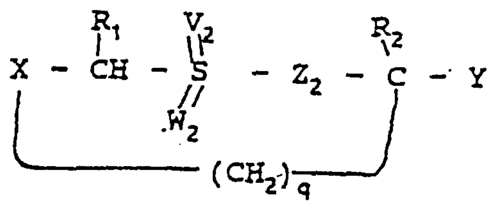

in der $R_1$, $R_2$, X, Y und q die gleiche Definition haben wie in Anspruch 1 und in der
$V_2$ O oder ein einsames Elektronenpaar ist;
$W_2$ O oder ein einsames Elektronenpaar ist;
$Z_2$ O, NH oder $CH_2$ ist, wenn es nicht an die Verknüpfungskomponente gebunden ist, und N oder CH ist, wenn es gebunden ist, und

(b) ein Trägermolekül, wobei das Amid durch eine geeignete Kupplungskomponente an das Trägermolekül gekuppelt ist.

**4.** Immunogen nach Anspruch 3, in dem $V_2$ und $W_2$ O oder ein einsames Elektronenpaar sind, $Z_2$ O, $CH_2$ oder NH ist und q 0 oder 1 ist.

**5.** Immunogen, umfassend

(a) ein cyclisches Amid der Formel

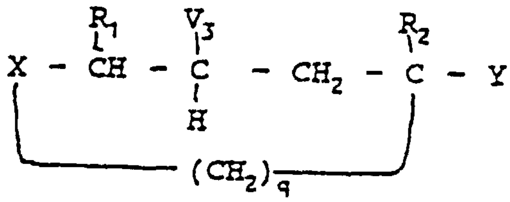

in der $R_1$, $R_2$, X, Y und q die gleiche Definition haben wie in Anspruch 1 und $V_3$ OH oder $NH_2$ ist; und

(b) ein Trägermolekül, wobei das Amid durch eine geeignete Kupplungskomponente an das Trägermolekül gekoppelt ist.

**6.** Immunogen nach Anspruch 5, in dem q 0 oder 1 ist.

**7.** Immunogen, umfassend

(a) ein cyclisches Amid der Formel

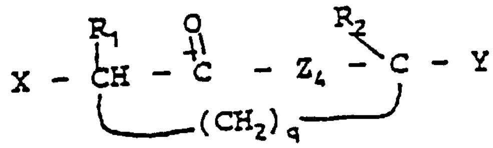

in der $R_1$, $R_2$, X, Y und q die gleiche Definition haben wie in Anspruch 1 und $Z_4$ ungebunden $CF_2$ oder $CF_2CO$ ist und gebunden CF oder CFCO ist; und

(b) ein Trägermolekül, wobei das Amid durch eine geeignete Kupplungskomponente an das Trägermolekül gekoppelt ist.

**8.** Immunogen nach Anspruch 7, in dem $Z_4$ $CF_2$ ist.

**9.** Immunogen nach Anspruch 8, in dem q 0 oder 1 ist.

**10.** Immunogen, umfassend

(a) ein cyclisches Amid der Formel

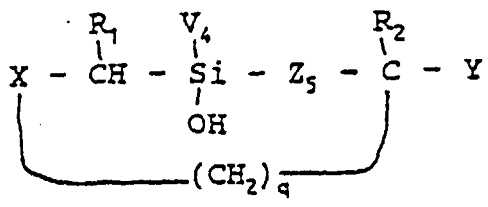

in der $R_1$, $R_2$, X, Y und q die gleiche Definition haben wie in Anspruch 1 und in der $V_4$ OH oder $NH_2$ ist; $Z_5$ ungebunden O oder $CH_2$ ist und gebunden CH ist;

(b) ein Trägermolekül, wobei das Amid durch eine geeignete Kupplungskomponente an das Trägermolekül gekuppelt ist.

**11.** Immunogen nach Anspruch 10, in dem $V_4$ OH ist.

**12.** Immunogen nach Anspruch 10, in dem $V_4$ OH, $Z_5$ $CH_2$ und q 0 oder 1 ist.

**13.** Immunogen nach einem der Ansprüche 1, 3, 5, 7 oder 11, in dem $R_1$ aus $CH_2OH$ und $CH(OH)CH_3$ ausgewählt ist; $R_2$ aus $CH(OH)CH_3$ und $CH_2CONH_2$ ausgewählt ist; X aus Ala-NH, Ala-Ser-NH, Ala-Ser-Thr-NH und Ala-Ser-Thr-Thr-NH ausgewählt ist und Y aus CO-Thr-Thr-Asn-Tyr-Cys, CO-Thr-Asn-Tyr-Cys, CO-Asn-Tyr-Cys und CO-Tyr-Cys ausgewählt ist.

**14.** Immunogen nach einem der Ansprüche 1, 3, 5, 7 und 11, in dem $R_1$ $CH(OH)CH_3$, $R_2$ H, X Cys-Leu-Arg-Tyr-Ser-NH und Y CO-Thr-Val-Cys ist.

**15.** Immunogen nach Anspruch 14, in dem das Hapten eine β-Turnkonfiguration hat, die die Konfiguration von nativem Protein nachahmt, wobei die Schwefelatome in den beiden endständigen Cysteinresten verbunden sind, um eine Disulfidbrücke zu bilden.

**16.** Verfahren zur Katalyse der Spaltung oder Bildung einer spezifischen Amidbindung innerhalb eines Moleküls, bei dem man das Molekül mit einer Menge eines monoklonalen Antikörpers in Kontakt bringt, die ausreicht, um die Spaltung oder Bildung dieser Amidbindung unter Bedingungen zu bewirken, die für eine solche Spaltung oder Bildung geeignet sind, wobei der monoklonale Antikörper durch ein Verfahren mit folgenden Schritten hergestellt wurde:

(a) Wahl der spezifischen Amidbindung, die gespalten oder gebildet werden soll;

(b) Wahl eines Immunogens nach einem der Ansprüche 1 bis 15, das ein Analogon der zu spaltenden oder zu bildenden Amidbindung umfasst und außerdem Komponenten umfasst, die dieses Analogon der Aminbindung umgeben, wobei die Komponenten im Wesentlichen einigen oder allen Komponenten entsprechen, die die zu spaltende oder zu bildende Amidbindung umgeben;

(c) Einwirkenlassen des Immunogens auf Zellen, die Antikörper produzieren können, wodurch Antikörper produzierende Zellen erzeugt werden;

(d) Hybridisieren der Antikörper erzeugenden Zellen mit Myelomzellen, wodurch eine Vielzahl von Hybridomzellen erzeugt wird, die jeweils monoklonale Antikörper erzeugen; und

(e) Screenen der Vielzahl der monoklonalen Antikörper, um einen monoklonalen Antikörper zu identifizieren, der die Spaltung oder Bildung der Amidbindung katalysiert.

**17.** Verfahren nach Anspruch 16 zur Katalyse der Spaltung oder Bildung einer spezifischen Amidbindung innerhalb einer im Molekül enthaltenen spezifischen Aminosäuresequenz.

**18.** Verfahren nach Anspruch 17, bei dem der monoklonale Antikörper durch ein Verfahren mit folgenden Schritten hergestellt wurde:

(a) Wahl der spezifischen Amidbindung, die gespalten oder gebildet werden soll;

(b) Wahl eines Immunogens, umfassend ein Dipeptidanalogon, das (i) eines oder mehrere hochenergetische Zwischenprodukte bzw. Übergangszustände in der Spaltung oder Bildung der Amidbindung nachahmt oder (ii) eine oder mehrere hochenergetische Konformationen der Amid- oder Esterbindung nachahmt oder (iii) sowohl eines oder mehrere hochenergetische Zwischenprodukte bzw. Übergangszustände bei der Spaltung oder Bildung der Amidbindung als auch eine oder mehrere Konformationen der Amidbindung mit hoher Energie nachahmt, wobei das Immunogen auch Komponenten umfasst, die das Dipeptidanalogon umgeben und im Wesentlichen einer oder allen Komponenten entsprechen, die die zu spaltende oder zu bildende Amidbindung umgeben;

(c) Einwirkenlassen des Immunogens auf Zellen, die Antikörper produzieren können, wodurch Antikörper produzierende Zellen erzeugt werden;

(d) Hybridisieren der Antikörper erzeugenden Zellen mit Myelomzellen, wodurch eine Vielzahl von Hybridomzellen erzeugt wird, die jeweils monoklonale Antikörper erzeugen; und

(e) Screenen der Vielzahl der monoklonalen Antikörper, um einen monoklonalen Antikörper zu identifizieren, der die Spaltung oder Bildung der Amidbindung katalysiert.

**19.** Verfahren zur Herstellung katalytischer Antikörper, die die Spaltung oder Bildung einer Amidbindung katalysieren können, bei dem man

(a) Zellen, die Antikörper produzieren können, der Einwirkung eines Immunogens nach einem der Ansprüche 1 bis 15 aussetzt und dadurch Antikörper erzeugende Zellen herstellt;

(b) die Antikörper erzeugenden Zellen mit Myelomzellen hybridisert und dadurch eine Vielzahl von Hybridomzellen erzeugt, die jeweils monoklonale Antikörper herstellen, und

(c) die Vielzahl der monoklonalen Antikörper screent, um einen monoklonalen Antikörper zu identifizieren, der die Spaltung oder die Bildung katalysiert.

**20.** Verwendung eines durch das Verfahren von Anspruch 19 hergestellten katalytischen Antikörpers bei der Herstellung einer pharmazeutischen Substanz zur Behandlung des erworbenen Immunschwächesyndroms (AIDS) oder zur Hemmung des Humanimmunschwächevirus (HIV).

**21.** Verwendung eines durch das Verfahren von Anspruch 19 hergestellten katalytischen Antikörpers zur Herstellung einer pharmazeutischen Substanz zur Behandlung von Bluthochdruck durch Hemmung der Reninaktivität beim Menschen.

**Revendications**

**1.** Immunogène comprenant:

(a) un amide cyclique de formule:

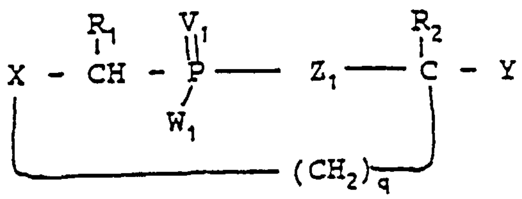

dans laquelle $R_1$ et $R_2$ peuvent être identiques ou différents et représentent chacun une chaîne latérale d'un aminoacide naturel; une chaîne latérale d'un aminoacide naturel qui contient un groupe hydroxy et dans laquelle ledit groupe hydroxy peut être glycosylé, phosphorylé, sulfonylé ou protégé par un groupe protecteur d'hydroxy; une chaîne latérale d'un aminoacide naturel qui contient un groupe amido primaire et dans laquelle ledit groupe amido peut être glycosylé; ou un groupe alkyle en $C_1$-$C_4$, $-CH_2CH(CO_2H)_2$, $-(CH_2)_2S(O)CH_3$, $-(CH_2)_2S(O)_2CH_3$, $-(CH_2)_3NH_2$ ou $-(CH_2)_3ONHC(=NH)NH_2$;

X est un atome d'oxygène, un groupe amino, un groupe amino protégé par un groupe protecteur choisi dans le groupe constitué par les groupes protecteurs d'amino terminal, un groupe amino lié à l'extrémité C-terminale d'un aminoacide naturel pour former une liaison peptidique, un groupe amino lié à l'extrémité C-terminale d'un peptide pour former une liaison peptidique, ledit aminoacide et ledit peptide étant non protégés ou protégés par ledit groupe protecteur, ou X est un groupe alcène, alkyle en $C_1$-$C_9$, alcoxy en $C_1$-$C_9$, phényle, phénoxy, cyclohexyle, phénylthio, phénylsulfinyle ou phénylsulfonyle, les groupes phényle précités pouvant être non substitués ou mono-, di- ou trisubstitués par halogène, alkyle en $C_1$-$C_4$, alcoxy en $C_1$-$C_4$ ou (alcoxy en $C_1$-$C_4$)carbonyle;

Y est un atome d'hydrogène ou un groupe carboxyle, un groupe carboxyle protégé par un groupe protecteur choisi dans le groupe constitué par les groupes protecteurs de carboxyles terminaux, un groupe carbonyle lié à l'extrémité N-terminale d'un aminoacide naturel pour former une liaison peptidique, un groupe carbonyle lié à l'extrémité N-terminale d'un peptide pour former une liaison peptidique, ledit aminoacide et ledit peptide étant non protégés ou protégés par ledit groupe protecteur, ou Y est un groupe alkyle en $C_1$-$C_9$, alcoxy en $C_1$-$C_9$, phényle, phénoxy, cyclohexyle, phénylthio, phénylsulfinyle ou phénylsulfonyle, les groupes phényle précités pouvant être non substitués ou mono-, di- ou trisubstitués par halogène, alkyle en $C_1$-$C_4$, alcoxy en $C_1$-$C_4$ ou (alcoxy en $C_1$-$C_4$)carbonyle; et

chacun des $R_1$, $R_2$, X et Y peut être non lié ou lié à un ou plusieurs $R_1$, $R_2$, X et Y, et, lorsqu'il est lié, il est lié par une liaison covalente ou par un élément de liaison choisi dans le groupe constitué par $-(CH_2)_s$-S-S-$(CH_2)_t$-, $-(CH_2)_t$-, -S-$(CH_2)_t$-S-, $-(CH_2)_s$-S-$(CH_2)_t$-, $-(CH_2)_s$-CH=CH-$(CH_2)_t$-, $-(CH_2)_s$-NH-CO-$(CH_2)_t$-, $-(CH_2)_s$-NH-$(CH_2)_t$- et $-(CH_2)_s$-phényl-$(CH_2)_t$-;

$W_1$ est OH, $NH_2$, SH ou H;

$V_1$ est O ou S;

$Z_1$ est O, NH, $CH_2$ ou S lorsqu'il n'est pas lié et N ou CH lorsqu'il est lié;

s et t peuvent être identiques ou différents et sont chacun 0 ou un entier de 1 à 10, sauf si l'élément de liaison est $-(CH_2)_t$-, auquel cas t est un entier de 1 à 10;

q est 0 ou un entier de 1 à 10; et

(b) une molécule support, ledit amide étant couplé à ladite molécule support par un élément de couplage approprié.

**2.** Immunogène selon la revendication 1, dans lequel $V_1$ est O, $W_1$ est OH, SH ou H, $Z_1$ est O, NH ou $CH_2$ et q est 0 ou 1.

**3.** Immunogène comprenant:

(a) un amide cyclique de formule:

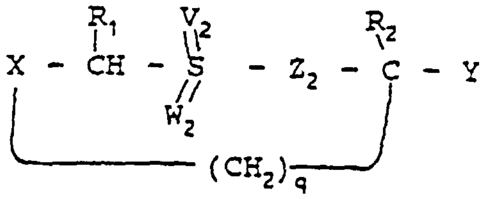

dans laquelle $R_1$, $R_2$, X, Y et q ont la définition indiquée dans la revendication 1, et
$V_2$ est O ou une paire isolée d'électrons;
$W_2$ est O ou une paire isolée d'électrons;
$Z_2$ est O, NH ou $CH_2$ lorsqu'il n'est pas lié audit élément de liaison, et N ou CH lorsqu'il est lié; et

(b) une molécule support, ledit amide étant couplé à ladite molécule support par un élément de couplage approprié.

**4.** Immunogène selon la revendication 3, dans lequel $V_2$ et $W_2$ sont O ou une paire isolée d'électrons, $Z_2$ est O, $CH_2$ ou NH et q est 0 ou 1.

**5.** Immunogène comprenant

(a) un amide cyclique de formule:

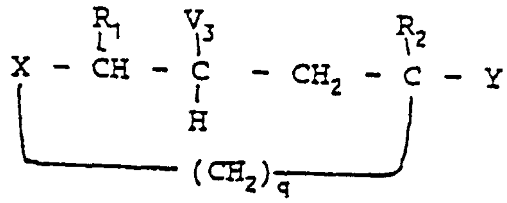

dans laquelle $R_1$, $R_2$, X, Y et q ont la définition indiquée dans la revendication 1 et
$V_3$ est OH ou $NH_2$; et

(b) une molécule support, ledit amide étant couplé à ladite molécule support par un élément de couplage approprié.

**6.** Immunogène selon la revendication 5, dans lequel q est 0 ou 1.

**7.** Immunogène comprenant

(a) un amide cyclique de formule:

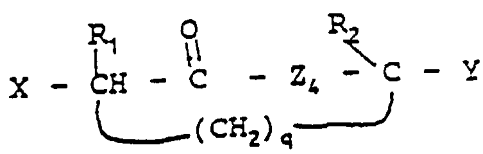

dans laquelle $R_1$, $R_2$, X, Y et q ont la définition indiquée dans la revendication 1 et $Z_4$ est $CF_2$ ou $CF_2CO$ lorsqu'il n'est pas lié, et CF ou CFCO lorsqu'il est lié; et

(b) une molécule support, ledit amide étant couplé à ladite molécule support par un élément de couplage approprié.

**8.** Immunogène selon la revendication 7, dans lequel $Z_4$ est $CF_2$.

**9.** Immunogène selon la revendication 8, dans lequel q est 0 ou 1.

**10.** Immunogène comprenant

(a) un amide cyclique de formule:

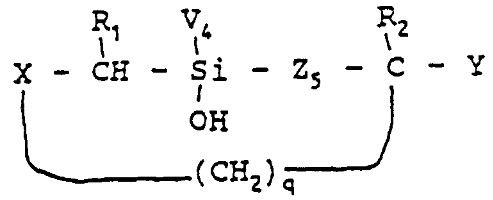

dans laquelle $R_1$, $R_2$, X, Y et q ont la définition indiquée dans la revendication 1 et
$V_4$ est OH ou $NH_2$;
$Z_5$ est O ou $CH_2$ lorsqu'il n'est pas lié, et CH lorsqu'il est lié; et

(b) une molécule support, ledit amide étant couplé à ladite molécule support par un élément de couplage approprié.

**11.** Immunogène selon la revendication 10, dans lequel $V_4$ est OH.

**12.** Immunogène selon la revendication 10, dans lequel $V_4$ est OH, $Z_5$ est $CH_2$ et q est 0 ou 1.

**13.** Immunogène selon l'une quelconque des revendications 1, 3, 5, 7 ou 11, dans lequel $R_1$ est choisi parmi $CH_2OH$ et $CH(OH)CH_3$; $R_2$ est choisi parmi $CH(OH)CH_3$ et $CH_2CONH_2$; X est choisi parmi Ala-NH, Ala-Ser-NH, Ala-Ser-Thr-NH et Ala-Ser-Thr-Thr-NH; et Y est choisi parmi CO-Thr-Thr-Asn-Tyr-Cys, CO-Thr-Asn-Tyr-Cys, CO-Asn-Tyr-Cys et CO-Tyr-Cys.

**14.** Immunogène selon l'une quelconque des revendications 1, 3, 5, 7 et 11, dans lequel $R_1$ est $CH(OH)CH_3$; $R_2$ est H; X est Cys-Leu-Arg-Tyr-Ser-NH et Y est CO-Thr-Val-Cys.

**15.** Immunogène selon la revendication 14, dans lequel ledit haptène a une configuration de spire β simulant la configuration de la protéine naturelle dans laquelle les atomes de soufre des deux résidus de cystéine terminaux sont liés pour former un pont disulfure.

**16.** Procédé de catalyse de la coupure ou de la formation d'une liaison amide spécifique dans une molécule, qui comprend la mise en contact de ladite molécule avec une quantité d'un anticorps monoclonal efficace pour catalyser la coupure ou la formation de ladite liaison amide dans des conditions appropriées pour que se produise ladite coupure ou formation d'une liaison, ledit anticorps monoclonal ayant été préparé par un procédé comprenant les étapes selon lesquelles:

(a) on sélectionne la liaison amide spécifique à couper ou à former;
(b) on sélectionne un immunogène selon l'une quelconque des revendications 1 à 15 et comprenant un analogue de ladite liaison amide à couper ou à former, et comprenant aussi des éléments entourant ledit analogue de ladite liaison amide, lesdits éléments correspondant essentiellement à certains éléments ou à tous les éléments entourant la liaison amide à couper ou à former;

(c) on expose des cellules capables de produire des anticorps audit immunogène, ce qui entraîne la formation de cellules productrices d'anticorps;

(d) on hybride lesdites cellules productrices d'anticorps avec des cellules de myélome, ce qui entraîne la formation d'une pluralité de cellules d'hybridomes produisant chacune des anticorps monoclonaux; et

(e) on crible cette pluralité d'anticorps monoclonaux pour identifier un anticorps monoclonal qui catalyse la coupure ou la formation de ladite liaison amide.

**17.** Procédé selon la revendication 16 pour la catalyse de la coupure ou de la formation d'une liaison amide spécifique dans une séquence d'aminoacides spécifique contenue dans la molécule.

**18.** Procédé selon la revendication 17, ledit anticorps monoclonal ayant été préparé par un procédé comprenant les étapes selon lesquelles:

(a) on sélectionne la liaison amide spécifique à couper ou à former;

(b) on sélectionne un immunogène comprenant un analogue de dipeptide qui (i) simule un ou plusieurs intermédiaires ou états de transition de haute énergie dans la coupure ou la formation de ladite liaison amide ou (ii) simule une ou plusieurs conformations de haute énergie de ladite liaison amide ou ester ou (iii) simule à la fois des intermédiaires ou des états de transition de haute énergie dans la coupure ou la formation de ladite liaison amide et une ou plusieurs conformations de haute énergie de ladite liaison amide, et ledit immunogène comprenant aussi des éléments entourant ledit analogue de dipeptide qui correspondent essentiellement à certains éléments ou à tous les éléments entourant la liaison amide à couper ou à former;

(c) on expose des cellules capables de produire des anticorps audit immunogène, ce qui entraîne la formation de cellules productrices d'anticorps;

(d) on hybride lesdites cellules productrices d'anticorps avec des cellules de myélome, ce qui entraîne la formation d'une pluralité de cellules d'hybridomes produisant chacune des anticorps monoclonaux; et

(e) on crible cette pluralité d'anticorps monoclonaux pour identifier un anticorps monoclonal qui catalyse la coupure ou la formation de ladite liaison amide.

**19.** Procédé de production d'anticorps catalytiques capables de catalyser la coupure ou la formation d'une liaison amide, comprenant les étapes selon lesquelles:

(a) on expose des cellules capables de produire des anticorps à un immunogène selon l'une quelconque des revendications 1 à 15, ce qui entraîne la formation de cellules productrices d'anticorps;

(b) on hybride lesdites cellules productrices d'anticorps avec des cellules de myélome, ce qui entraîne la formation d'une pluralité de cellules d'hybridomes produisant chacune des anticorps monoclonaux; et

(c) on crible cette pluralité d'anticorps monoclonaux pour identifier un anticorps monoclonal qui catalyse ladite coupure ou ladite formation.

**20.** Utilisation d'un anticorps catalytique produit par le procédé de la revendication 19 dans la préparation d'un médicament pour le traitement du sida ou l'inhibition du virus de l'immunodéficience humaine.

**21.** Utilisation d'un anticorps catalytique produit par le procédé de la revendication 19 dans la préparation d'un médicament pour le traitement de l'hypertension par l'inhibition de l'activité de la rénine humaine.

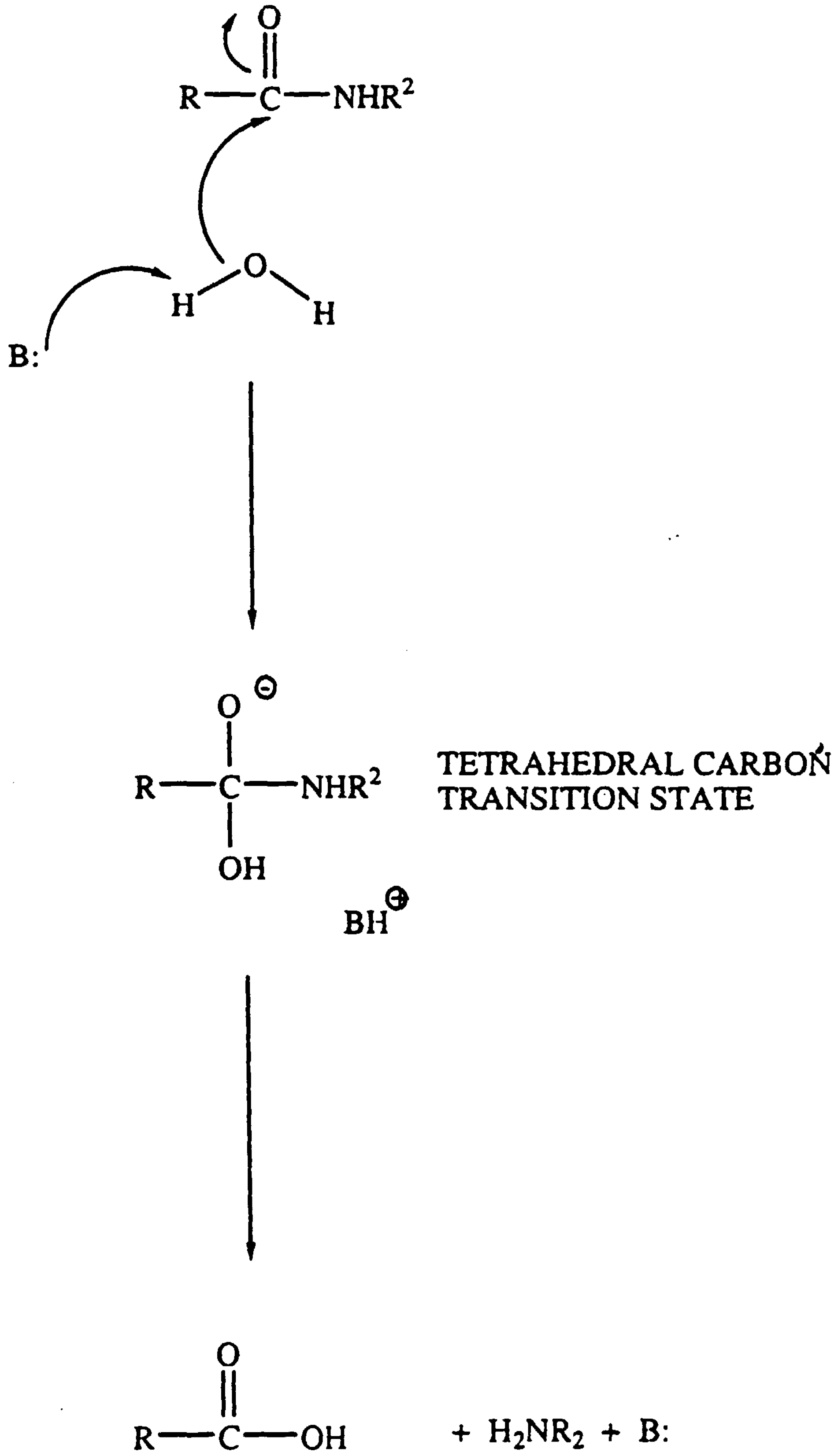


Fig. 1

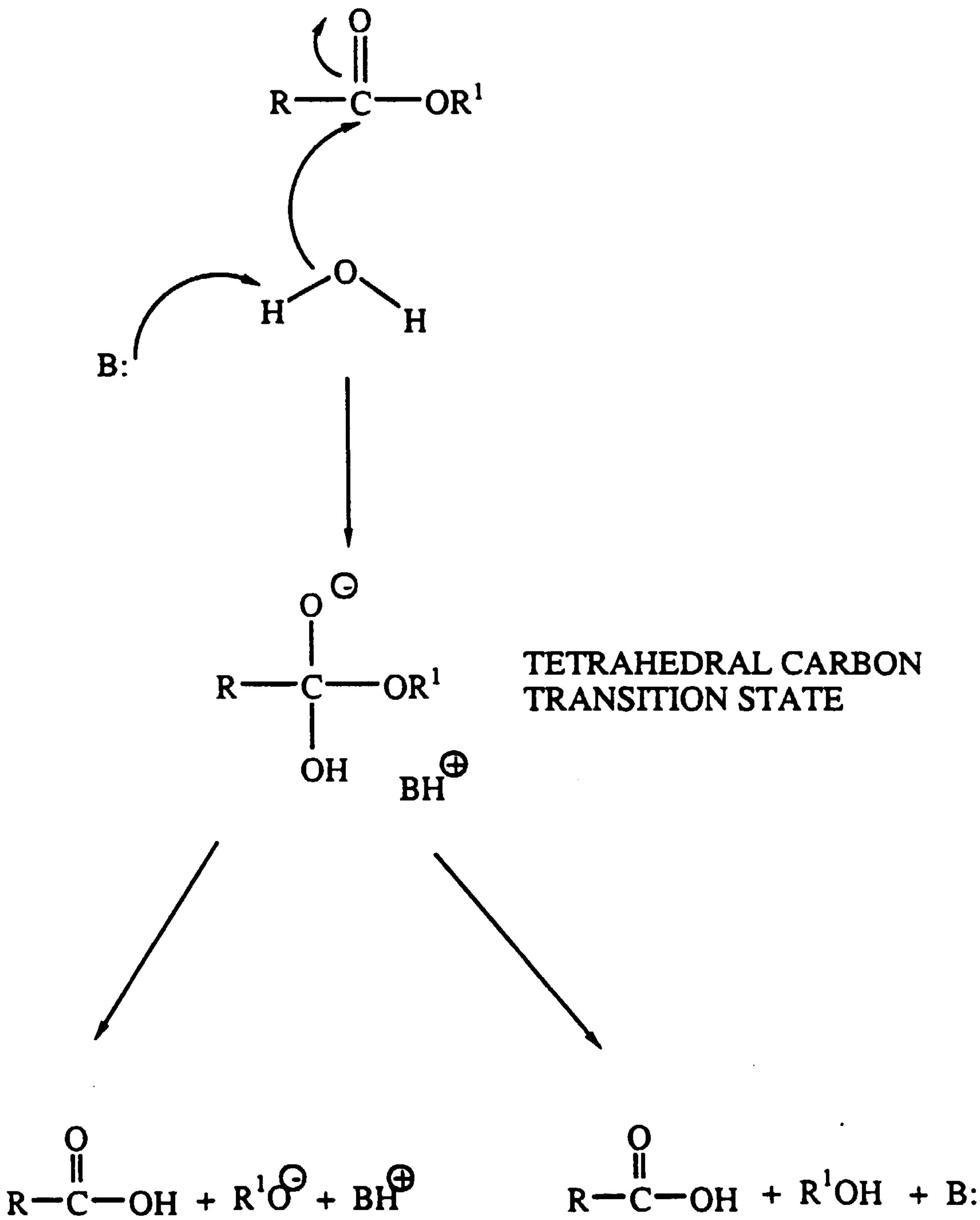


Fig. 2

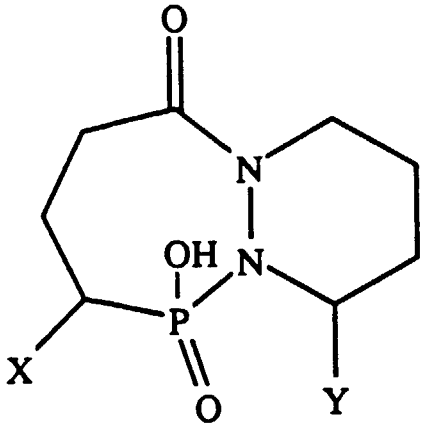

(A)

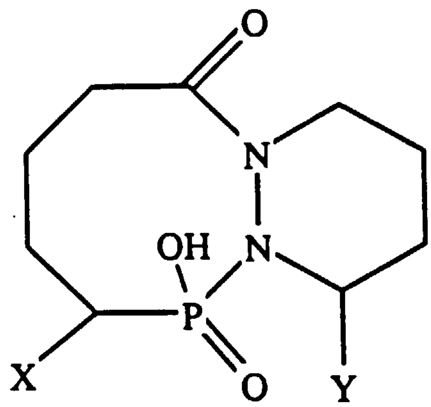

(B)

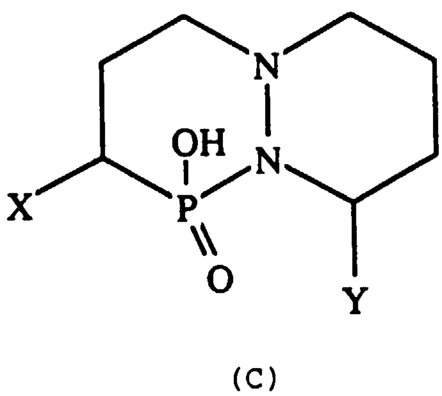

(C)

Fig. 3

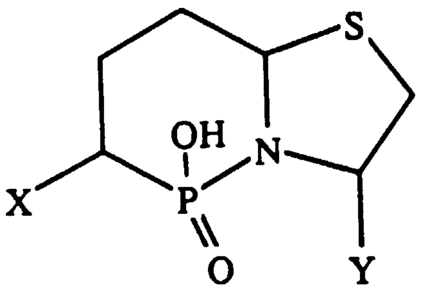

(A)

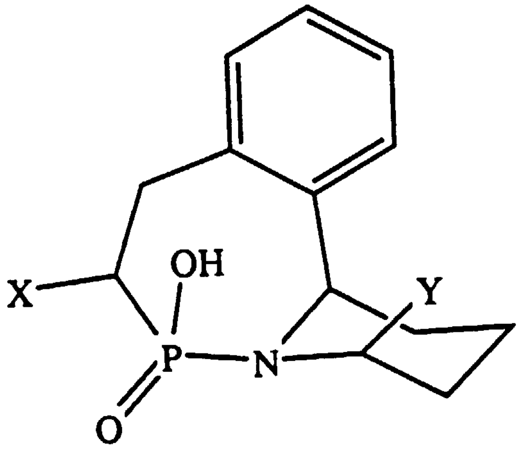

(B)

Fig. 4

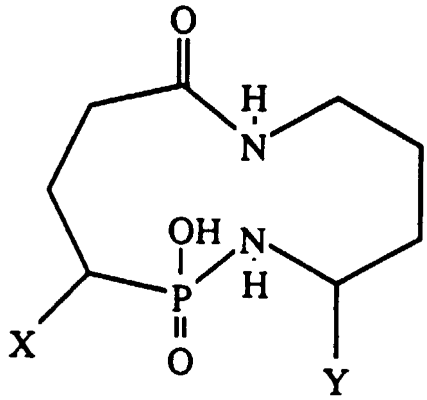

Fig. 5

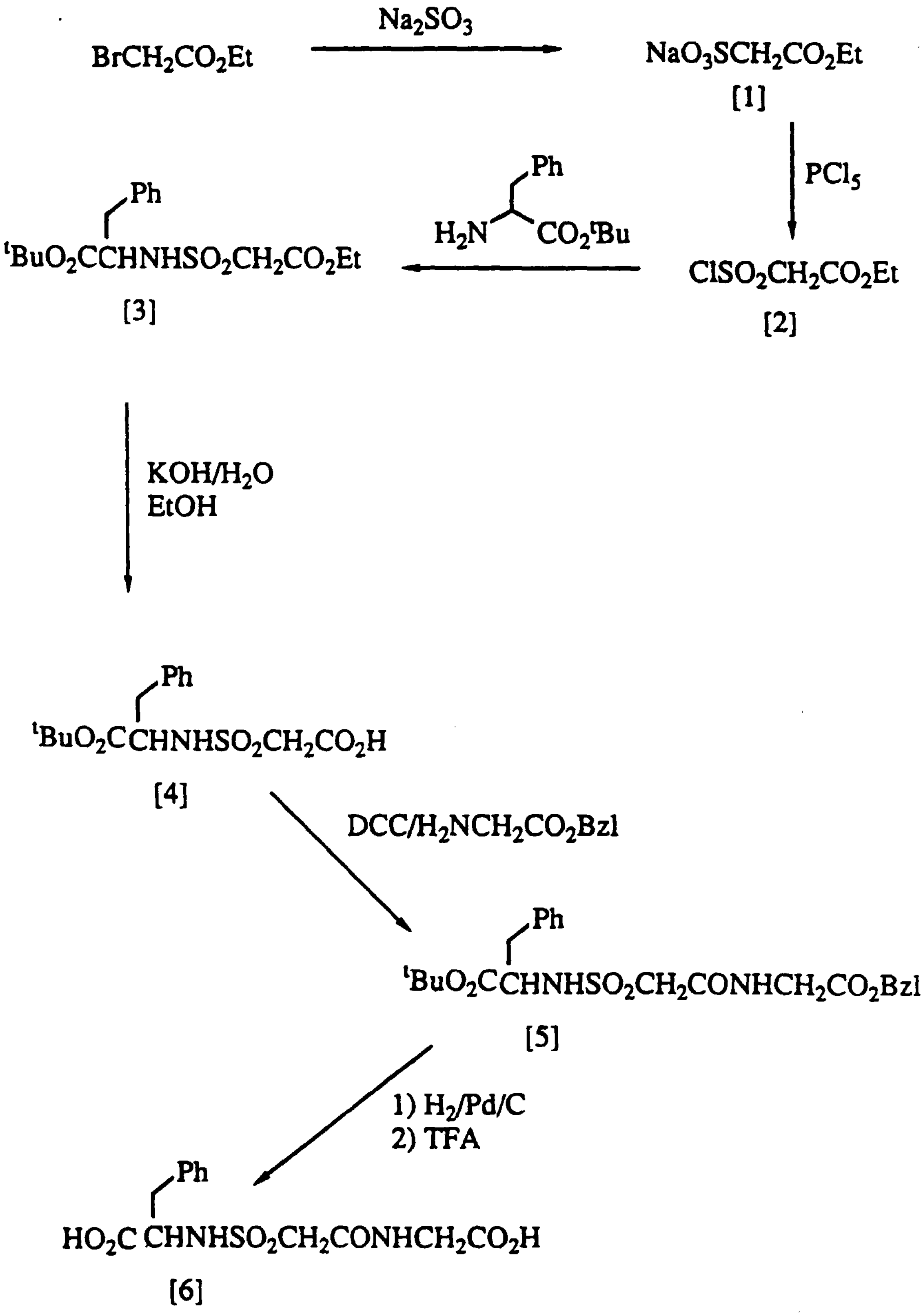
$Na_2SO_3$
[1]
$PCl_5$
[2]
[3]
KOH/$H_2O$
EtOH
[4]
DCC/$H_2NCH_2CO_2Bzl$
[5]
1) $H_2$/Pd/C
2) TFA
[6]

Fig. 6

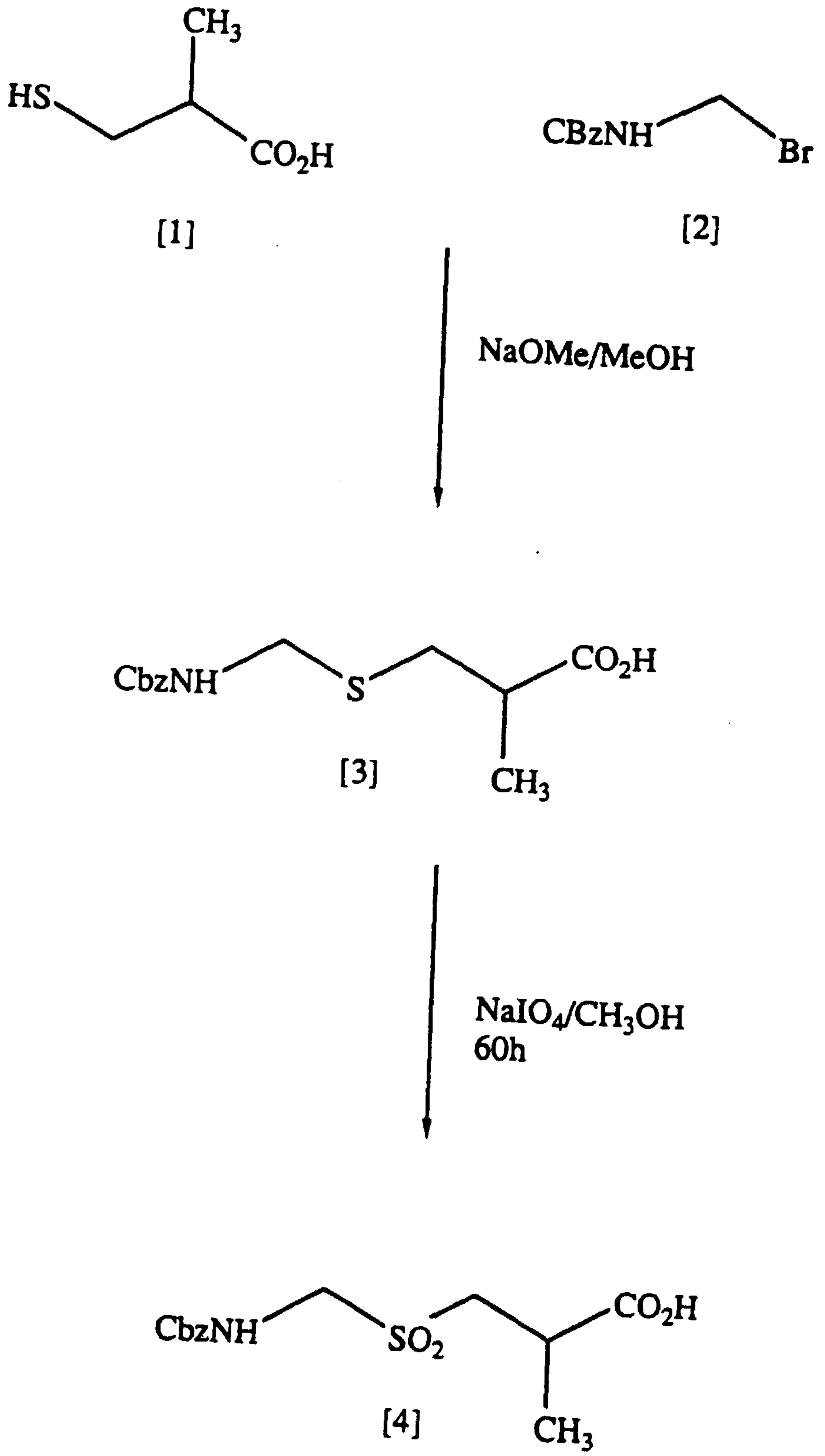


[1] [2] [3] [4]

Fig. 7

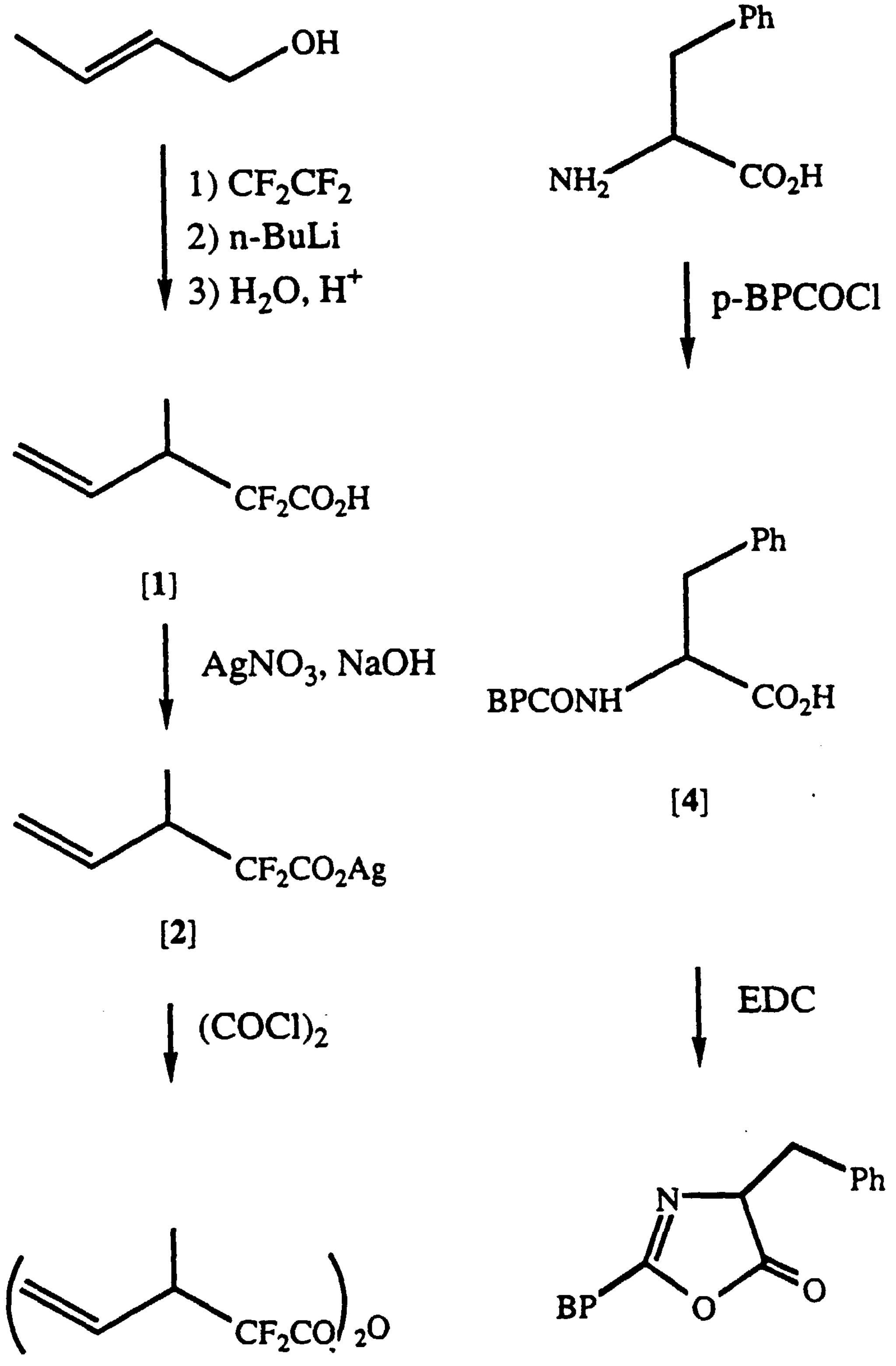


[3] [5]

Fig. 8

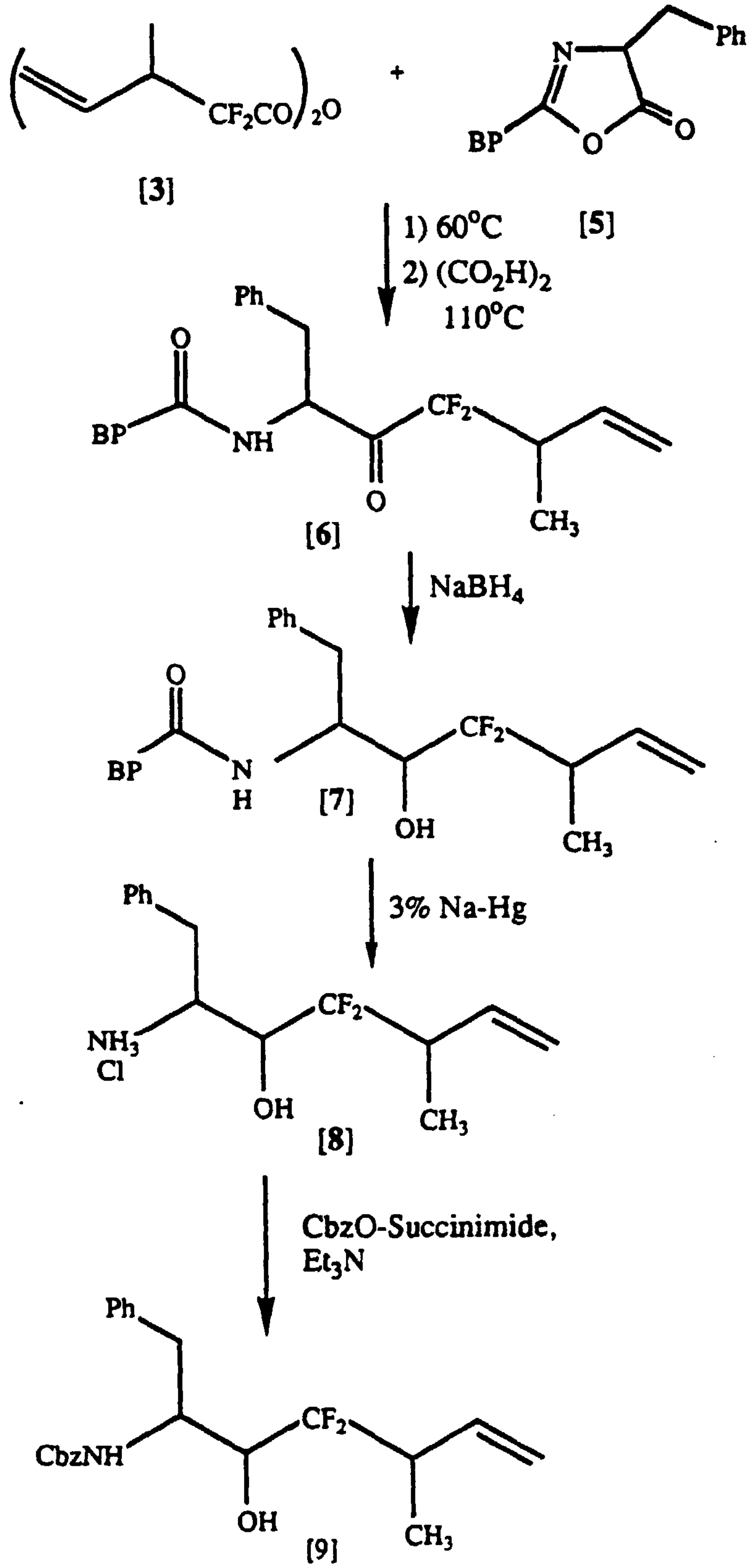


[3] [5] [6] [7] [8] [9]

Fig. 8 (cont'd)

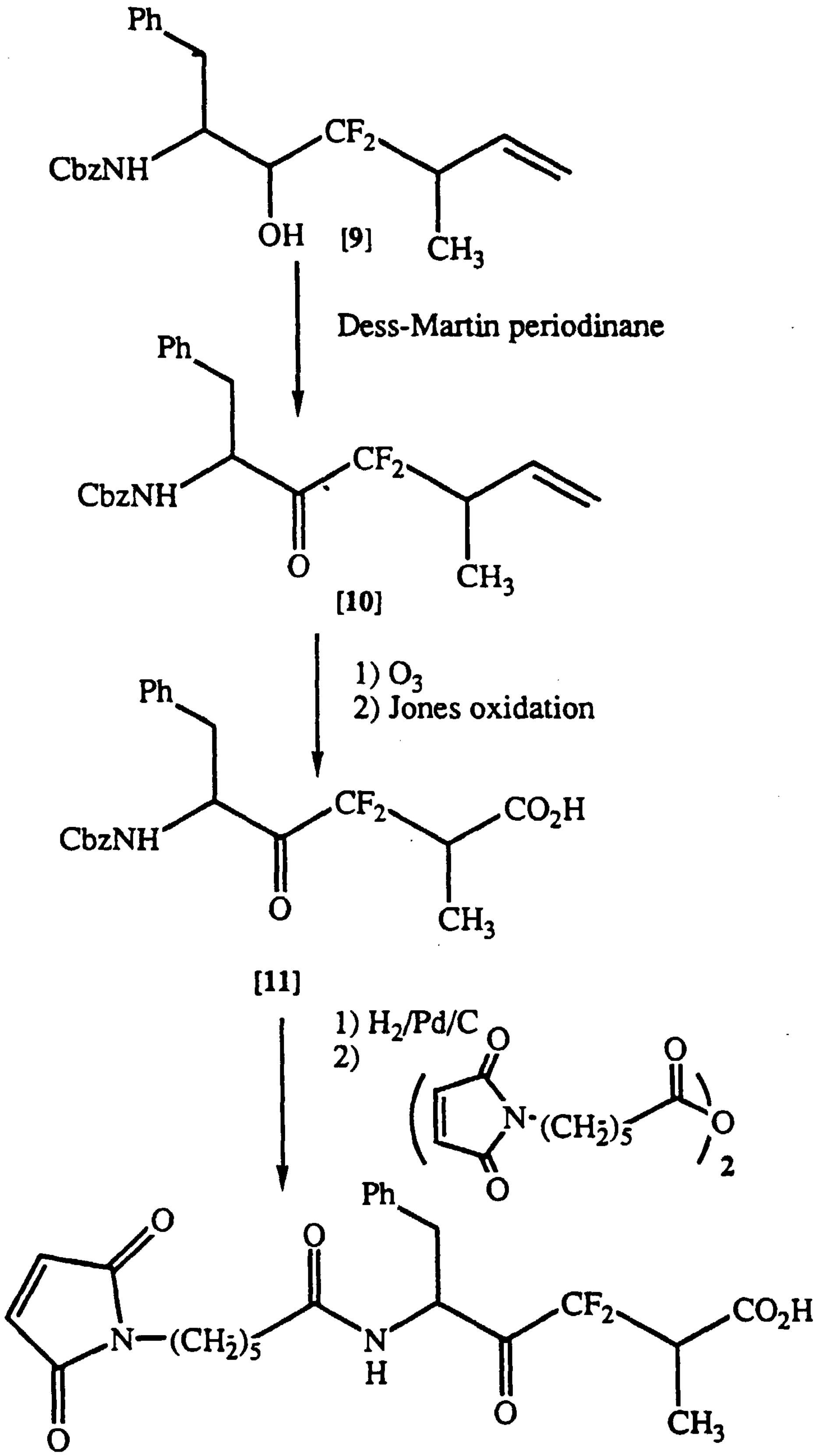


[9]

[10]

[11]

[12]

Fig. 8 (cont'd)

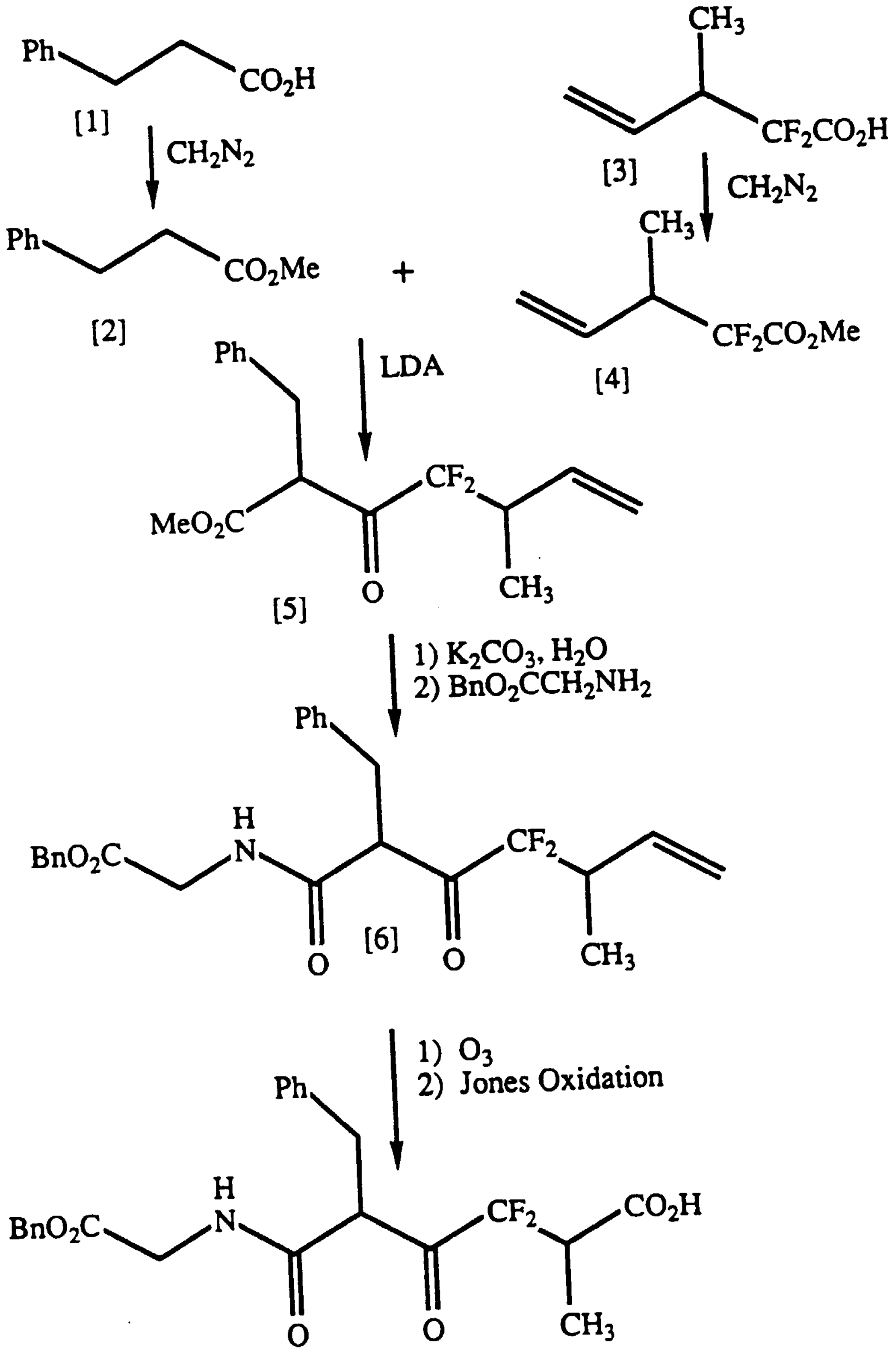
[1]
$CH_2N_2$
[3]
$CH_2N_2$
[2]
+
[4]
LDA
[5]
1) $K_2CO_3$, $H_2O$
2) $BnO_2CCH_2NH_2$
[6]
1) $O_3$
2) Jones Oxidation

[7]

Fig. 9

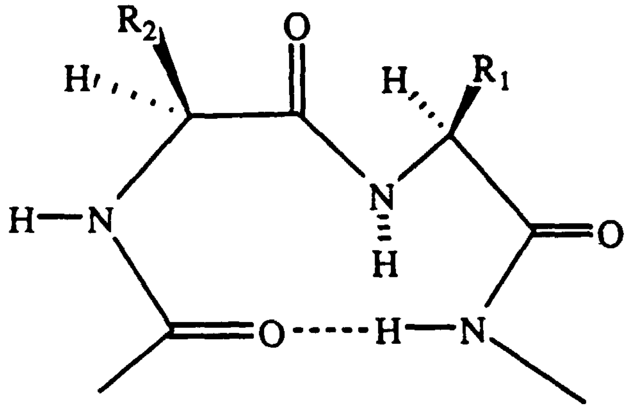

Fig. 10

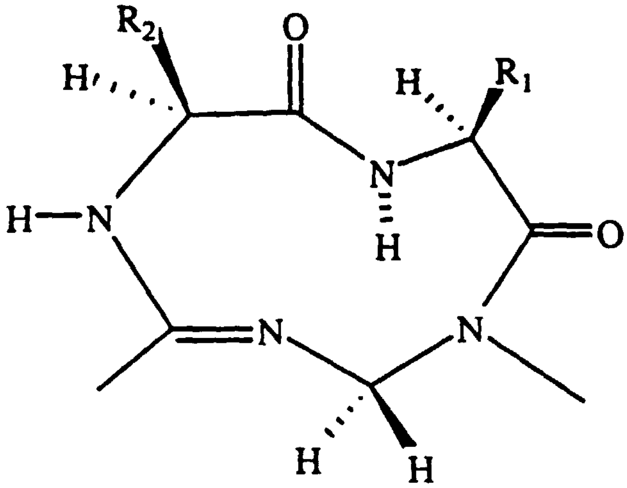

Fig. 11

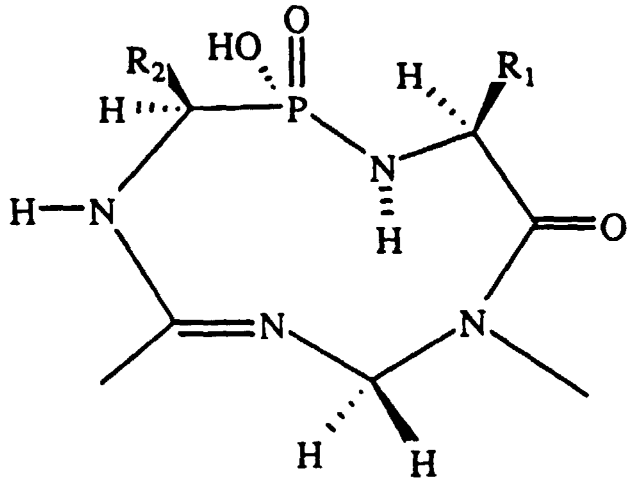

Fig. 12

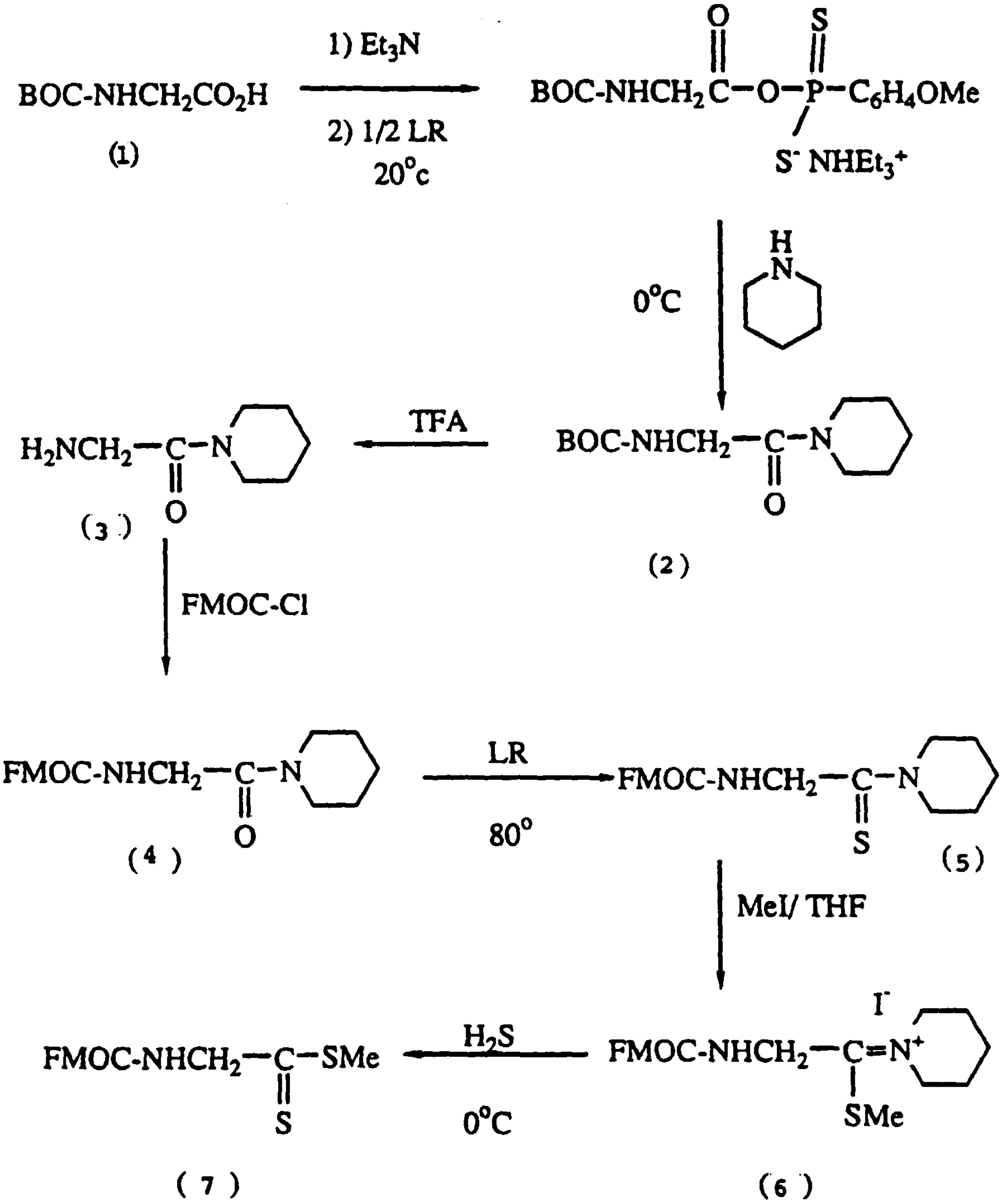


(1) (2) (3) (4) (5) (6) (7)

LR=
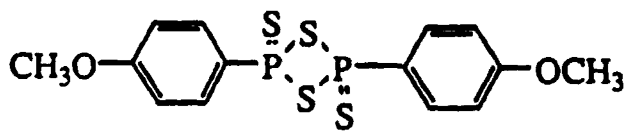

(Lawesson's reagent)

Fig. 13

Scheme 1

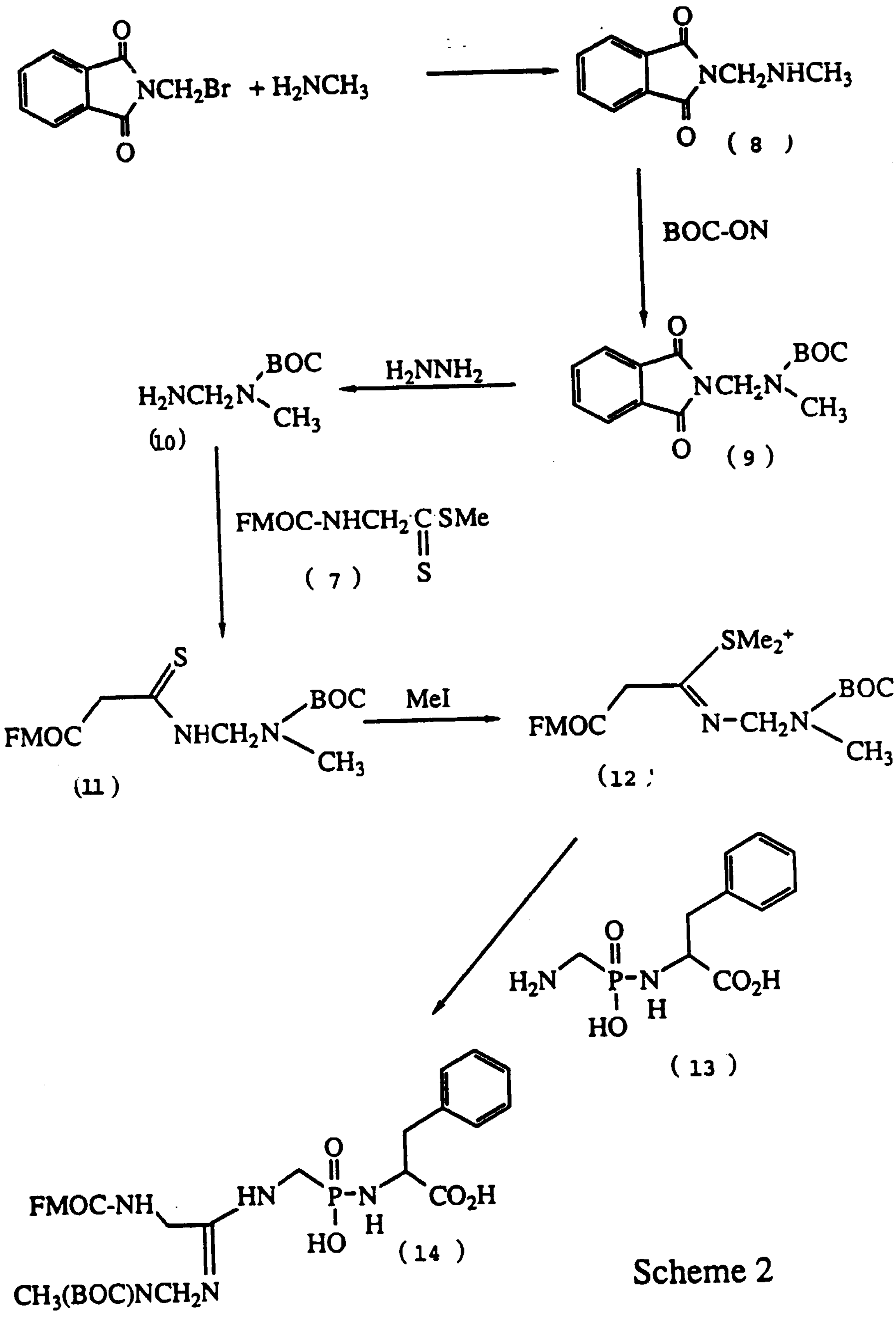


Scheme 2

Fig. 14

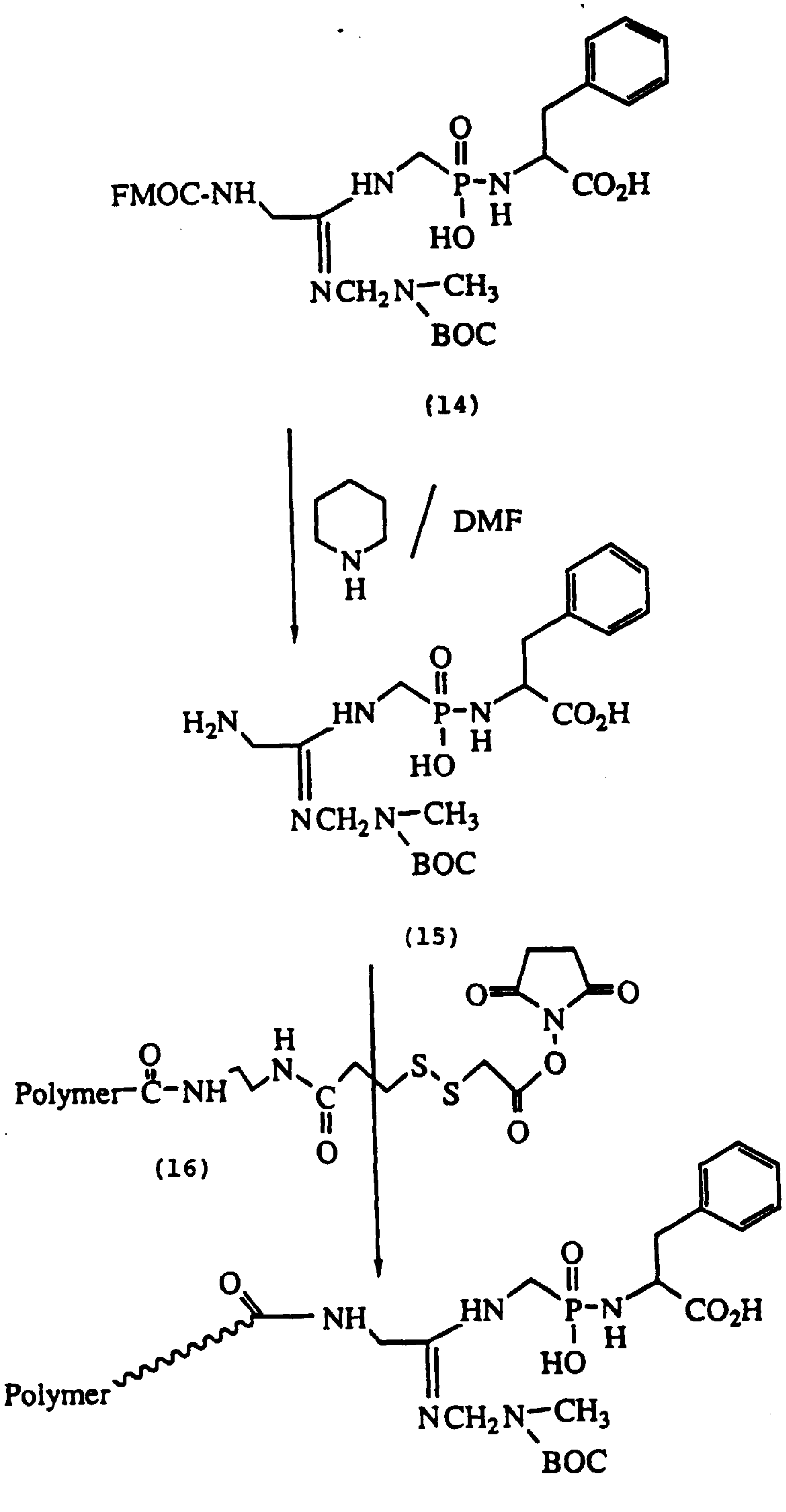


(14)

(15)

(16)

(17)

Fig. 14 (cont'd)

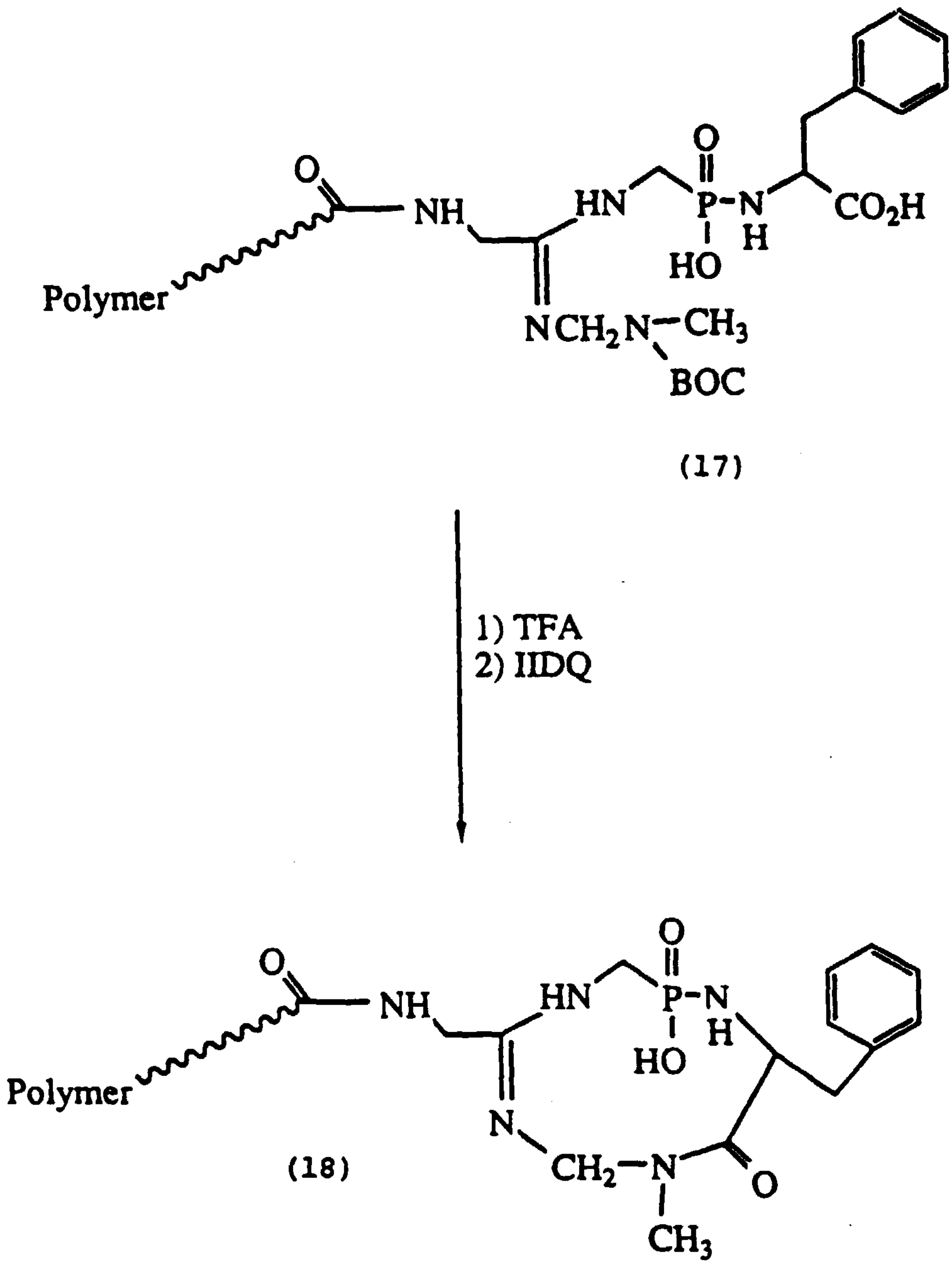


(17)

(18)

Scheme 2 (continued)

Fig. 14 (cont'd)

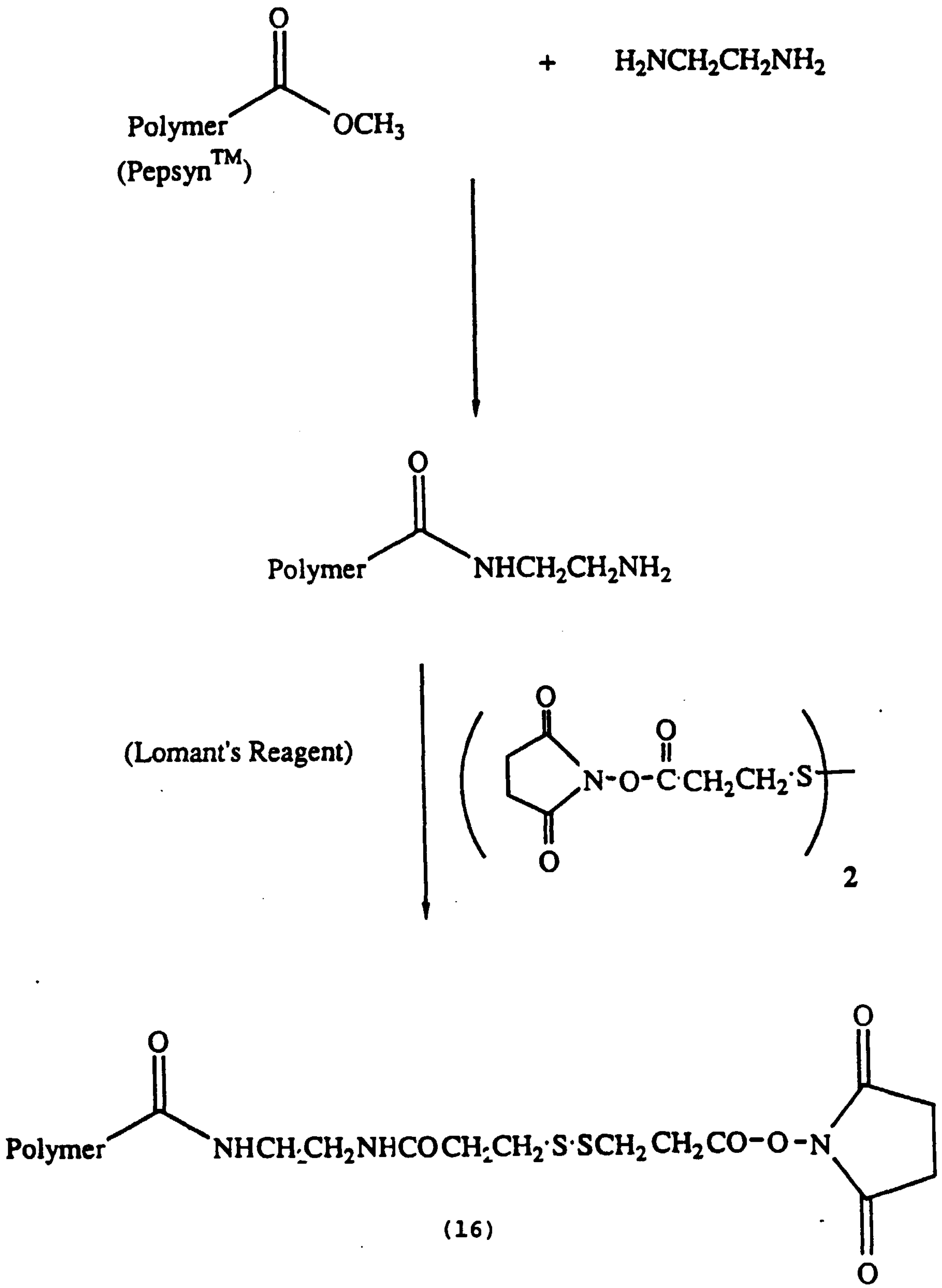


(16)

Scheme 3

Fig. 15

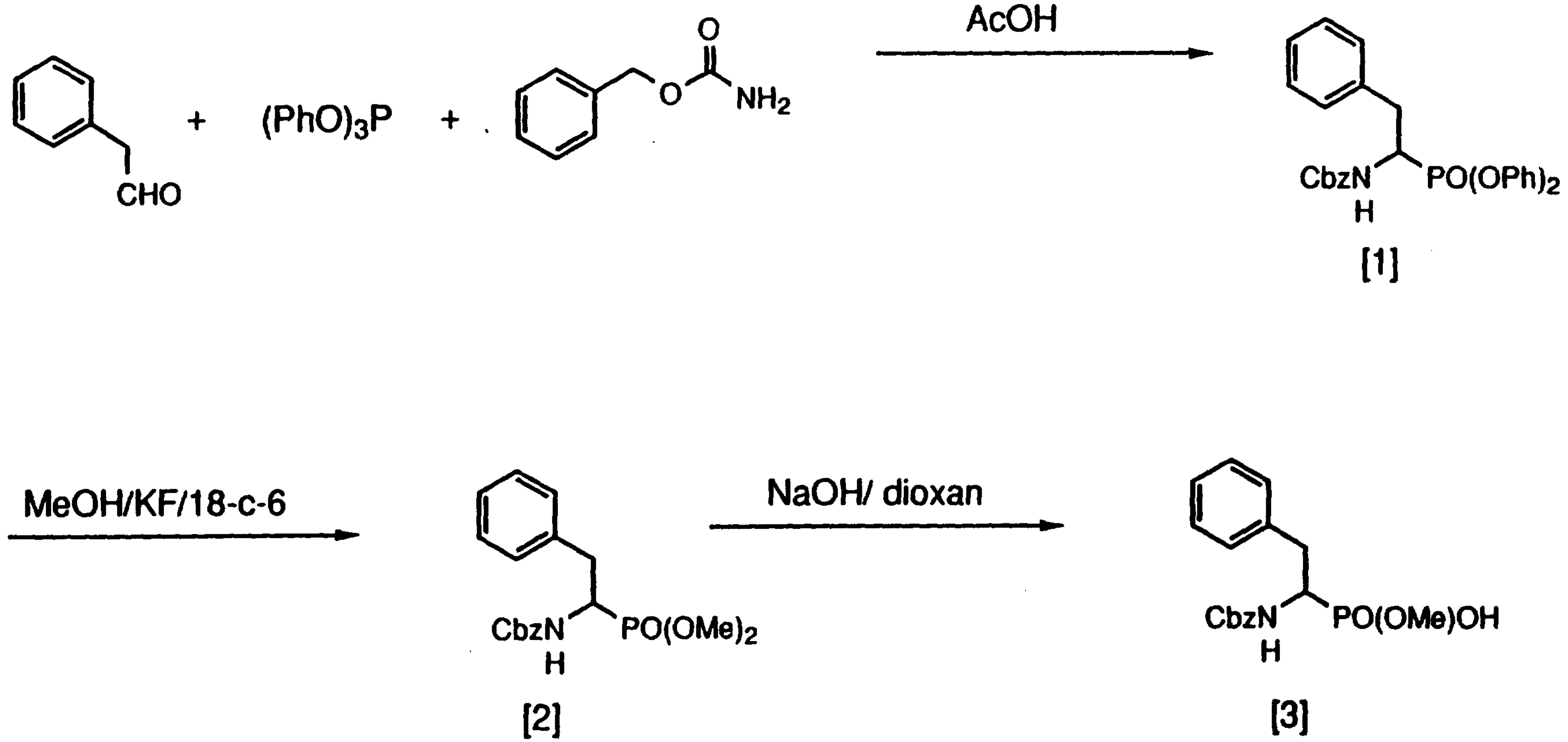


[1]

[2]

[3]

Fig 16

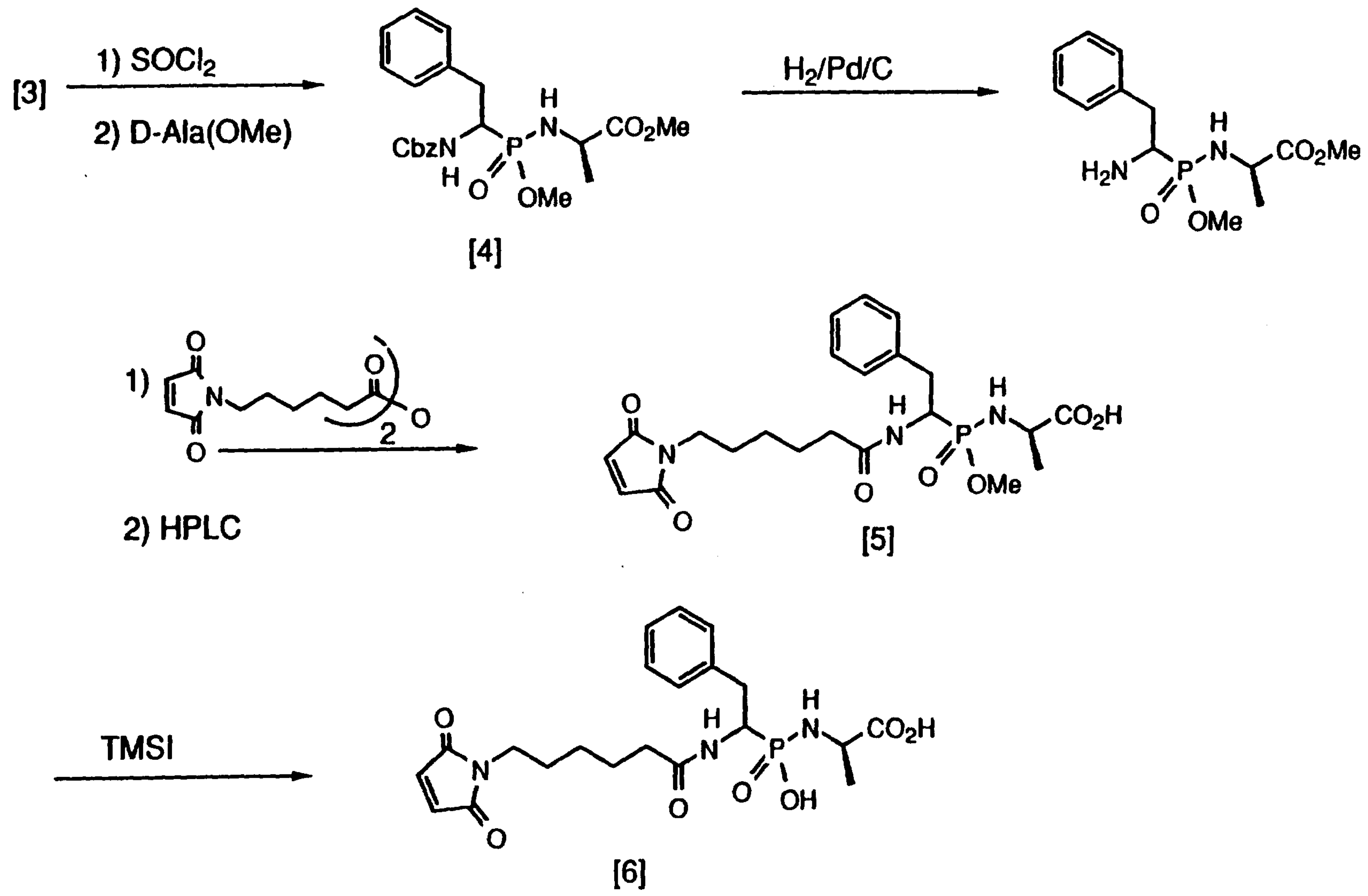


[3]

[4]

[5]

[6]

Fig 16 (cont'd)

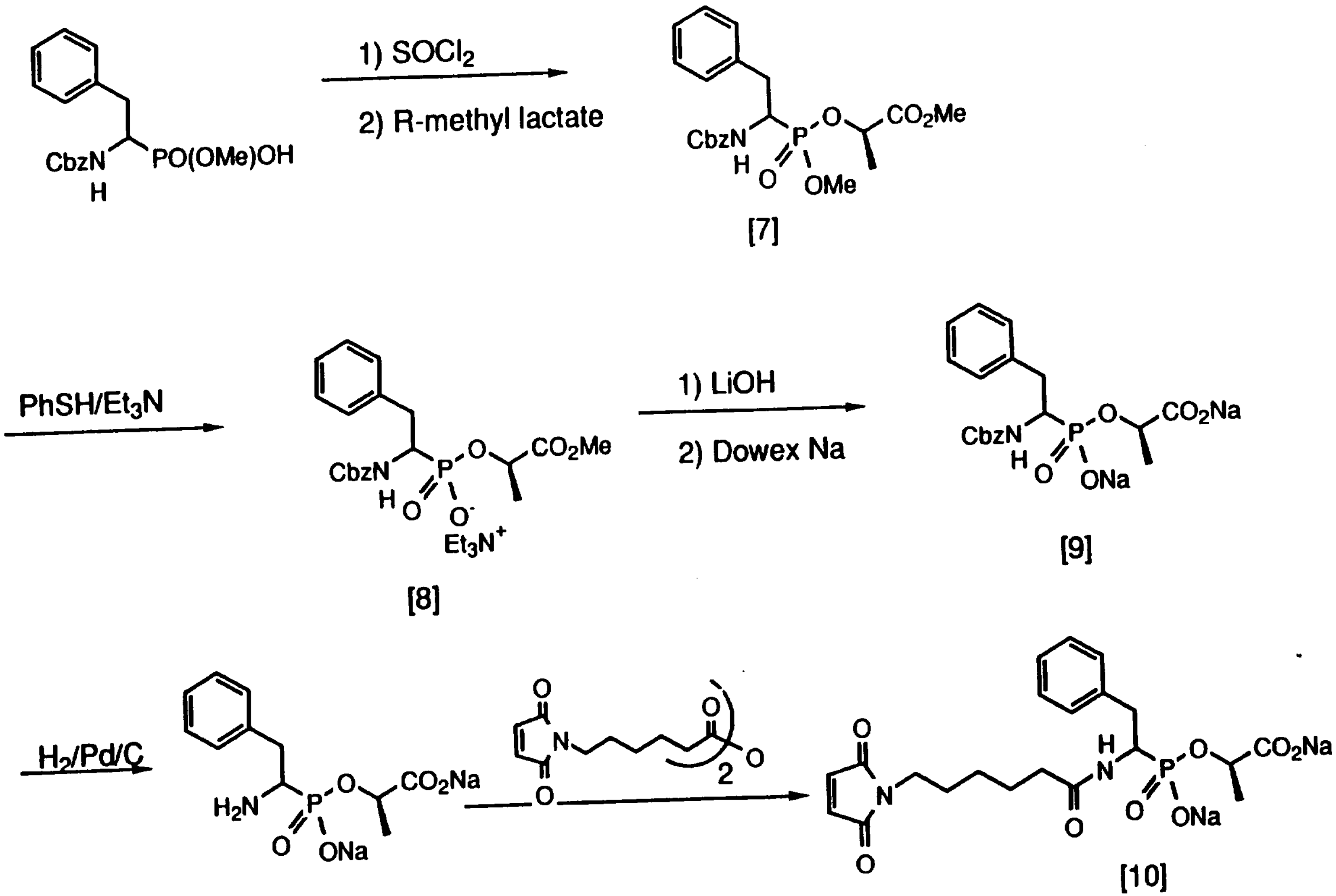


[7]

[8]

[9]

[10]

Fig 17

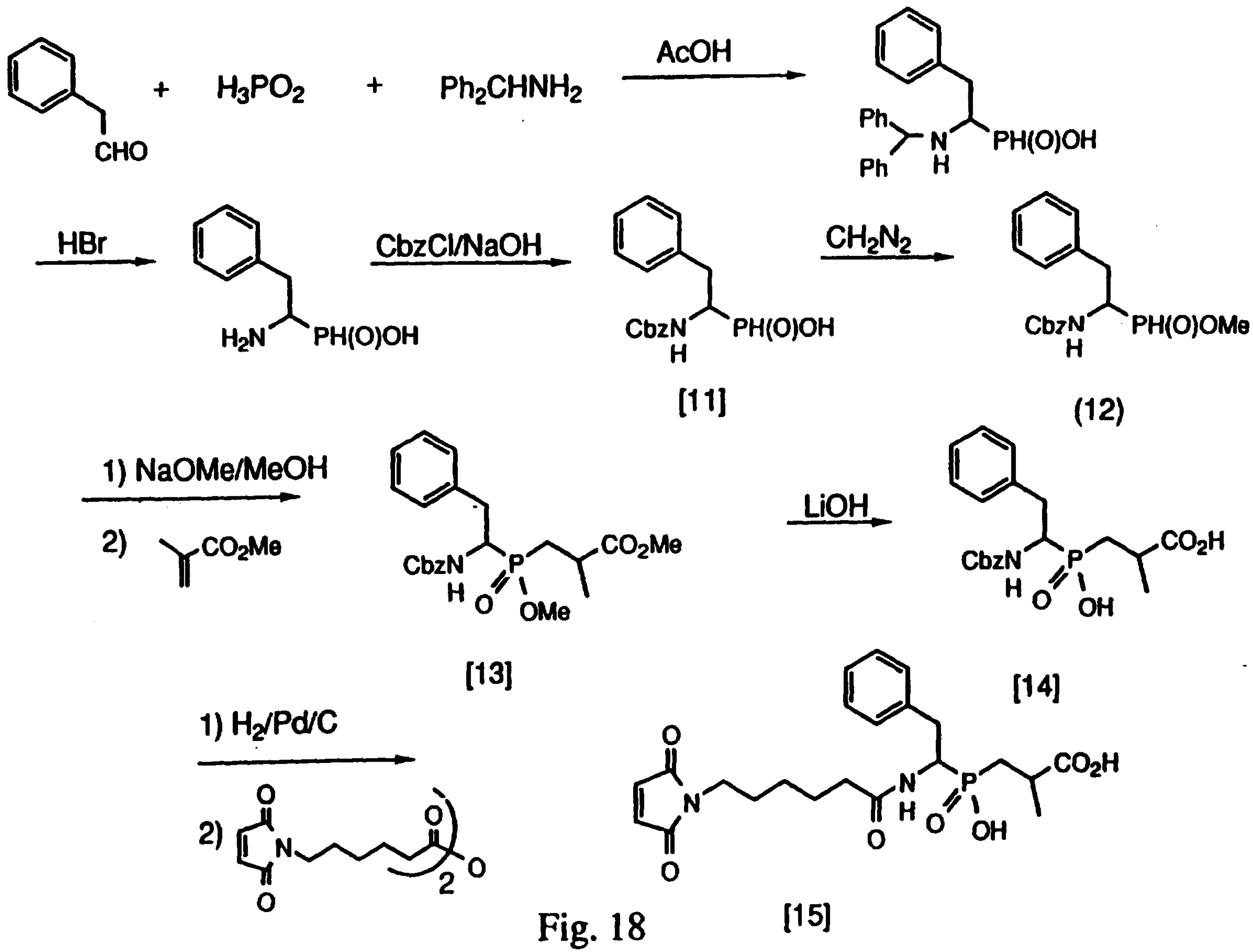

[11]
(12)
[13]
[14]
[15]

Fig. 18

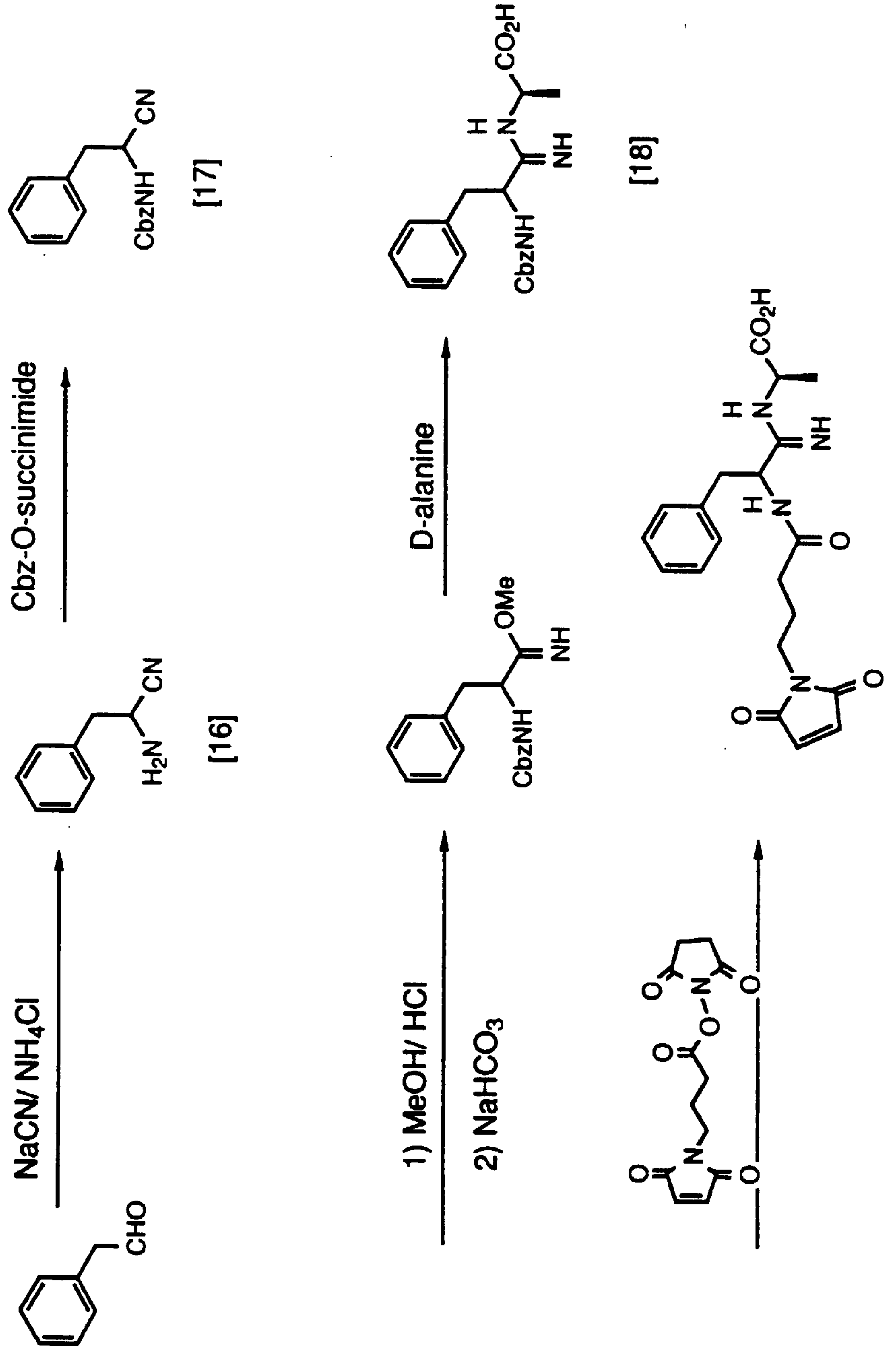


[16]

[17]

[18]

[19]

Fig. 19

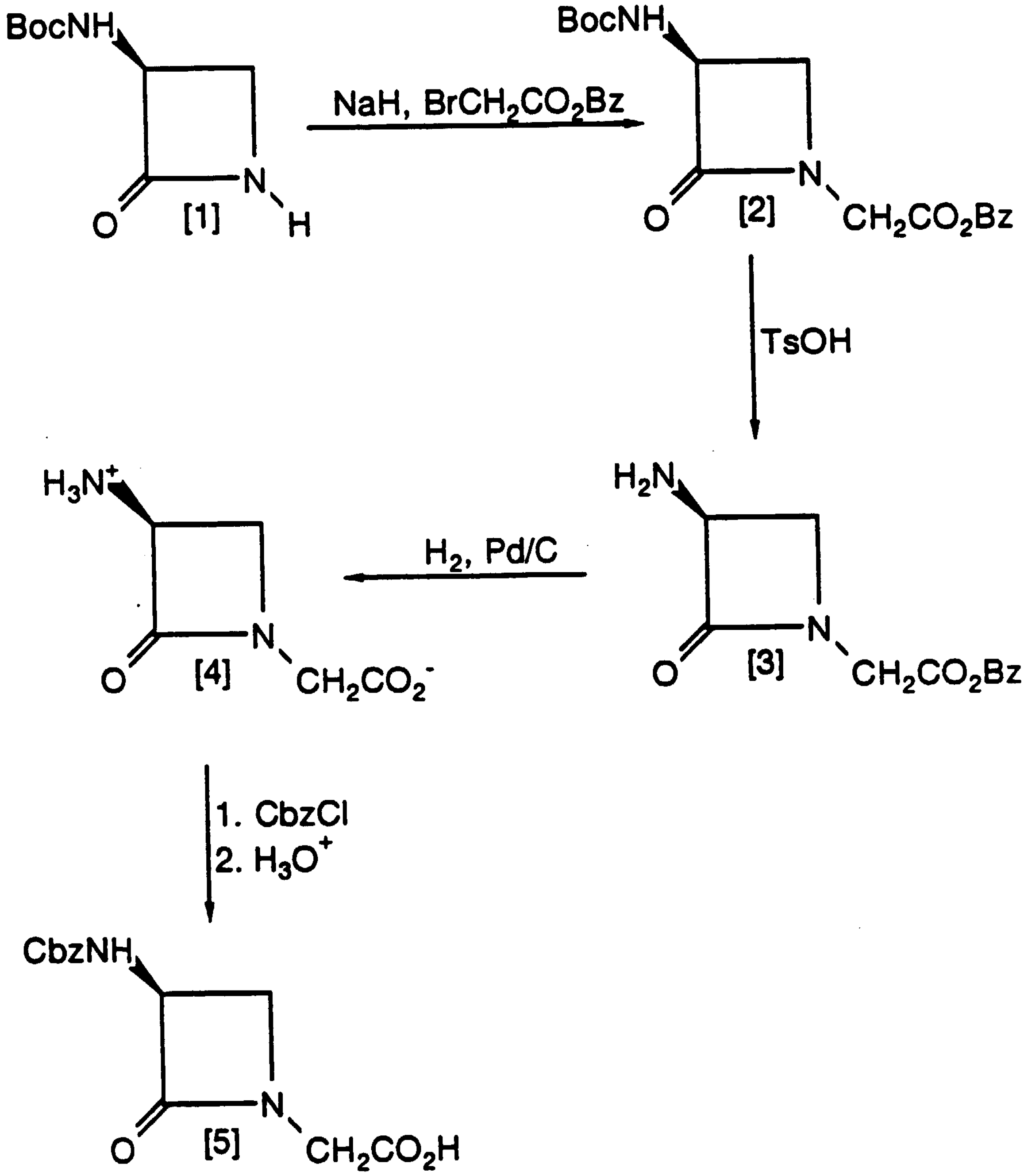


Figure 20

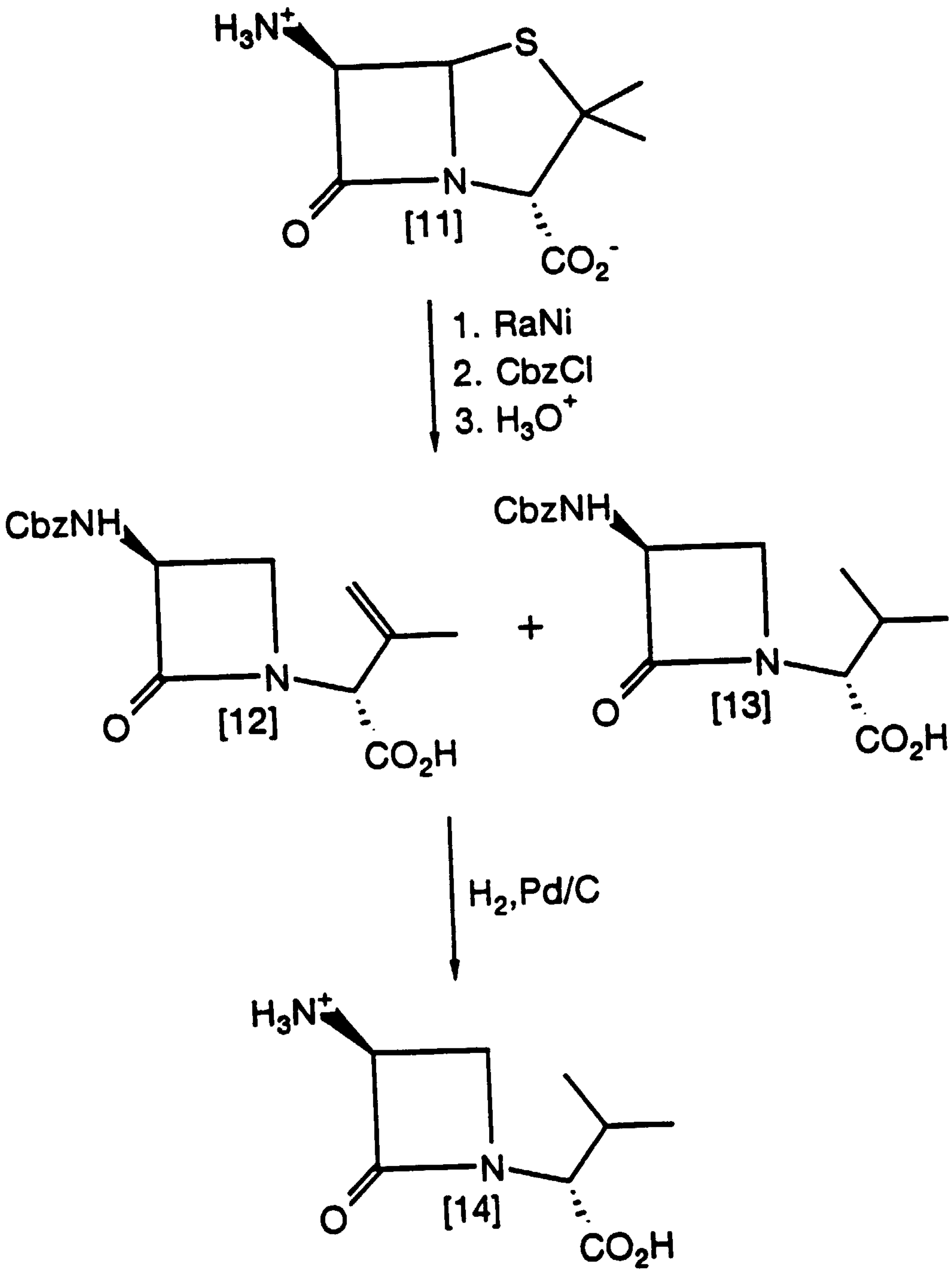


[11]

[12] + [13]

[14]

Figure 21

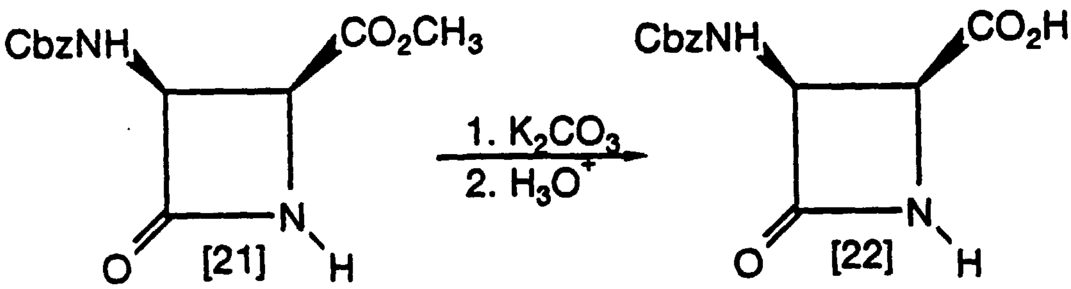


[21] [22]

Figure 22

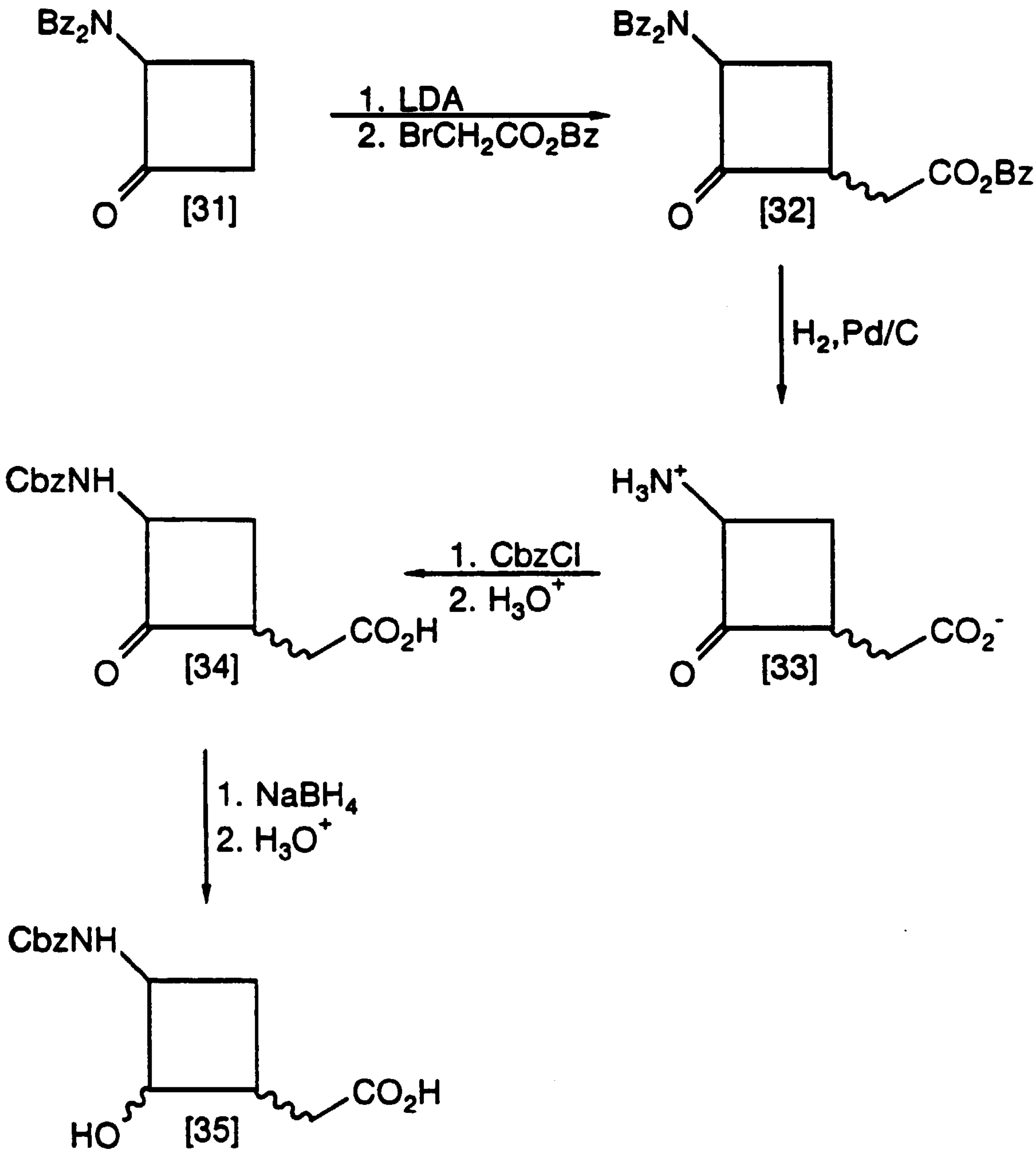
[31]
1. LDA
2. $BrCH_2CO_2Bz$
[32]
$H_2$,Pd/C
[33]
1. CbzCl
2. $H_3O^+$
[34]
1. $NaBH_4$
2. $H_3O^+$
[35]

Figure 23

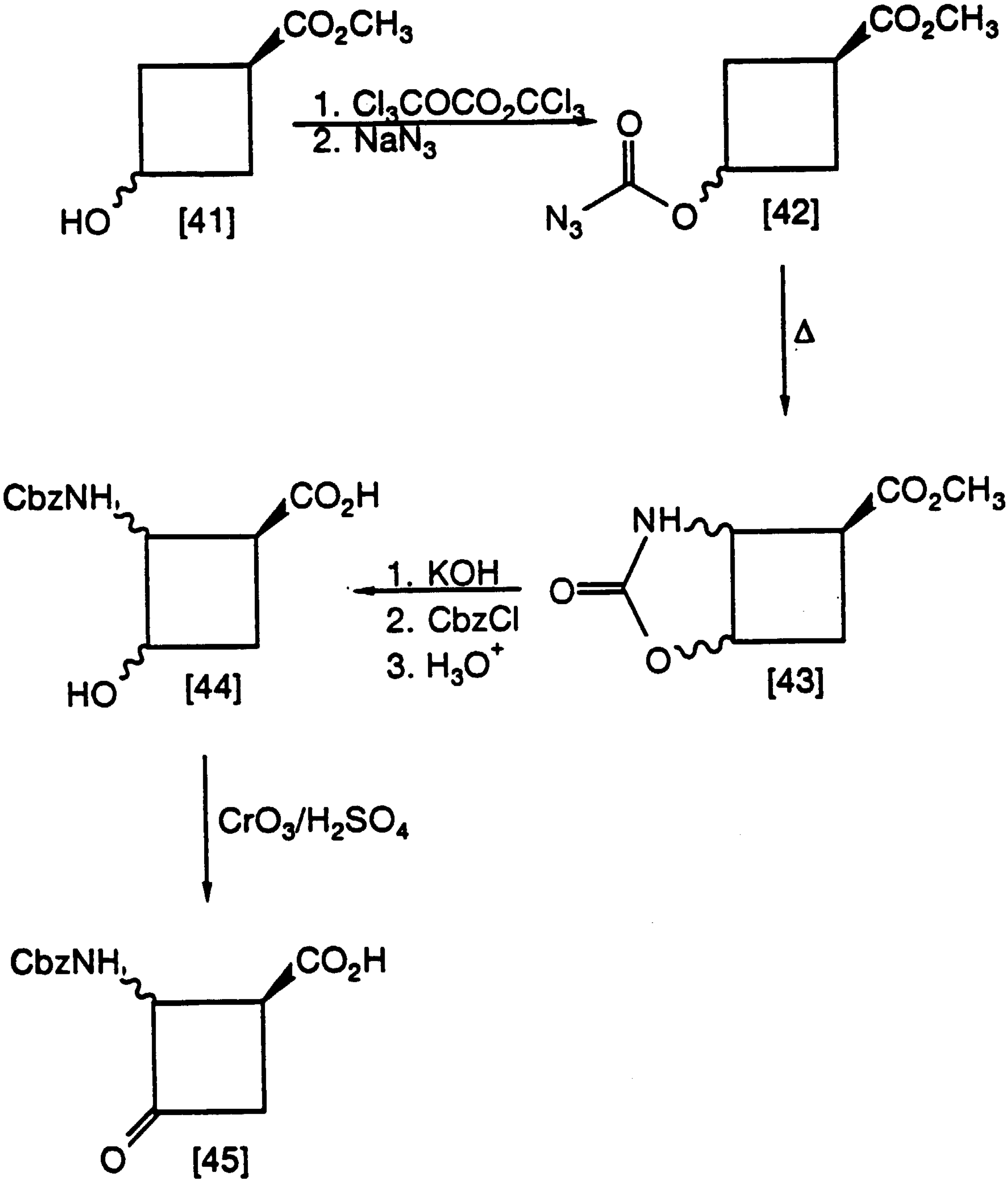


[41] [42] [43] [44] [45]

Figure 24

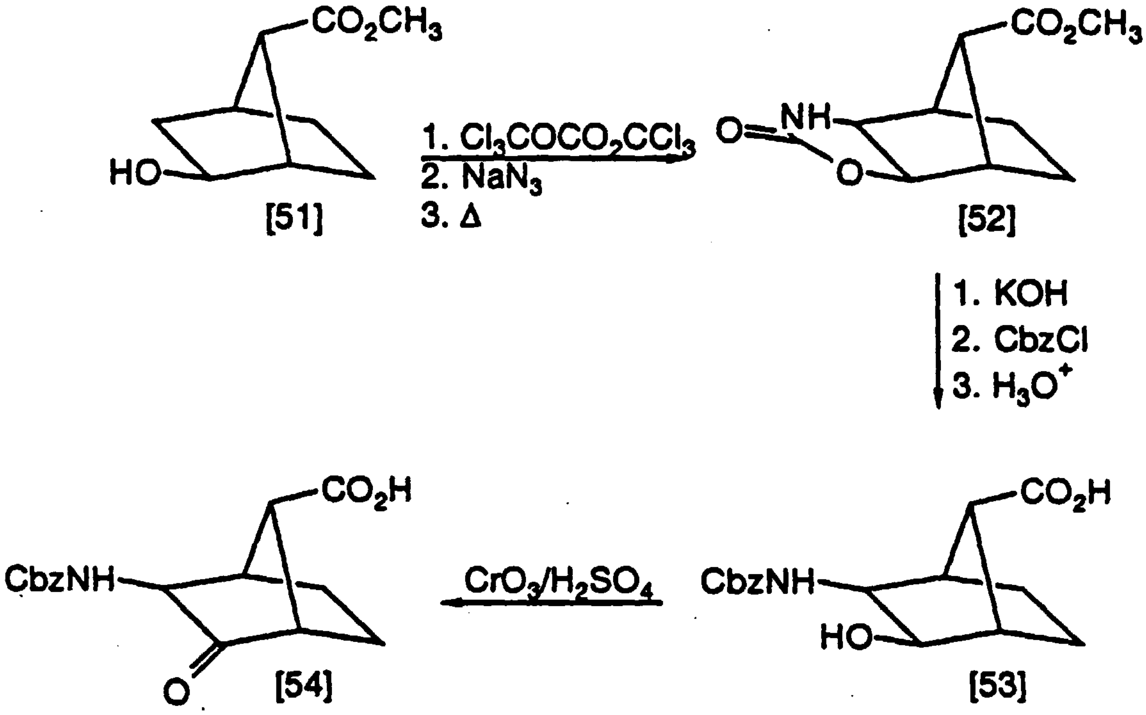


[51]

[52]

[53]

[54]

Figure 25

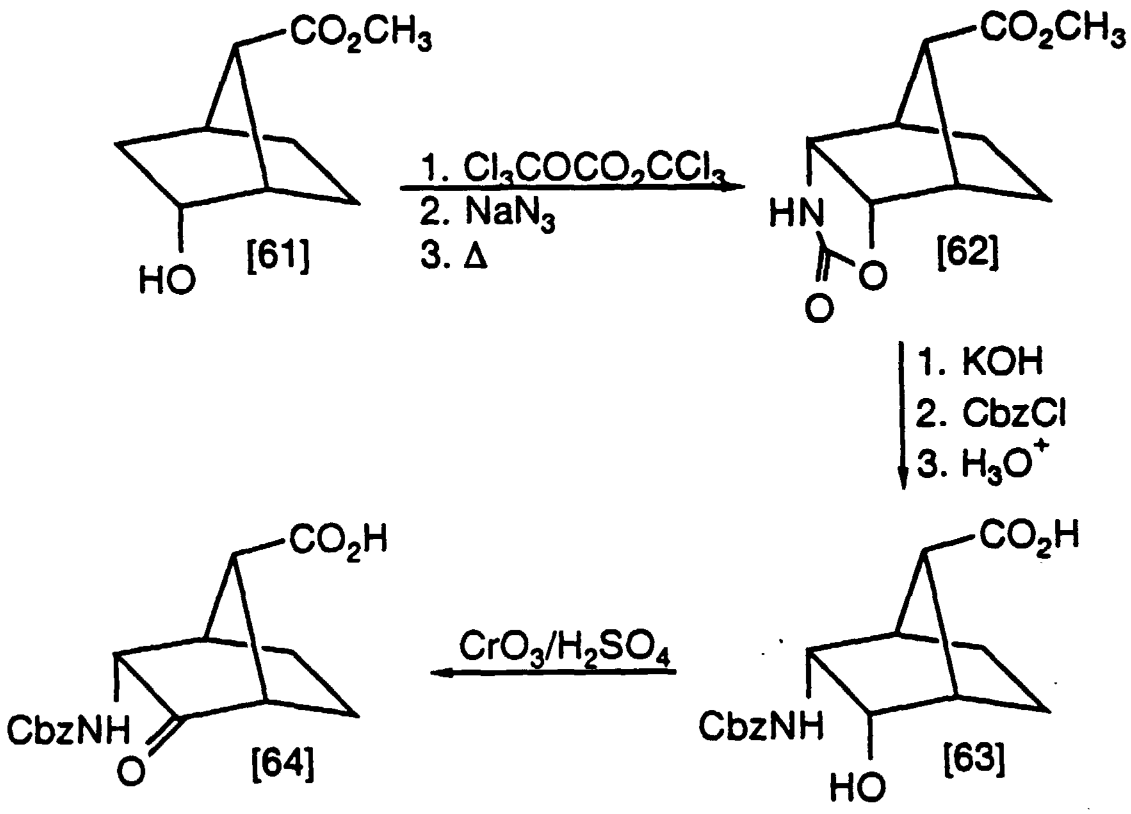


Figure 26

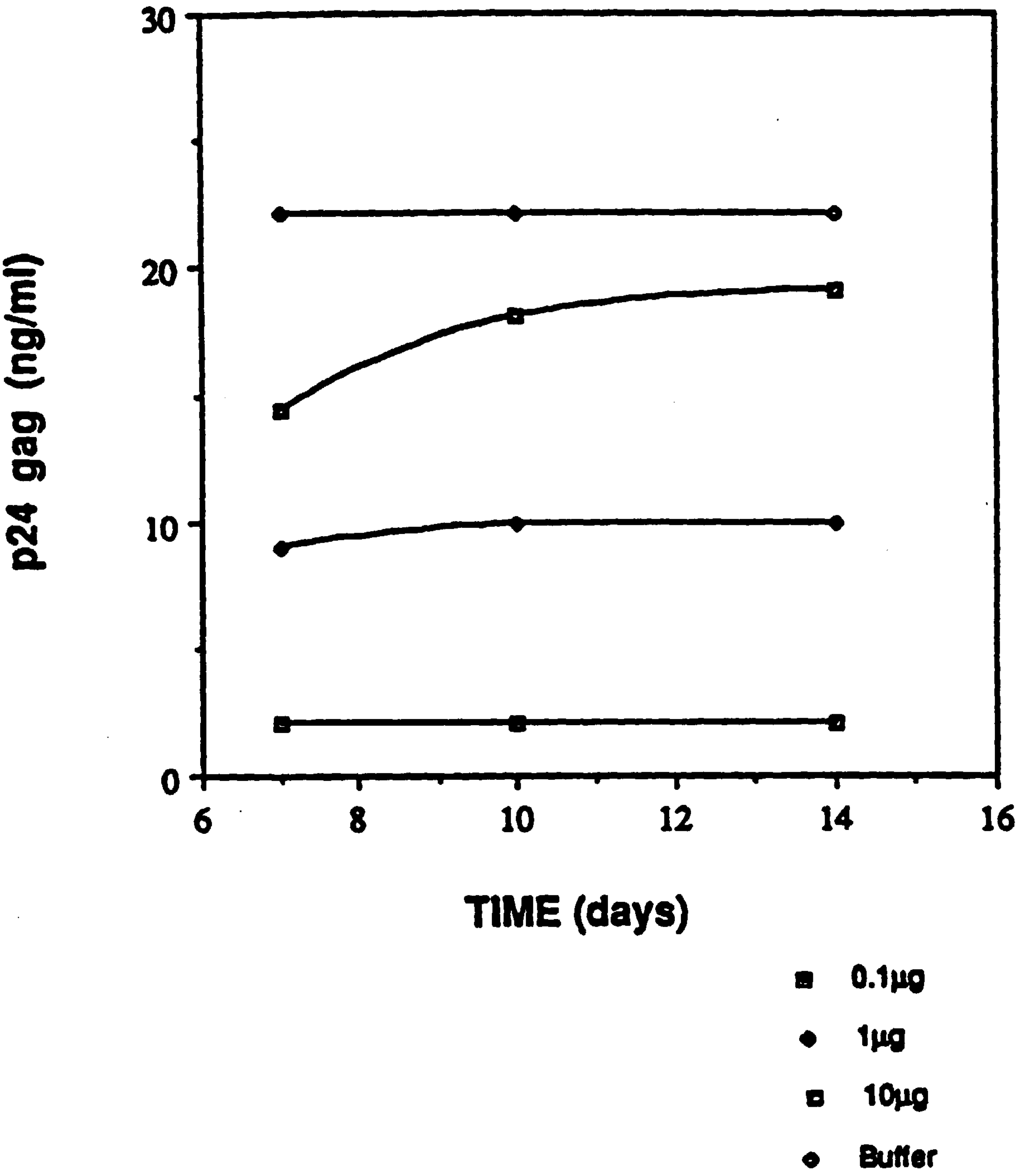
30
20
10
0
p24 gag (ng/ml)
6
8
10
12
14
16
TIME (days)
0.1µg
1µg
10µg
Buffer

Figure 27

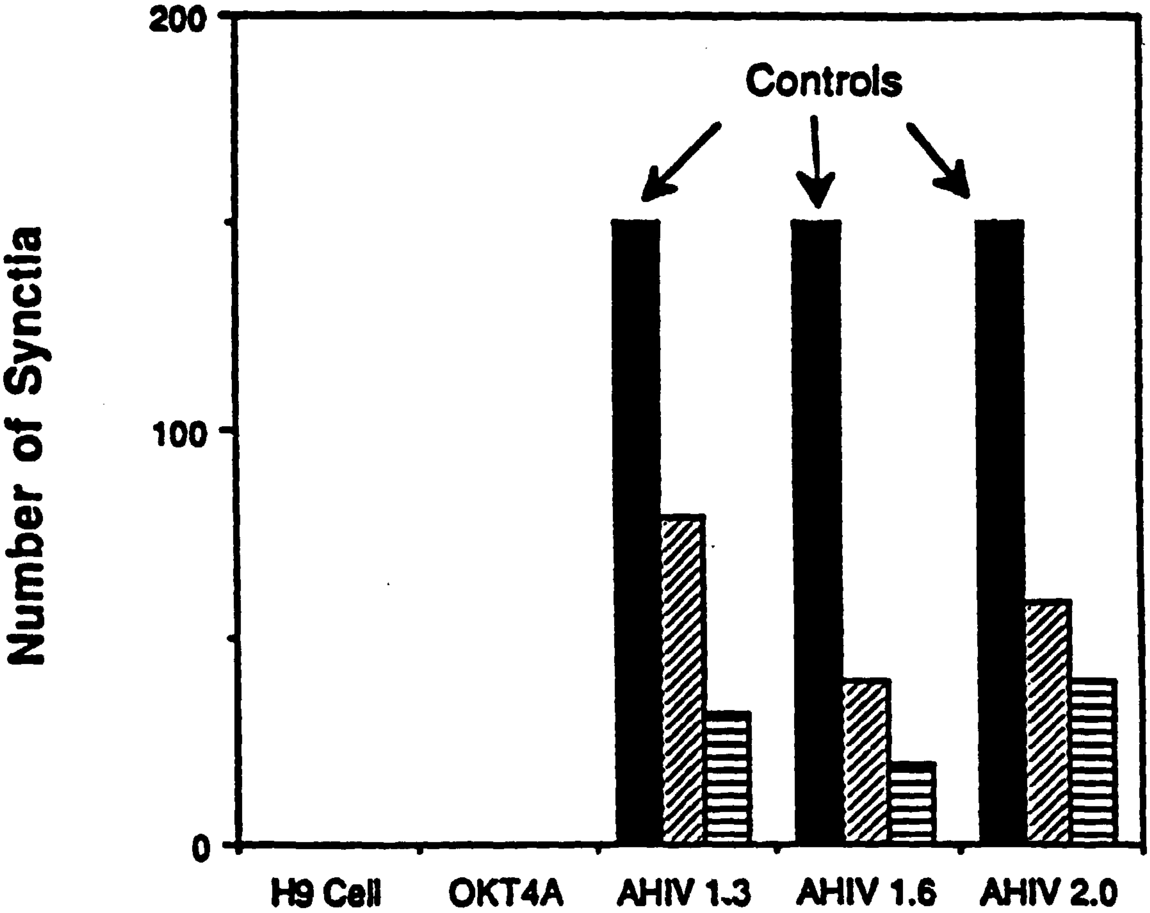
200
100
0
Number of Synctia
Controls
H9 Cell
OKT4A
AHIV 1.3
AHIV 1.6
AHIV 2.0

Figure 28